# EUROPEAN PATENT SPECIFICATION

(11) **EP 2 152 916 B1**
(45) Date of publication and mention of the grant of the patent: **20.04.2016**
(21) Application number: 08754986.1
(22) Date of filing: 01.05.2008
(51) Int. Cl.: C12Q 1/68, C40B 40/08

(54) **A TRANSCRIPTOMIC BIOMARKER OF MYOCARDITIS**
TRANSKRIPTOMISCHER BIOMARKER FÜR MYOKARDITIS
BIOMARQUEUR TRANSCRIPTOMIQUE DE LA MYOCARDITE

(30) Priority: 01.05.2007 US 915215 P
(43) Date of publication of application: 17.02.2010
(73) Proprietor: University of Miami, Miami FL 33136 (US)
(72) Inventor: HARE, Joshua, M., Miami, FL 33141 (US); HEIDECKER, Bettina, Miami, FL 33137 (US)
(74) Representative: Inspicos P/S
(86) International application number: PCT/US2008/062290
(87) International publication number: WO 2008/137595

(56) References cited:
- US-A1- 2005 143 628
- US-A1- 2006 172 311
- NANNI L ET AL: "Differential gene expression profiling in genetic and multifactorial cardiovascular diseases", JOURNAL OF MOLECULAR AND CELLULAR CARDIOLOGY, ACADEMIC PRESS, GB, vol. 41, no. 6, 1 December 2006 (2006-12-01), pages 934-948, XP024949841, ISSN: 0022-2828, DOI: DOI:10.1016/J.YJMCC.2006.08.009 [retrieved on 2006-12-01]
- DATABASE Gene Expression Omnibus [Online] NCBI; 7 November 2003 (2003-11-07), "Affymetrix Human Genome U133 Plus 2.0 Array", XP002627319, retrieved from http://www.ncbi.nlm.nih.gov/geo/ Database accession no. GPL570
- HEIDECKER ET AL.: 'The use of transcriptomic biomarkers for personalized medicine' HEART FALL REV. vol. 12, 28 March 2007, pages 1 - 11, XP019501327
- KITTLESON M M ET AL: "Gene expression in giant cell myocarditis: Altered expression of immune response genes", INTERNATIONAL JOURNAL OF CARDIOLOGY, ELSEVIER SCIENCE PUBLISHERS, AMSTERDAM, NL, vol. 102, no. 2, 10 July 2005 (2005-07-10) , pages 333-340, XP027744168, ISSN: 0167-5273 [retrieved on 2005-07-10]
- Bruce M. Mcmanus ET AL: "Genetic Determinants of Coxsackievirus B3 Pathogenesis", Annals of the New York Academy of Sciences, vol. 975, no. 1, 1 December 2002 (2002-12-01), pages 169-179, XP055058452, ISSN: 0077-8923, DOI: 10.1111/j.1749-6632.2002.tb05950.x
- KITTLESON MICHELLE M ET AL: "Identification of a gene expression profile that differentiates between ischemic and nonischemic cardiomyopathy", CIRCULATION, LIPPINCOTT WILLIAMS & WILKINS, US, vol. 110, no. 22, 30 November 2004 (2004-11-30), pages 3444-3451, XP002600679, ISSN: 0009-7322, DOI: 10.1161/01.CIR.0000148178.19465.11 [retrieved on 2004-11-22]
- KITTLESON MICHELLE M ET AL: "Gene expression analysis of ischemic and nonischemic cardiomyopathy: shared and distinct genes in the development of heart failure", PHYSIOLOGICAL GENOMICS, AMERICAN PHYSIOLOGICAL SOCIETY, US, vol. 21, no. 3, 1 May 2005 (2005-05-01), pages 299-307, XP002600680, ISSN: 1094-8341, DOI: 10.1152/PHYSIOLGENOMICS.00255.2004 [retrieved on 2005-03-15]
- HEIDECKER BETTINA ET AL: "Transcriptomic biomarkers for individual risk assessment in new-onset heart failure", CIRCULATION, LIPPINCOTT WILLIAMS & WILKINS, US, vol. 118, no. 3, 1 July 2008 (2008-07-01), pages 238-246, XP002600681, ISSN: 0009-7322, DOI: 10.1161/CIRCULATIONAHA.107.756544 [retrieved on 2008-06-30]
- YANAGAWA BOBBY ET AL: "Affymetrix oligonucleotide analysis of gene expression in the injured heart.", METHODS IN MOLECULAR MEDICINE 2005, vol. 112, 2005, pages 305-320, XP008161209, ISSN: 1543-1894
- Andrey Morgun ET AL: "Molecular profiling improves diagnoses of rejection and infection in transplanted organs.", Circulation Research, vol. 98, no. 12, 1 June 2006 (2006-06-01), pages e74-e83, XP055004147, ISSN: 0009-7330

## Description

### FIELD OF THE INVENTION

This invention relates to biomarkers of heart disease, myocarditis, novel drug therapeutic targets, compositions and methods of predicting, diagnosing and treating heart diseases and related disorders thereof. More specifically, the invention concerns methods and compositions based on unique molecular signatures associated with various aspects of cardiac diseases and disorders.

### BACKGROUND

The current approach to the treatment of patients with heart failure due to impaired cardiac function lacks individualization. This issue is of increasing importance as the number of classes of medicine for heart failure increase. Moreover there is growing appreciation that there may be utility to cause specific therapies. Accurate biomarkers are needed to refine diagnostic accuracy so as to enhance the application of personalized medicine in the field of heart failure.

There is a need in the art to provide early diagnosis and prognosis of heart disease. Myocarditis causes a significant minority of depressed heart function and thus causes heart failure and premature and unexpected sudden cardiac death. Myocarditis affects humans throughout life including children. The current diagnostic approach using histologic analysis of heart tissue obtained by biopsy lacks sensitivity and specificity. Given high risk of developing serious cardiac complications from myocarditis and the availability of disease specific therapies, there is a need for better biomarkers, to adjust treatment appropriately and early enough.

NANNI L ET AL (2006) JOURNAL OF MOLECULAR AND CELLULAR CARDIOLOGY, vol 41, 934-948 reviews gene expression profiling in cardiovascular diseases including myocarditis.

### SUMMARY

Molecular signatures that function as very sensitive diagnostic biomarker for myocarditis, heart disease and disorders thereof, were identified.

In a preferred embodiment, a molecular composition comprises gene sequences: 1553145_at (hypothetical protein FLJ39653), 1553575_at, 1557236_at (apolipoprotein L, 6), 1558142_at (trinucleotide repeat containing 6B), 1560752_at (F-box and WD-40 domain protein 2), 1565614_at (Zinc finger protein 337), 1567100_at (Dachshund homolog 1 (Drosophila)), 200068_s_at (calnexin /// calnexin), 201031_s_at (heterogeneous nuclear ribonucleoprotein H1 (H)), 202646_s_at (cold shock domain containing E1, RNA-binding), 205758_at (CD8a molecule /// CD8a molecule), 206188_at (zinc finger protein 623), 212637_s_at (WW domain containing E3 ubiquitin protein ligase 1), 212920_at, 213317_at (chloride intracellular channel 5), 213619_at (Heterogeneous nuclear ribonucleoprotein H1 (H)), 215443_at (thyroid stimulating hormone receptor), 216198_at (activating transcription factor 7 interacting protein), 217870_s_at (cytidylate kinase), 218087_s_at (sorbin and SH3 domain containing 1), 222145_at (CDNA: FLJ23572 fis, clone LNG12403), 223577_x_at (PRO1073 protein), 224321_at (transmembrane protein with EGF-like and two follistatin-like domains 2), 224373_s_at (IQ motif and WD repeats 1), 224644_at (CDNA clone IMAGE:5278517), 226173_at (ornithine aminotransferase-like 1), 226773_at (CDNA FLJ35131 fis, clone PLACE6008824), 226880_at (nuclear casein kinase and cyclin-dependent kinase substrate 1), 228980_at (ring finger and FYVE-like domain containing 1), 229569_at (CDNA clone IMAGE:5263455), 231735_s_at (PRO1073 protein), 233765_at (Hypothetical LOCI 97135), 235803_at (Cytokine receptor-like factor 3), 236131_at (CDNA clone IMAGE:6622963), 236953_s_at (similar to RIKEN cDNA 8030451K01), 240544_at (Zinc finger, AN1-type domain 3), 240971_x_at (Cullin 4A), 244042_x_at (Similar to retinoic acid receptor responder (tazarotene induced) 2), complementary sequences, fragments, alleles, variants and gene products thereof.

In another preferred embodiment, detection of the gene sequences, complementary sequences, fragments, alleles, variants and gene products thereof, is diagnostic of myocarditis and myocardial disorders.

In another preferred embodiment, detection in a cell or patient of at least ten gene sequences, complementary sequences, fragments, alleles, variants and gene products thereof, is diagnostic of diagnostic of myocarditis, idiopathic cardiomyopathy, heart diseases and disorders thereof.

In another preferred embodiment, the gene sequences, complementary sequences, fragments, alleles, variants and gene products thereof, are over-expressed at levels by at least 1%, 2%, 5%, 10% in a cell or patient as compared to levels in a normal cell or normal subject.

A biomarker (TBB-I) for the diagnosis of myocarditis comprises: nucleic acid sequences/biomolecules comprising: 1553145_at (hypothetical protein FLJ39653), 1553575_at, 1557236_at (apolipoprotein L, 6), 1558142_at (trinucleotide repeat containing 6B), 1560752_at (F-box and WD-40 domain protein 2), 1565614_at (Zinc finger protein 337), 1567100_at (Dachshund homolog 1 (Drosophila)), 200068_s_at (calnexin /// calnexin), 201031_s_at (heterogeneous nuclear ribonucleoprotein H1 (H)), 202646_s_at (cold shock domain containing E1, RNA-binding), 205758_at (CD8a molecule /// CD8a molecule), 206188_at (zinc finger protein 623), 212637_s_at (WW domain containing E3 ubiquitin protein ligase 1), 212920_at, 213317_at (chloride intracellular channel 5), 213619_at (Heterogeneous nuclear ribonucleoprotein H1 (H)), 215443_at (thyroid stimulating hormone receptor), 216198_at (activating transcription factor 7 interacting protein), 217870_s_at (cytidylate kinase), 218087_s_at (sorbin and SH3 domain containing 1), 222145_at (CDNA: FLJ23572 fis, clone LNG12403), 223577_x_at (PCRO1073 protein), 224321_at (transmembrane protein with EGF-like and two follistatin-like domains 2), 224373_s_at (IQ motif and WD repeats 1), 224644_at (CDNA clone IMAGE:5278517), 226173_at (ornithine aminotransferase-like 1), 226773_at (CDNA FLJ35131 fis, clone PLACE6008824), 226880_at (nuclear casein kinase and cyclin-dependent kinase substrate 1), 228980_at (ring finger and FYVE-like domain containing 1), 229569_at (CDNA clone IMAGE:5263455), 231735_s_at (PRO1073 protein), 233765_at (Hypothetical LOC197135), 235803_at (Cytokine receptor-like factor 3), 236131_at (CDNA clone IMAGE:6622963), 236953_s_at (similar to RIKEN cDNA 8030451K01), 240544_at (Zinc finger, AN1-type domain 3), 240971_x_at (Cullin 4A), 244042_x_at (Similar to retinoic acid receptor responder (tazarotene induced) 2), complementary sequences, fragments, alleles, variants and gene products thereof, complementary sequences, fragments, alleles, variants and gene products thereof.

In an alternative embodiment, detection of at least ten biomolecules is diagnostic of mycocarditis.

In another preferred embodiment, an antibody or aptamer specific for each gene sequence comprising nucleic acid sequences/biomolecules comprising: 1553145_at (hypothetical protein FLJ39653), 1553575_at, 1557236_at (apolipoprotein L, 6), 1558142_at (trinucleotide repeat containing 6B), 1560752_at (F-box and WD-40 domain protein 2), 1565614_at (Zinc finger protein 337), 1567100_at (Dachshund homolog 1 (drosophilia)), 200068_s_at (calnexin /// calnexin), 201031_s_at (heterogeneous nuclear ribonucleoprotein H1 (H)), 202646_s_at (cold shock domain containing E1, RNA-binding), 205758_at (CD8a molecule /// CD8a molecule), 206188_at (zinc finger protein 623), 212637_s_at (WW domain containing E3 ubiquitin protein ligase 1), 212920_at, 213317_at (chloride intracellular channel 5), 213619_at (Heterogeneous nuclear ribonucleoprotein H1 (H)), 215443_at (thyroid stimulating hormone receptor), 216198_at (activating transcription factor 7 interacting protein), 217870_s_at (cytidylate kinase), 218087_s_at (sorbin and SH3 domain containing 1), 222145_at (CDNA: FLJ23572 fis, clone LNG12403), 223577_x_at (PRO1073 protein), 224321_at (transmembrane protein with EGF-like and two follistatin-like domains 2), 224373_s_at (IQ motif and WD repeats 1), 224644_at (CDNA clone IMAGE:5278517), 226173_at (ornithine aminotransferase-like 1), 226773_at (CDNA FLJ35131 fis, clone PLACE6008824), 226880_at (nuclear casein kinase and cyclin-dependent kinase substrate 1), 228980_at (ring finger and FYVE-like domain containing 1), 229569_at (CDNA clone IMAGE:5263455), 231735_s_at (PRO1073 protein), 233765_at (Hypothetical LOC197135), 235803_at (Cytokine receptor-like factor 3), 236131_at (CDNA clone IMAGE:6622963), 236953_s_at (similar to RIKEN cDNA 8030451K01), 240544_at (Zinc finger, AN1-type domain 3), 240971_x_at (Cullin 4A), 244042_x_at (Similar to retinoic acid receptor responder (tazarotene induced) 2), complementary sequences, fragments, alleles, variants and gene products thereof, complementary sequences, fragments, alleles, variants and gene products thereof.

In another preferred embodiment, a biochip comprises nucleic acid sequences: 1553145_at (hypothetical protein FLJ39653), 1553575_at, 1557236_at (apolipoprotein L, 6), 1558142_at (trinucleotide repeat containing 6B), 1560752_at (F-box and WD-40 domain protein 2), 1565614_at (Zinc finger protein 337), 1567100_at (Dachshund homolog 1 (Drosophila)), 200068_s_at (calnexin /// calnexin), 201031_s_at (heterogeneous nuclear ribonucleoprotein H1 (H)), 202646_s_at (cold shock domain containing E1, RNA-binding), 205758_at (CD8a molecule /// CD8a molecule), 206188_at (zinc finger protein 623), 212637_s_at (WW domain containing E3 ubiquitin protein ligase 1), 212920_at, 213317 _at (chloride intracellular channel 5), 213619_at (Heterogeneous nuclear ribonucleoprotein H1 (H)), 215443_at (thyroid stimulating hormone receptor), 216198_at (activating transcription factor 7 interacting protein), 217870_s_at (cytidylate kinase), 218087_s_at (sorbin and SH3 domain containing 1), 222145_at (CDNA: FLJ23572 fis, clone LNG12403), 223577_x_at (PRO1073 protein), 224321_at (transmembrane protein with EGF-like and two follistatin-like domains 2), 224373_s_at (IQ motif and WD repeats 1), 224644_at (CDNA clone IMAGE:5278517), 226173_at (ornithine aminotransferase-like 1), 226773_at (CDNA FLJ35131 fis, clone PLACE6008824), 226880_at (nuclear casein kinase and cyclin-dependent kinase substrate 1), 228980_at (ring finger and FYVE-like domain containing 1), 229569_at (CDNA clone IMAGE:5263455), 231735_s_at (PRO1073 protein), 233765_at (Hypothetical LOC197135), 235803_at (Cytokine receptor-like factor 3), 236131_at (CDNA clone IMAGE:6622963), 236953_s_at (similar to RIKEN cDNA 8030451K01), 240544_at (Zinc finger, AN 1-type domain 3), 24097_1_x_at (Cullin 4A), 244042_x_at (Similar to retinoic acid receptor responder (tazarotene induced) 2), complementary sequences, fragments, alleles, variants and gene products thereof, complementary sequences, fragments, alleles, variants and gene products thereof.

In another preferred embodiment, the biochip comprises at least ten nucleic sequences, complementary sequences, fragments, alleles, variants and gene products thereof.

In one preferred embodiment, a transcriptomic biomarker (TBB- II) comprises gene sequences: 1552419_s_at (tubulin tyrosine ligase-like family, member 10), 1553212_at (keratin 78), 1555124_at (hypothetical protein MGC40574), 1556192_x_at (Metastasis suppressor 1), 1556320_at (Stomatin (EPB72)-like 1), 1556510_at (CDNA clone IMAGE:4796864), 1558484_s_at (leucine rich repeat containing 27), 1565662_at (Mucin 6, oligomeric mucus/gel-forming), 1567410_at (zinc finger protein 135), 1568513_x_at (Protease, serine, 1 (trypsin 1)), 1570408_at (Serine/threonine kinase 24 (STE20 homolog, yeast)), 203307_at (guanine nucleotide binding protein-like 1), 204581_at (CD22 molecule, myelin associated glycoprotein), 205586_x_at (VGF nerve growth factor inducible), 206333_at (musashi homolog 1 (Drosophila)), 207004_at (B-cell CLL/lymphoma 2), 210059_s_at (mitogen-activated protein kinase 13), 210228_at (colony stimulating factor 2 (granulocyte-macrophage)), 210384_at (protein arginine methyltransferase 2), 210923_at (solute carrier family 1 (glutamate transporter), member 7), 211024_s_at (thyroid transcription factor 1 /// thyroid transcription factor 1), 211062_s_at (carboxypeptidase Z /// carboxypeptidase Z), 211096_at (pre-B-cell leukemia transcription factor 2), 211181_x_at (runt-related transcription factor 1 (acute myeloid leukemia 1; aml1 oncogene)), 211710_x_at (ribosomal protein L4 /// ribosomal protein L4), 213096_at (transmembrane and coiled-coil domain family 2), 213121_at (small nuclear ribonucleoprotein 70kDa polypeptide (RNP antigen)), 213242_x_at (KIAA0284), 213568_at (odd-skipped related 2 (Drosophila)), 213770_at kinase suppressor of ras 1 (214171_s_at (U2 small nuclear RNA auxiliary factor 2), 216116_at (NCK interacting protein with SH3 domain), 216427_at (CDNA: FLJ22786 fis, clone KAIA2150), 216820_at, 217054_at (CDNA FLJ39484 fis, clone PROST2014925), 217180_at (Hypothetical protein similar to KIAA0187 gene product), 217182_at (mucin 5AC, oligomeric mucus/gel-forming), 217322_x_at, 217430_x_at (collagen, type 1, alpha 1), 219070_s_at (motile sperm domain containing 3), 219425_at (sulfotransferase family 4A, member 1), 221663_x_at (histamine receptor H3), 221684_s_at (Nyctalopin), 223974_at (hypothetical protein MGC11082), 226640_at (diacylglycerol lipase beta), 228074_at (hypothetical protein LOC162073), 229191_at (tubulin folding cofactor D), 229257_at (KIAA1856 protein), 229335_at (immunoglobulin superfamily, member 4C), 229358_at (Indian hedgehog homolog (Drosophila)), 230341_x_at (ADAM metallopeptidase with thrombospondin type 1 motif, 10), 230693_at (ATPase, Ca⁺⁺ transporting, cardiac muscle, fast twitch 1), 230768_at (FERM, RhoGEF and pleckstrin domain protein 2), 231510_at (GLI-Kruppel family member GLI2), 231629_x_at (Kallikrein-related peptidase 3), 231998_at, 233794_at (Single stranded DNA binding protein 3), 233974_s_at (family with sequence similarity 129, member B), 234495_at (kallikrein-related peptidase 15), 234637_at (keratin associated protein 4-5), 234881_at, 235568_at (chromosome 19 open reading frame 59), 235600_at (Transcribed locus), 236496_at (degenerative spermatocyte homolog 2, lipid desaturase (Drosophila)), 237087_at (Chromosome 14 open reading frame 105), 237144_at (Latent transforming growth factor beta binding protein 3), 237398_at (Transcribed locus), 237547_at (Hypothetical protein LOC728730), 237679_at (tripartite motif-containing 66), 238267_s_at, 238445_x_at (mannosyl (alpha-1,6-)-glycoprotein beta-1,6-N-acetyl-glucosaminyltransferase, isozyme B), 239026_x_at (centaurin, gamma 3), 239463_at (Transcribed locus), 239756_at (MAD mitotic arrest deficient-like 1 (yeast)), 240039_at (Transcribed locus /// Transcribed locus), 240147_at (hypothetical protein MGC11257), 240517_at (Cystathionine-beta-synthase), 241270_at (Rhomboid 5 homolog 2 (Drosophila)), 241431_at, 242365_at (Coiled-coil domain containing 32), 243297_at (Vacuolar protein sorting 13 homolog D (*S*. *cerevisiae*)), 243497_at (Transcribed locus), 243766_s_at (TEA domain family member 2), 43934_at (G protein-coupled receptor 137), complementary sequences, fragments, alleles, variants and gene products thereof.

In another preferred embodiment, the detection of the gene sequences, complementary sequences, fragments, alleles, variants and gene products thereof, is diagnostic of myocarditis and myocardial disorders, cardiac and cardiovascular diseases and disorders, such as for example, Coronary Heart Disease, angina, Acute Coronary Syndrome, Aortic Aneurysm and Dissection, arrhythmias, Cardiomyopathy, Congenital Heart Disease, congestive heart failure or chronic heart failure, pericarditis, and the like.

In another alternative embodiment, detection in a cell or patient of at least ten gene sequences, complementary sequences, fragments, alleles, variants and gene products thereof, is diagnostic of diagnostic of myocarditis, idiopathic cardiomyopathy, heart diseases and disorders thereof.

In another embodiment, the gene sequences, complementary sequences, fragments, alleles, variants and gene products thereof, are modulated at levels by at least about 1%, 5%, 10%, 100%, 200% or more in a cell or patient as compared to levels in a normal cell or normal subject.

In another preferred embodiment, a biochip comprises nucleic acid sequences: 1552419_s_at (tubulin tyrosine ligase-like family, member 10), 1553212_at (keratin 78), 1555124_at (hypothetical protein MGC40574), I 556192_x_at (Metastasis suppressor 1), 1556320_at (Stomatin (EPB72)-like 1), 1556510_at (CDNA clone IMAGE:4796864), 1558484_s_at (leucine rich repeat containing 27), 1565662_at (Mucin 6, oligomeric mucus/gel-forming), 15674!0_at (zinc finger protein 135), 1568513_x_at (Protease, serine, 1 (trypsin 1)), 1570408_at (Serine/threonine kinase 24 (STE20 homolog, yeast)), 203307_at (guanine nucleotide binding protein-like 1), 204581_at (CD22 molecule, myelin associated glycoprotein), 205586_x_at (VGF nerve growth factor inducible), 206333_at (musashi homolog 1 (Drosophila)), 207004_at (B-cell CLL/lymphoma 2), 210059_s_at (mitogen-activated protein kinase 13), 210228_at (colony stimulating factor 2 (granulocyte-macrophage)), 210384_at (protein arginine methyltransferase 2), 210923_at (solute carrier family 1 (glutamate transporter), member 7), 211024_s_at (thyroid transcription factor 1 /// thyroid transcription factor 1), 211062_s_at (carboxypeptidase Z /// carboxypeptidase Z), 211096_at (pre-B-cell leukemia transcription factor 2), 211181_x_at (runt-related transcription factor 1 (acute myeloid leukemia 1; amll oncogene)), 211710_x_at (ribosomal protein L4 /// ribosomal protein L4), 213096_at (transmembrane and coiled-coil domain family 2), 213121_at (small nuclear ribonucleoprotein 70kDa polypeptide (RNP antigen)), 213242_x_at (KIAA0284), 213568_at (odd-skipped related 2 (Drosophila)), 213770_at kinase suppressor of ras 1 (214171_s_at (U2 small nuclear RNA auxiliary factor 2), 216116_at (NCK interacting protein with SH3 domain), 216427_at (CDNA: FLJ22786 fis, clone KAIA2150), 216820_at, 217054_at (CDNA FLJ39484 tis, clone PROST2014925), 2!7180_at (Hypothetical protein similar to KIAA0187 gene product), 217182_at (mucin 5AC, oligomeric mucus/gel-forming), 217322_x_at, 217430_x_at (collagen, type I, alpha 1), 219070_s_at (motile sperm domain containing 3), 219425_at (sulfotransferase family 4A, member 1), 221663_x_at (histamine receptor H3), 221684_s_at (Nyctalopin), 223974_at (hypothetical protein MGC11082), 226640_at (diacylglycerol lipase beta), 228074_at (hypothetical protein LOC162073), 229191_at (tubulin folding cofactor D), 229257_at (KIAA1856 protein), 229335_at (immunoglobulin superfamily, member 4C), 229358_at (Indian hedgehog homolog (Drosophila)), 230341_x_at (ADAM metallopeptidase with thrombospondin type 1 motif, 10), 230693_at (ATPase, Ca⁺⁺ transporting, cardiac muscle, fast twitch 1), 230768_at (FERM, RhoGEF and pleckstrin domain protein 2), 231510_at (GLI-Kruppel family member GLI2), 231629_x_at (Kallikrein-related peptidase 3), 231998_at, 233794_at (Single stranded DNA binding protein 3), 233974_s_at (family with sequence similarity 129, member B), 234495_at (kallikrein-related peptidase 15), 234637_at (keratin associated protein 4-5), 234881_at, 235568_at (chromosome 19 open reading frame 59), 235600_at (Transcribed locus), 236496_at (degenerative spermatocyte homolog 2, lipid desaturase (Drosophila)), 237087_at (Chromosome 14 open reading frame 105), 237144_at (Latent transforming growth factor beta binding protein 3), 237398_at (Transcribed locus), 237547_at (Hypothetical protein LOC728730), 237679_at (tripartite motif-containing 66), 238267_s_at, 238445_x_at (mannosyl (alpha-1,6-)-glycoprotein beta-1,6-N-acetyl-glucosaminyltransferase, isozyme B), 239026_x_at (centaurin, gamma 3), 239463_at (Transcribed locus), 239756_at (MAD1 mitotic arrest deficient-like 1 (yeast)), 240039_at (Transcribed locus /// Transcribed locus), 240147_at (hypothetical protein MGC 11257), 240517_at (Cystathionine-beta-synthase), 241270_at (Rhomboid 5 homolog 2 (Drosophila)), 241431_at, 242365_at (Coiled-coil domain containing 32), 243297_at (Vacuolar protein sorting 13 homolog D (*S*. *cerevisiae*))*,* 243497_at (Transcribed locus), 243766_s_at (TEA domain family member 2), 43934_at (G protein-coupled receptor 137), complementary sequences, fragments, alleles, variants and gene products thereof.

In yet another embodiment, an antibody or aptamer specific for each gene sequence comprising nucleic acid sequences/biomolecules comprising: 1552419_s_at (tubulin tyrosine ligase-like family, member 10), 1553212_at (keratin 78), 1555124_at (hypothetical protein MGC40574), 1556192_x_at (Metastasis suppressor 1), 1556320_at (Stomatin (EPB72)-like 1), 1556510_at (CDNA clone IMAGE:4796864), 1558484_s_at (leucine rich repeat containing 27), 1565662_at (Mucin 6, oligomeric mucus/gel-forming), 1567410_at (zinc finger protein 135), 1568513_x_at (Protease, serine, 1 (trypsin 1)), 1570408_at (Serine/threonine kinase 24 (STE20 homolog, yeast)), 203307_at (guanine nucleotide binding protein-like 1), 204581_at (CD22 molecule, myelin associated glycoprotein), 205586_x_at (VGF nerve growth factor inducible), 206333_at (musashi homolog 1 (Drosophila)), 207004_at (B-cell CLL/lymphoma 2), 210059_s_at (mitogen-activated protein kinase 13), 210228_at (colony stimulating factor 2 (granulocyte-macrophage)), 210384_at (protein arginine methyltransferase 2), 210923_at (solute carrier family 1 (glutamate transporter), member 7), 211024_s_at (thyroid transcription factor 1 /// thyroid transcription factor 1), 211062_s_at (carboxypeptidase Z /// carboxypeptidase Z), 211096_at (pre-B-cell leukemia transcription factor 2), 211181_x_at (runt-related transcription factor 1 (acute myeloid leukemia 1; aml1 oncogene)), 211710_x_at (ribosomal protein L4 /// ribosomal protein L4), 213096_at (transmembrane and coiled-coil domain family 2), 213121_at (small nuclear ribonucleoprotein 70kDa polypeptide (RNP antigen)), 213242_x_at (KIAA0284), 213568_at (odd-skipped related 2 (Drosophila)), 213770_at kinase suppressor of ras 1 (214171_s_at (U2 small nuclear RNA auxiliary factor 2), 216116_at (NCK interacting protein with SH3 domain), 216427_at (CDNA: FLJ22786 fis, clone KAIA2150), 216820_at, 217054_at (CDNA FLJ39484 fis, clone PROST2014925), 217180_at (Hypothetical protein similar to KIAA0187 gene product), 217182_at (mucin 5AC, oligomeric mucus/gel-forming), 217322_x_at, 217430_x_at (collagen, type I, alpha 1), 219070_s_at (motile sperm domain containing 3), 219425_at (sulfotransferase family 4A, member 1), 221663_x_at (histamine receptor H3), 221684_s_at (Nyctalopin), 223974_at (hypothetical protein MGC11082), 226640_at (diacylglycerol lipase beta), 228074_at (hypothetical protein LOC162073), 229191_at (tubulin folding cofactor D), 229257_at (KIAA1856 protein), 229335_at (immunoglobulin superfamily, member 4C), 229358_at (Indian hedgehog homolog (Drosophila)), 230341_x_at (ADAM metallopeptidase with thrombospondin type 1 motif, 10), 230693_at (ATPase, Ca⁺⁺ transporting, cardiac muscle, fast twitch 1), 230768_at (FERM, RhoGEF and pleckstrin domain protein 2), 231510_at (GLI-Kruppel family member GLI2), 231629_x_at (Kallikrein-related peptidase 3), 231998_at, 233794_at (Single stranded DNA binding protein 3), 233974_s_at (family with sequence similarity 129, member B), 234495_at (kallikrein-related peptidase 15), 234637_at (keratin associated protein 4-5), 234881_at, 235568_at (chromosome 19 open reading frame 59), 235600_at (Transcribed locus), 236496_at (degenerative spermatocyte homolog 2, lipid desaturase (Drosophila)), 237087_at (Chromosome 14 open reading frame 105), 237144_at (Latent transforming growth factor beta binding protein 3), 237398_at (Transcribed locus), 237547_at (Hypothetical protein LOC728730), 237679_at (tripartite motif-containing 66), 238267_s_at, 238445_x_at (mannosyl (alpha-1,6-)-glycoprotein beta-1,6-N-acetyl-glucosaminyltransferase, isozyme B), 239026_x_at (centaurin, gamma 3), 239463_at (Transcribed locus), 239756_at (MAD1 mitotic arrest deficient-like 1 (yeast)), 240039_at (Transcribed locus /// Transcribed locus), 240147_at (hypothetical protein MGC 11257), 240517_at (Cystathionine-beta-synthase), 24!270_at (Rhomboid 5 homolog 2 (Drosophila)), 241431_at, 242365_at (Coiled-coil domain containing 32), 243297_at (Vacuolar protein sorting 13 homolog D (*S cerevisiae*)), 243497_at (Transcribed locus), 243766_s_at (TEA domain family member 2), 43934_at (G protein-coupled receptor 137), complementary sequences, fragments, alleles, variants and gene products thereof.

In another embodiment, a biomarker (TBB-III) comprises nucleic acid sequences/biomolecules comprising: 1553212_at (keratin 78), 1557236_at (apolipoprotein L), 1558142_at (trinucleotide repeat containing 6B), 1558484_s_at (leucine rich repeat containing 27), 1565614_at (Zinc finger protein 337), 1565662_at (Mucin 6, oligomeric mucus/gel-forming), 1567100_at (Dachshund homolog 1 (Drosophila)), 203307_at (guanine nucleotide binding protein-like 1), 205758_at (CD8a molecule /// CD8a molecule), 206333_at (musashi homolog 1 (Drosophila)), 212920_at, 213242_x_at (KIAA0284), 213619_at (Heterogeneous nuclear ribonucleoprotein H1 (H)), 213770_at (kinase suppressor of ras 1), 214171_s_at (U2 small nuclear RNA auxiliary factor 2), 215443_at (thyroid stimulating hormone receptor), 216198_at (activating transcription factor 7 interacting protein), 216427_at (CDNA: FLJ22786 fis, clone KAIA2150), 217054_at (CDNA FLJ39484 fis, clone PROST2014925), 217182_at (mucin 5AC, oligomeric mucus/gel-forming), 217322_x_at, 219425_at (sulfotransferase family 4A, member 1), 222!45_at (CDNA: FLJ23572 fis, clone LNG12403), 229191_at (tubulin folding cofactor D), 229569_at (CDNA clone IMAGE:5263455), 231_629_x_at (Kallikrein-related peptidase 3), 233765_at (Hypothetical LOC197135), 233794_at (Single stranded DNA binding protein 3), 233974_s_at (family with sequence similarity 129, member B), 234495_at (kallikrein-related peptidase 15), 235568_at (chromosome 19 open reading frame 59), 235803_at (Cytokine receptor-like factor 3), 236496_at (degenerative spermatocyte homolog 2, lipid desaturase (Drosophila)), 236953_s_at, 238445_x_at (mannosyl (alpha-1,6-)-glycoprotein beta-1,6-N-acetyl-glucosaminyltransferase, isozyme B), 239463_at (Transcribed locus), 240544_at (Zinc finger, AN 1-type domain 3), 243766_s_at (TEA domain family member 2), and 244042_x_at complementary sequences, fragments, alleles, variants and gene products thereof.

In another preferred embodiment, a method of diagnosing myocarditis, comprises identifying in a biological sample from a patient a molecular signature of nucleic acid sequences/biomolecules comprising transcriptomic based biomarker-I (TBB-I) comprising: 1553145_at (hypothetical protein FLJ39653), 1553575_at, 1557236_at (apolipoprotein L, 6), 1558142_at (trinucleotide repeat containing 6B), 1560752_at (F-box and WD-40 domain protein 2), 1565614_at (Zinc finger protein 337), 1567100_at (Dachshund homolog 1 (Drosophila)), 200068_s_at (calnexin /// calnexin), 201031_s_at (heterogeneous nuclear ribonucleoprotein H1 (H)), 202646_s_at (cold shock domain containing E1, RNA-binding), 205758_at (CD8a molecule /// CD8a molecule), 206188_at (zinc finger protein 623), 212637_s_at (WW domain containing E3 ubiquitin protein ligase 1), 212920_at, 213317_at (chloride intracellular channel 5), 213619_at (Heterogeneous nuclear ribonucleoprotein H1 (H)), 215443_at (thyroid stimulating hormone receptor), 216198_at (activating transcription factor 7 interacting protein), 217870_s_at (cytidylate kinase), 218087_s_at (sorbin and SH3 domain containing 1), 222145_at (CDNA: FLJ23572 fis, clone LNG12403), 223577_x_at (PRO1073 protein), 224321_at (transmembrane protein with EGF-like and two follistatin-like domains 2), 224373_s_at (IQ motif and WD repeats 1), 224644_at (CDNA clone IMAGE:5278517), 226173_at (ornithine aminotransferase-like 1), 226773_at (CDNA FLJ35131 fis, clone PLACE6008824), 226880_at (nuclear casein kinase and cyclin-dependent kinase substrate 1), 228980_at (ring finger and FYVE-like domain containing 1), 229569_at (CDNA clone IMAGE:5263455), 231735_s_at (PRO1073 protein), 233765_at (Hypothetical LOC197135), 235803_at (Cytokine receptor-like factor 3), 236131_at (CDNA clone IMAGE:6622963), 236953_s_at (similar to RIKEN cDNA 8030451K01), 240544_at (Zinc finger, AN1-type domain 3), 240971-x-at (Cullin 4A), 244042_x_at (Similar to retinoic acid receptor responder (tazarotene induced) 2), complementary sequences, fragments, alleles, variants and gene products thereof; assessing the probability of identification of each component gene in each sample; assigning each to a class; and, differentiating between idiopathic cardiomyopathy and myocarditis.

In another preferred embodiment, a method of diagnosing heart disease or myocarditis comprises identifying in a biological sample from a patient a molecular signature of nucleic acid sequences/biomolecules comprising transcriptomic based biomarker-II (TBB-II) comprising: 1552419_s_at (tubulin tyrosine ligase-like family, member 10), 1553212_at (keratin 78), 1555124_at (hypothetical protein MGC40574), 1556192_x_at (Metastasis suppressor 1), 1556320_at (Stomatin (EPB72)-like 1), 1556510_at (CDNA clone. IMAGE:4796864), 1558484_s_at (leucine rich repeat containing 27), 1565662_at (Mucin 6, oligomeric mucus/gel-forming), 1567410_at (zinc finger protein 135), 1568513_x_at (Protease, serine, 1 (trypsin 1)), 1570408_at (Serine/threonine kinase 24 (STE20 homolog, yeast)), 203307_at (guanine nucleotide binding protein-like 1), 204581_at (CD22 molecule, myelin associated glycoprotein), 205586_x_at (VGF nerve growth factor inducible), 206333_at (musashi homolog 1 (Drosophila)), 207004_at (B-cell CLL/lymphoma 2), 210059_s_at (mitogen-activated protein kinase 13), 210228_at (colony stimulating factor 2 (granulocyte-macrophage)), 210384_at (protein arginine methyltransferase 2), 210923_at (solute carrier family 1 (glutamate transporter), member 7), 211024_s_at (thyroid transcription factor 1 /// thyroid transcription factor 1), 211062_s_at (carboxypeptidase Z /// carboxypeptidase Z), 211096_at (pre-B-cell leukemia transcription factor 2), 211181_x_at (runt-related transcription factor 1 (acute myeloid leukemia 1; aml1 oncogene)), 211710_x_at (ribosomal protein L4 /// ribosomal protein L4), 213096_at (transmembrane and coiled-coil domain family 2), 213121_at (small nuclear ribonucleoprotein 70kDa polypeptide (RNP antigen)), 213242_x_at (KIAA0284), 213568_at (odd-skipped related 2 (Drosophila)), 213770_at kinase suppressor of ras 1 (214171_s_at (U2 small nuclear RNA auxiliary factor 2), 216116_at (NCK interacting protein with SH3 domain), 216427_at (CDNA: FLJ22786 fis, clone KAIA2150), 21 6820_at, 217054_at (CDNA FLJ39484 fis, clone PROST2014925), 217180_at (Hypothetical protein similar to KIAA0187 gene product), 217182_at (mucin 5AC, oligomeric mucus/gel-forming), 217322_x_at, 217430_x_at (collagen, type I, alpha 1), 219070_s_at (motile sperm domain containing 3), 219425_at (sulfotransferase family 4A, member 1), 221663_x_at (histamine receptor H3), 22]684_s_at (Nyctalopin), 223974_at (hypothetical protein MGC11082), 226640_at (diacylglycerol lipase beta), 228074_at (hypothetical protein LOC162073), 229191_at (tubulin folding cofactor D), 229257_at (KIAA1856 protein), 229335_at (immunoglobulin superfamily, member 4C), 229358_at (Indian hedgehog homolog (Drosophila)), 230341_x_at (ADAM metallopeptidase with thrombospondin type 1 motif, 10), 230693_at (ATPase, Ca⁺⁺ transporting, cardiac muscle, fast twitch 1), 230768_at (FERM, RhoGEF and pleckstrin domain protein 2), 231510_at (GLI-Kruppel family member GLI2), 231629_x_at (Kallikrein-related peptidase 3), 231998_at, 233794_at (Single stranded DNA binding protein 3), 233974_s_at (family with sequence similarity 129, member B), 234495_at (kallikrein-related peptidase 15), 234637_at (keratin associated protein 4-5), 234881_at, 235568_at (chromosome 19 open reading frame 59), 235600_at (Transcribed locus), 236496_at (degenerative spermatocyte homolog 2, lipid desaturase (Drosophila)), 237087_at (Chromosome 14 open reading frame 105), 237144_at (Latent transforming growth factor beta binding protein 3), 237398_at (Transcribed locus), 237547_at (Hypothetical protein LOC728730), 237679_at (tripartite motif-containing 66), 238267_s_at, 238445_x_at (mannosyl (alpha-1,6-)-glycoprotein beta-1,6-N-acetyl-glucosaminyltransferase, isozyme B), 239026_x_at (centaurin, gamma 3), 239463_at (Transcribed locus), 239756_at (MAD 1 mitotic arrest deficient-like 1 (yeast)), 240039_at (Transcribed locus /// Transcribed locus), 240147_at (hypothetical protein MGC11257), 240517_at (Cystathionine-beta-synthase), 241270_at (Rhomboid 5 homolog 2 (Drosophila)), 241431_at, 242365_at (Coiled-coil domain containing 32), 243297_at (Vacuolar protein sorting 13 homolog D (S. *cerevisiae*))*,* 243497_at (Transcribed locus), 243766_s_at (TEA domain family member 2), 43934_at (G protein-coupled receptor 137), complementary sequences, fragments, alleles, variants and gene products thereof; assessing the probability of identification of each component gene in each sample; assigning each to a class; and, diagnosing heart disease or myocarditis.

In another preferred embodiment, a method of diagnosing heart disease or myocarditis comprises identifying in a biological sample from a patient a molecular signature of nucleic acid sequences/biomolecules comprising transcriptomic based biomarker-III (TBB-III) comprising: 1553212_at (keratin 78), 1557236_at (apolipoprotein L), 1558142_at (trinucleotide repeat containing 6B), 1558484_s_at (leucine rich repeat containing 27), 1565614_at (Zinc finger protein 337), 1565662_at (Mucin 6, oligomeric mucus/gel-forming), 1567100_at (Dachshund homolog 1 (Drosophila)), 203307_at (guanine nucleotide binding protein-like 1), 205758_at (CD8a molecule /// CD8a molecule), 206333_at (musashi homolog 1 (Drosophila)), 212920_at, 213242_x_at (KIAA0284), 213619_at (Heterogeneous nuclear ribonucleoprotein H1 (H)), 213770_at (kinase suppressor of ras 1), 214171_s_at (U2 small nuclear RNA auxiliary factor 2), 215443_at (thyroid stimulating hormone receptor), 216198_at (activating transcription factor 7 interacting protein), 216427_at (CDNA: FLJ22786 fis, clone KAIA2150), 217054_at (CDNA FLJ39484 fis, clone PROST2014925), 217182_at (mucin 5AC, oligomeric mucus/gel-forming), 217322_x_at, 219425_at (sulfotransferase family 4A, member 1), 222145_at (CDNA: FLJ23572 fis, clone LNG12403), 229191_at (tubulin folding cofactor D), 229569_at (CDNA clone IMAGE:5263455), 231629_x_at (Kallikrein-related peptidase 3), 233765_at (Hypothetical LOC197135), 233794_at (Single stranded DNA binding protein 3), 233974_s_at (family with sequence similarity 129, member B), 234495_at (kallikrein-related peptidase 15), 235568_at (chromosome 19 open reading frame 59), 235803_at (Cytokine receptor-like factor 3), 236496_at (degenerative spermatocyte homolog 2, lipid desaturase (Drosophila)), 236953_s_at, 238445_x_at (mannosyl (alpha-1,6-)-glycoprotein beta-1,6-N-acetyl-glucosaminyltransferase, isozyme B), 239463_at (Transcribed locus), 240544_at (Zinc finger, AN1-type domain 3), 243766_s_at (TEA domain family member 2), and 244042_x_at, complementary sequences, fragments, alleles, variants and gene products thereof; assessing the probability of identification of each component gene in each sample; assigning each to a class; and, diagnosing heart disease or myocarditis.

In another preferred embodiment a kit comprises a transcriptomic based biomarker-I (TBB-I): 1553212_at (keratin 78), 1557236_at (apolipoprotein L), 1558142_at (trinucleotide repeat containing 6B), I558484_s_at (leucine rich repeat containing 27), 1565614_at (Zinc finger protein 337), 1565662_at (Mucin 6, oligomeric mucus/gel-forming), 1567100_at (Dachshund homolog 1 (Drosophila)), 203307_at (guanine nucleotide binding protein-like 1), 205758_at (CD8a molecule /// CD8a molecule), 206333_at (musashi homolog 1 (Drosophila)), 212920_at, 213242_x_at (KIAA0284), 213619_at (Heterogeneous nuclear ribonucleoprotein H1 (H)), 213770_at (kinase suppressor of ras 1), 214171_s_at (U2 small nuclear RNA auxiliary factor 2), 215443_at (thyroid stimulating hormone receptor), 216198_at (activating transcription factor 7 interacting protein), 216427_at (CDNA: FLJ22786 fis, clone KAIA2150), 217054_at (CDNA FLJ39484 fis, clone PROST2014925), 217182_at (mucin 5AC, oligomeric mucus/gel-forming), 217322_x_at, 219425_at (sulfotransferase family 4A, member 1), 222145_at (CDNA: FLJ23572 fis, clone LNG12403), 229191_at (tubulin folding cofactor D), 229569_at (CDNA clone IMAGE:5263455), 231629_x_at (Kallikrein-related peptidase 3), 233765_at (Hypothetical LOC197135), 233794_at (Single stranded DNA binding protein 3), 233974_s_at (family with sequence similarity 129, member B), 234495_at (kallikrein-related peptidase 15), 235568_at (chromosome 19 open reading frame 59), 235803_at (Cytokine receptor-like factor 3), 236496_at (degenerative spermatocyte homolog 2, lipid desaturase (Drosophila)), 236953_s_at, 238445_x_at (mannosyl (alpha-1,6-)-glycoprotein beta-1,6-N-acetyl-glucosaminyltransferase, isozyme B), 239463_at (Transcribed locus), 240544_at (Zinc finger, AN1-type domain 3), 43766_s_at (TEA domain family member 2), and 244042_x_at complementary sequences, fragments, alleles, variants and gene products thereof.

In another preferred embodiment, a kit comprises a transcriptomic based biomarker-II (TBB-II): 1552419_s_at (tubulin tyrosine ligase-like family, member 10), 1553212_at (keratin 78), 1555124_at (hypothetical protein MGC40574), 1556192_x_at (Metastasis suppressor 1), 1556320_at (Stomatin (EPB72)-like 1), 1556510_at (CDNA clone IMAGE:4796864), 1558484_s_at (leucine rich repeat containing 27), 1565662_at (Mucin 6, oligomeric mucus/gel-forming), 1567410_at (zinc finger protein 135), 1568513_x_at (Protease, serine, 1 (trypsin 1)), 1570408_at (Serine/threonine kinase 24 (STE20 homolog, yeast)), 203307_at (guanine nucleotide binding protein-like 1), 204581_at (CD22 molecule, myelin associated glycoprotein), 20S586_x_at (VGF nerve growth factor inducible), 206333_at (musashi homolog 1 (Drosophila)), 207004_at (B-cell CLL/lymphoma 2), 210059_s_at (mitogen-activated protein kinase 13), 210228_at (colony stimulating factor 2 (granulocyte-macrophage)), 210384_at (protein arginine methyltransferase 2), 210923_at (solute carrier family 1 (glutamate transporter), member 7), 211024_s_at (thyroid transcription factor 1 /// thyroid transcription factor 1), 211062_s_at (carboxypeptidase Z /// carboxypeptidase Z), 211096_at (pre-B-cell leukemia transcription factor 2), 211181_x_at (runt-related transcription factor 1 (acute myeloid leukemia 1; aml1 oncogene)), 211710_x_at (ribosomal protein L4 /// ribosomal protein L4), 213096_at (transmembrane and coiled-coil domain family 2), 213121_at (small nuclear ribonucleoprotein 70kDa polypeptide (RNP antigen)), 213242_x_at (KIAA0284), 213568_at (odd-skipped related 2 (Drosophila)), 213770_at kinase suppressor of ras 1 (214171_s_at (U2 small nuclear RNA auxiliary factor 2), 216116_at (NCK interacting protein with SH3 domain), 216427_at (CDNA: FLJ22786 fis, clone KAIA2150), 216820_at, 217054_at (CDNA FLJ39484 fis, clone PROST2014925), 217180_at (Hypothetical protein similar to KIAA0187 gene product), 217182_at (mucin 5AC, oligomeric mucus/gel-forming), 217322_x_at, 217430_x_at (collagen, type I, alpha 1), 219070_s_at (motile sperm domain containing 3), 219425_at (sulfotransferase family 4A, member 1), 221663_x_at (histamine receptor H3), 221684_s_at (Nyctalopin), 223974_at (hypothetical protein MGC11082), 226640_at (diacylglycerol lipase beta), 228074_at (hypothetical protein LOC162073), 229191_at (tubulin folding cofactor D), 229257_at (KIAA1856 protein), 229335_at (immunoglobulin superfamily, member 4C), 229358_at (Indian hedgehog homolog (Drosophila)), 230341_x_at (ADAM metallopeptidase with thrombospondin type 1 motif, 10), 230693_at (ATPase, Ca⁺⁺ transporting, cardiac muscle, fast twitch 1), 230768_at (FERM, RhoGEF and pleckstrin domain protein 2), 231510_at (GLI-Kruppel family member GLI2), 231629_x_at (Kallikrein-related peptidase 3), 231998_at, 233794_at (Single stranded DNA binding protein 3), 233974_s_at (family with sequence similarity 129, member B), 234495_at (kallikrein-related peptidase 15), 234637_at (keratin associated protein 4-5), 234881_at, 235568_at (chromosome 19 open reading frame 59), 235600_at (Transcribed locus), 236496_at (degenerative spermatocyte homolog 2, lipid desaturase (Drosophila)), 237087_at (Chromosome 14 open reading frame 105), 237144_at (Latent transforming growth factor beta binding protein 3), 237398_at (Transcribed locus), 237547_at (Hypothetical protein LOC728730), 237679_at (tripartite motif-containing 66), 238267_s_at, 238445_x_at (mannosyl (alpha-1,6-)-glycoprotein beta-1,6-N-acetyl-glucosaminyltransferase, isozyme B), 239026_x_at (centaurin, gamma 3), 239463_at (Transcribed locus), 239756_at (MAD1 mitotic arrest deficient-like 1 (yeast)), 240039_at (Transcribed locus /// Transcribed locus), 240147_at (hypothetical protein MGC11257), 240517_at (Cystathionine-beta-synthase), 241270_at (Rhomboid 5 homolog 2 (Drosophila)), 24I431_at, 242365_at (Coiled-coil domain containing 32), 243297_at (Vacuolar protein sorting 13 homolog D (*S*, *cerevisiae*)), 243497_at (Transcribed locus), 243766_s_at (TEA domain family member 2), 43934_at (G protein-coupled receptor 137), complementary sequences, fragments, alleles, variants and gene products thereof.

In another preferred embodiment, a kit comprises a transcriptomic based biomarker-III (TBB-III): 1553212_at (keratin 78), 1557236_at (apolipoprotein L), 1558142_at (trinucleotide repeat containing 6B), 1558484_s_at (leucine rich repeat containing 27), 1565614_at (Zinc finger protein 337), 1565662_at (Mucin 6, oligomeric mucus/gel-forming), 1567100_at (Dachshund homolog 1 (Drosophila)), 203307_at (guanine nucleotide binding protein-like 1), 205758_at (CD8a molecule /// CD8a molecule), 206333_at (musashi homolog 1 (Drosophila)), 212920_at, 213242_x_at (KIAA0284), 213619_at (Heterogeneous nuclear ribonucleoprotein H1 (H)), 213770_at (kinase suppressor of ras 1), 214171_s_at (U2 small nuclear RNA auxiliary factor 2), 215443_at (thyroid stimulating hormone receptor), 216198_at (activating transcription factor 7 interacting protein), 216427_at (CDNA: FLJ22786 fis, clone KAIA2150), 217054_at (CDNA FLJ39484 fis, clone PROST2014925), 217182_at (mucin 5AC, oligomeric mucus/gel-forming), 217322_x_at, 219425_at (sulfotransferase family 4A, member 1), 222145_at (CDNA: FLJ23572 fis, clone LNG12403), 229191_at (tubulin folding cofactor D), 229569_at (CDNA clone IMAGE:5263455), 231629_x_at (Kallikrein-related peptidase 3), 233765_at (Hypothetical LOC197135), 233794_at (Single stranded DNA binding protein 3), 233974_s_at (family with sequence similarity 129, member B), 234495_at (kallikrein-related peptidase 15), 235568_at (chromosome 19 open reading frame 59), 235803_at (Cytokine receptor-like factor 3), 236496_at (degenerative spermatocyte homolog 2, lipid desaturase (Drosophila)), 236953_s_at, 238445_x_at (mannosyl (alpha-1,6-)-glycoprotein beta-1,6-N-acetyl-glucosaminyltransferase, isozyme B), 239463_at (Transcribed locus), 240544_at (Zinc finger, AN1-type domain 3), 243766_s_at (TEA domain family member 2), and 244042_x_at complementary sequences, fragments, alleles, variants and gene products thereof.

In another embodiment, a cell expresses one or more biomolecules comprising the biomarker TBB-I.

In another embodiment, a cell expresses one or more biomolecules comprising the biomarker TBB-II.

In another embodiment, a cell expresses one or more biomolecules comprising the biomarker TBB-III

In another preferred embodiment, a vector encodes one or more biomolecules comprising TBB-I.

In another preferred embodiment, a vector encodes one or more biomolecules comprising TBB-II.

In another preferred embodiment, a vector encodes one or more biomolecules comprising TBB-III. Aspects

Aspects of the invention are described *infra.*

### BRIEF DESCRIPTION OF THE DRAWINGS

The invention is pointed out with particularity in the appended claims. The above and further advantages of this invention may be better understood by referring to the following description taken in conjunction with the accompanying drawings, in which:

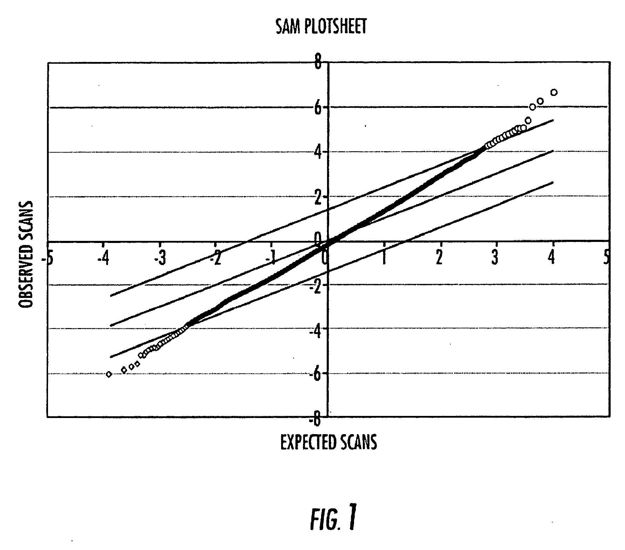

Figure 1 shows a SAM plot of samples from patients with idiopathic cardiomyopathy versus patients with myocarditis: Red color denotes genes that were significantly upregulated in patients with myocarditis, green color denotes genes that were significantly downregulated. The outer blue lines represent the positive and negative cutoff points that were chosen with the delta value. 134 genes were significantly different between the two groups, when a delta value of 1.41 was chosen (FDR=0.49). We reduced this subset of genes to 122 candidate genes with a q-value of 0% that we used for hierarchical clustering and PAM analysis.

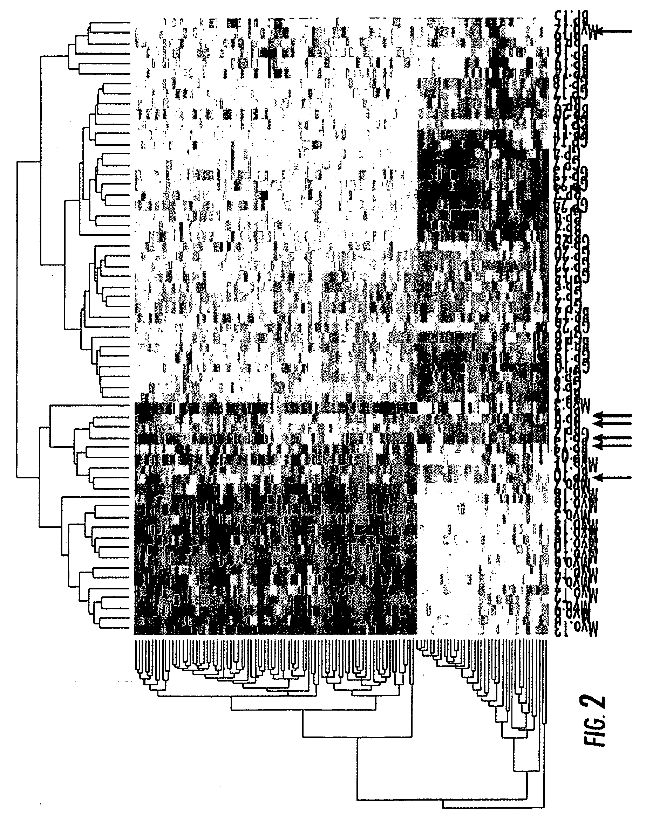

Figure 2 shows a heatmap created by hierarchical clustering using 122 candidate genes: Samples from patients with myocarditis are labeled "Myo-", samples from patients with idiopathic cardiomyopathy are labeled either with "GP-" or "BP-". Each column represents a sample and each line corresponds to a gene, for which the IDs from Affymetrix are listed on the right side. For further details about the gene annotations see Table 1. A red color means low expression of the gene, whereas a blue color demonstrates high gene expression levels. Six samples were grouped wrong.

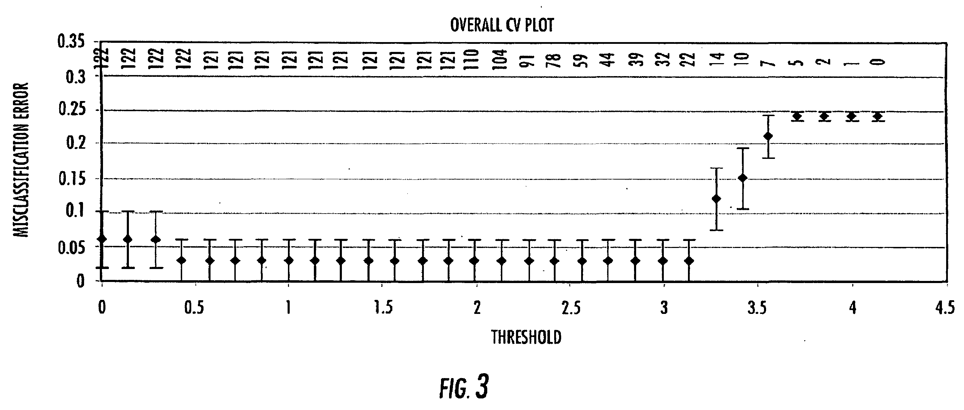

Figure 3 is a graph showing the misclassification error of the training set. The classifier was trained on 8 samples from patients with myocarditis and 25 samples from patients with idiopathic cardiomyopathy. After increasing the threshold to 3.2 and reducing the genes of the classifier to less than 22, the misclassification error increased dramatically.

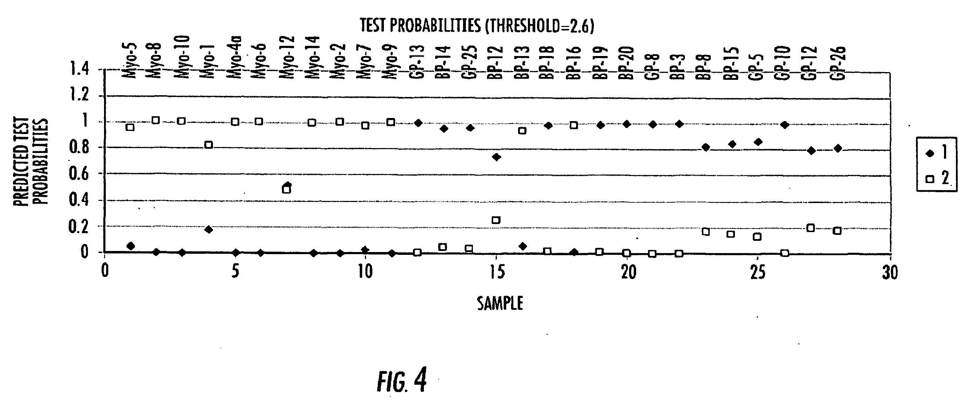

Figure 4 is a graph showing results from the "39 genes classifier" for myocarditis: This graph visualizes the calculated probabilities for each class after the "39 genes molecular signature" was applied with a threshold of 2.6. The probability can be read from the y-axis. Group 1 represents the samples from patients with idiopathic cardiomyopathy, group 2 represents the samples from patients with myocarditis.

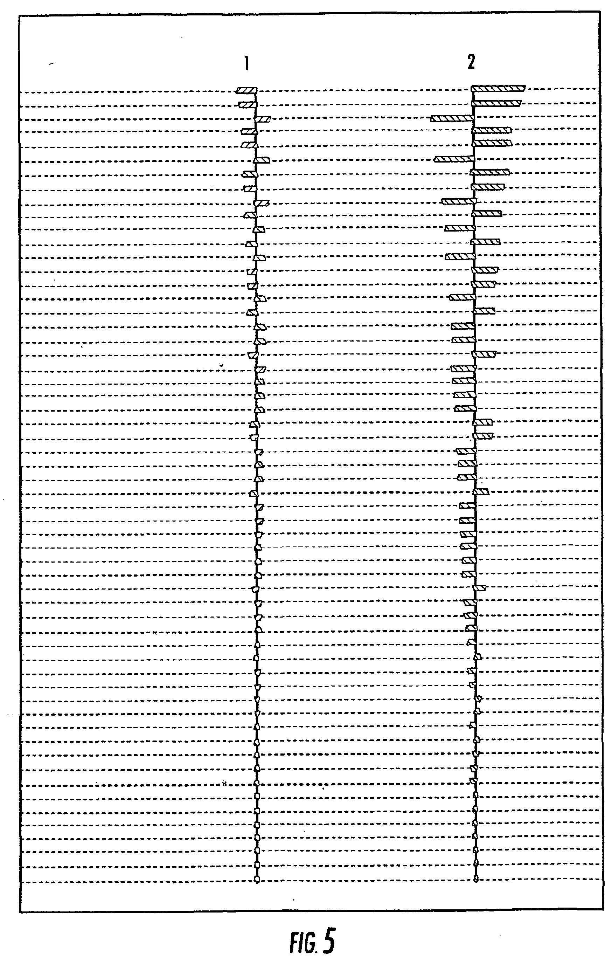

Figure 5 shows the nearest shrunken centroid of the "39 genes classifier": The centroids were calculated in PAM from the average expression for each gene in each class divided by the within-class standard deviation for that gene. The nearest shrunken centroid classification "shrinks" each of the class centroids toward the overall centroid for all classes by the threshold. Nearest centroid classification takes the gene expression profile of a new sample, and compares it to each of these class centroids. The class whose centroid that it is closest to, in squared distance, is the predicted class for that new sample. Each line in the graph represents a gene. The red centroid characterizes group 1 (idiopathic cardiomyopathy), the green centroid characterizes group 2 (myocarditis). Upregulation is illustrated as a vector to the right, downregulation as a vector to the left on the graph.

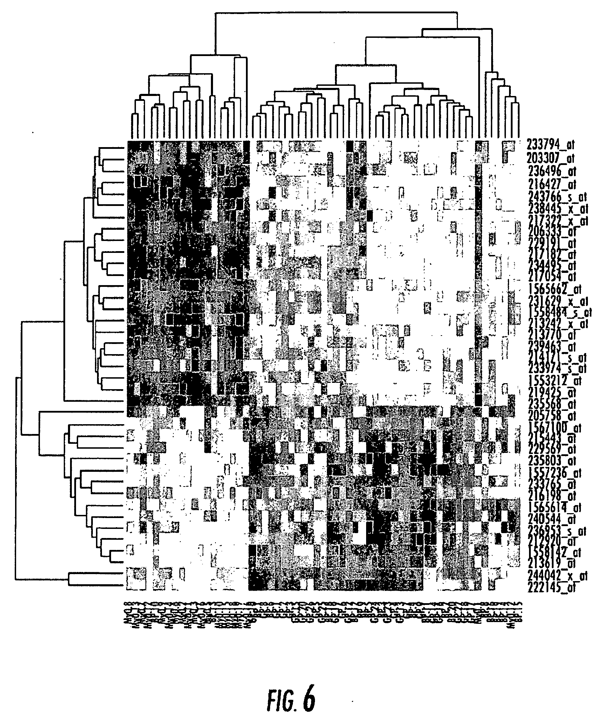

Figure 6 shows a heatmap of the "39 genes transcriptomic biomarker": This heatmap was created by the same unsupervised clustering method as figure 2.

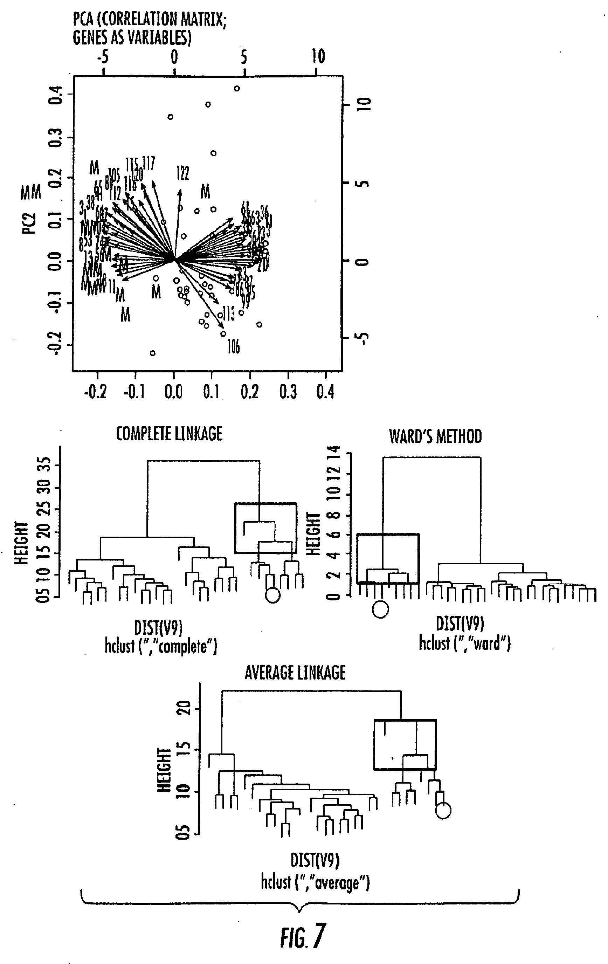

Figure 7 shows the Principal Components Analysis (PCA) and various Cluster Algorithms in samples of myocarditis vs. other types of cardiomyopathy: To illustrate the contribution of each of the 122 genes (FC>1.2; q<0.1%) to every phenotype, we performed PCA (n=61). We used correlation matrix with genes as variables. Less significant genes are denoted with vectors close to the center and correspond to genes that were excluded using PAM analysis. Myocarditis samples were labeled with "M", samples from patients with other forms of cardiomyopathy were labeled with "O". Genes that were overexpressed are labeled with serial numbers and are clustered with the corresponding class. All myocarditis samples, except two, were clearly grouped together. We further tested the robustness of the reduced set of 39 genes with 3 different types of clustering algorithms (Ward's method, complete linkage and average linkage). Myocarditis samples are highlighted in red - incorrectly classified samples are encircled in blue. All methods achieved the same accuracy as PAM (n=33, 97% accuracy).

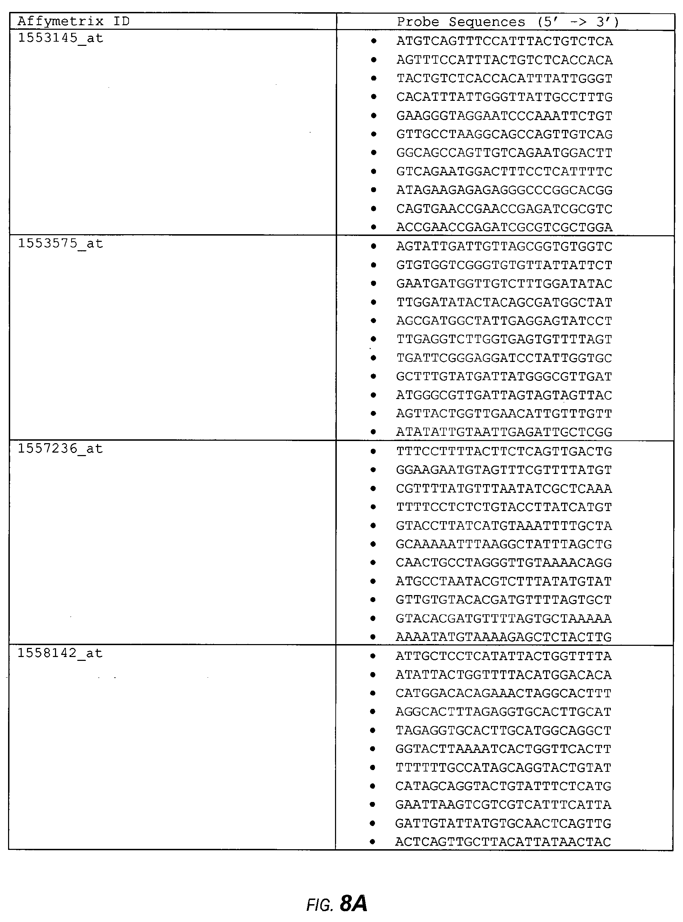

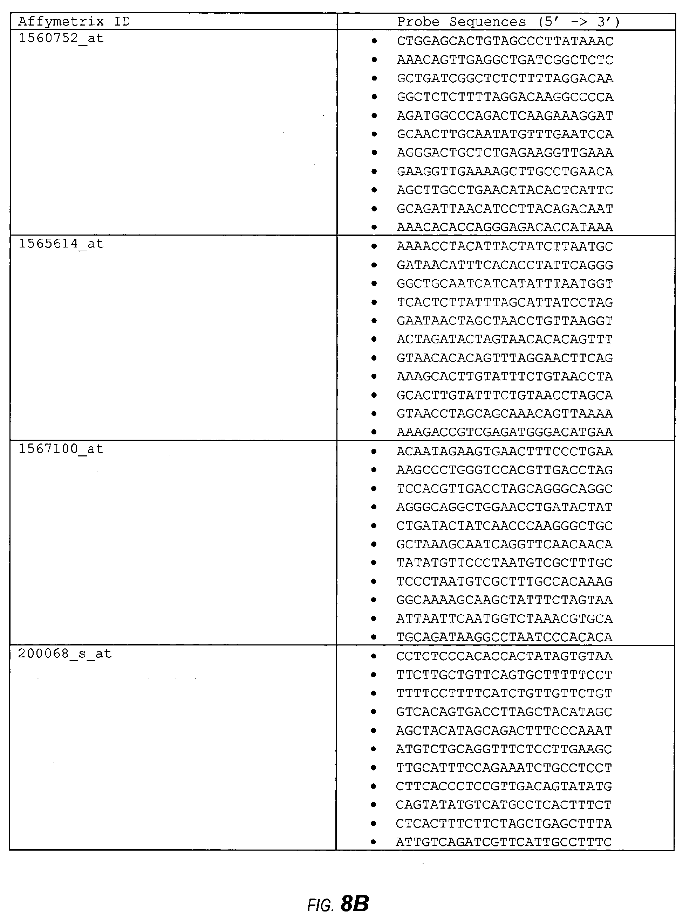

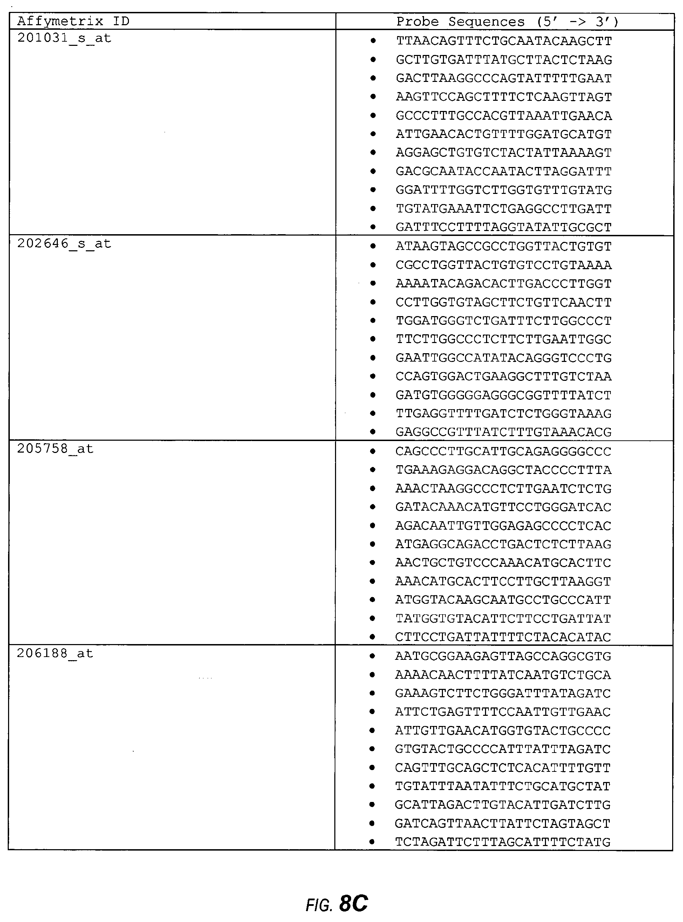

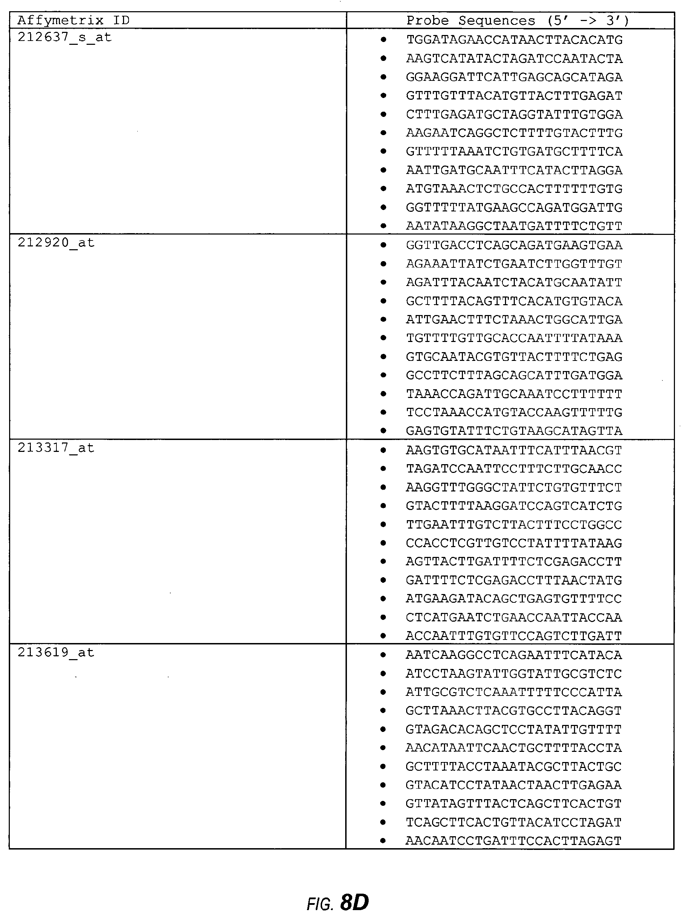

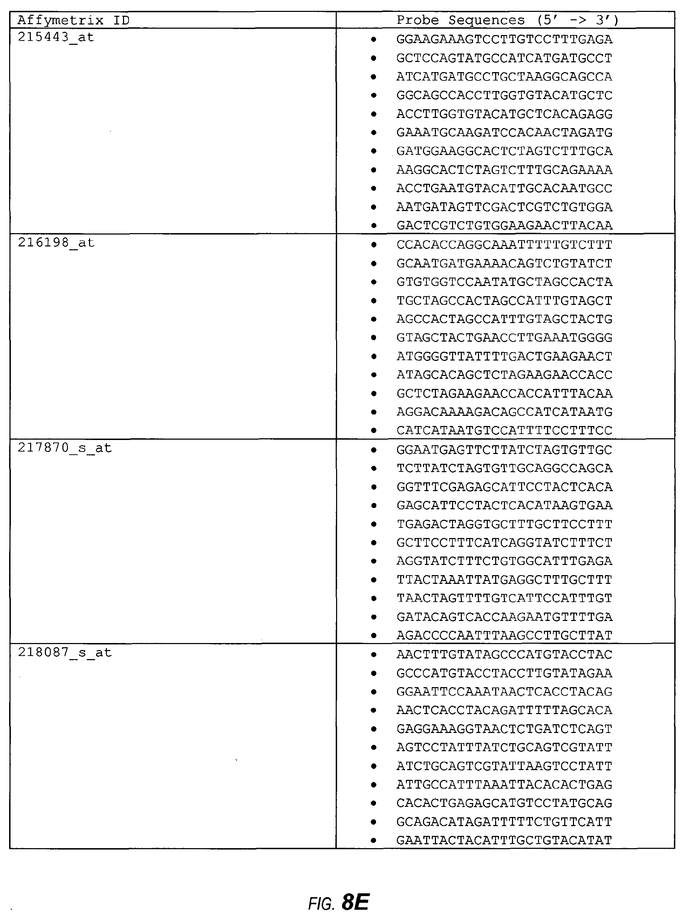

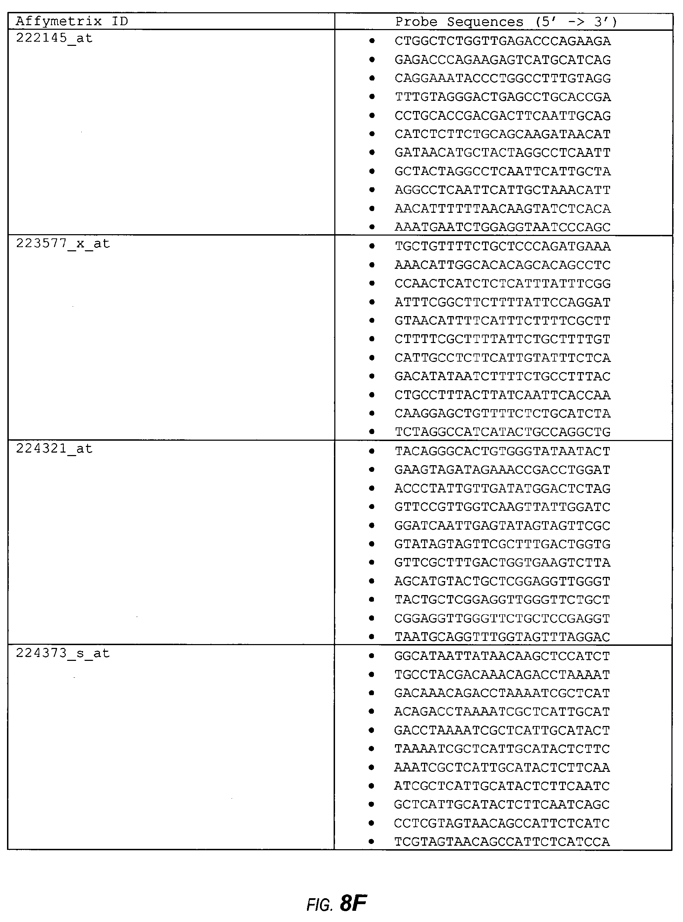

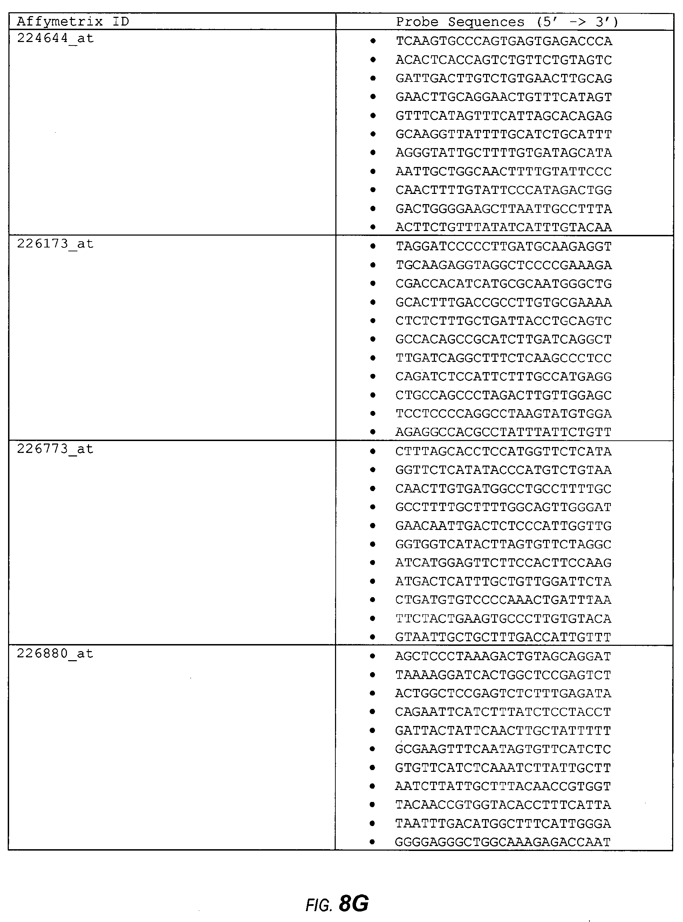

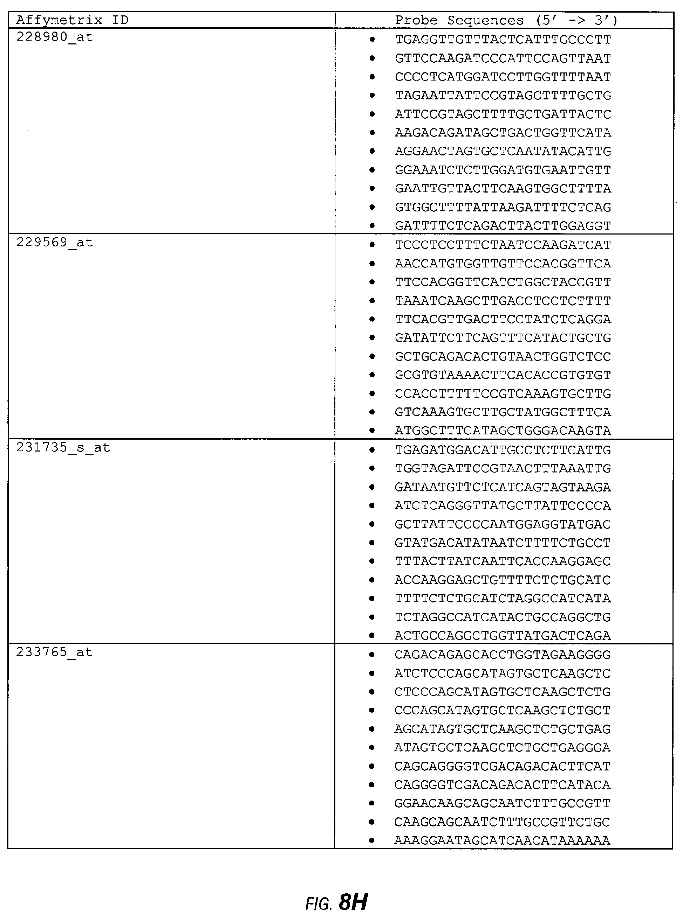

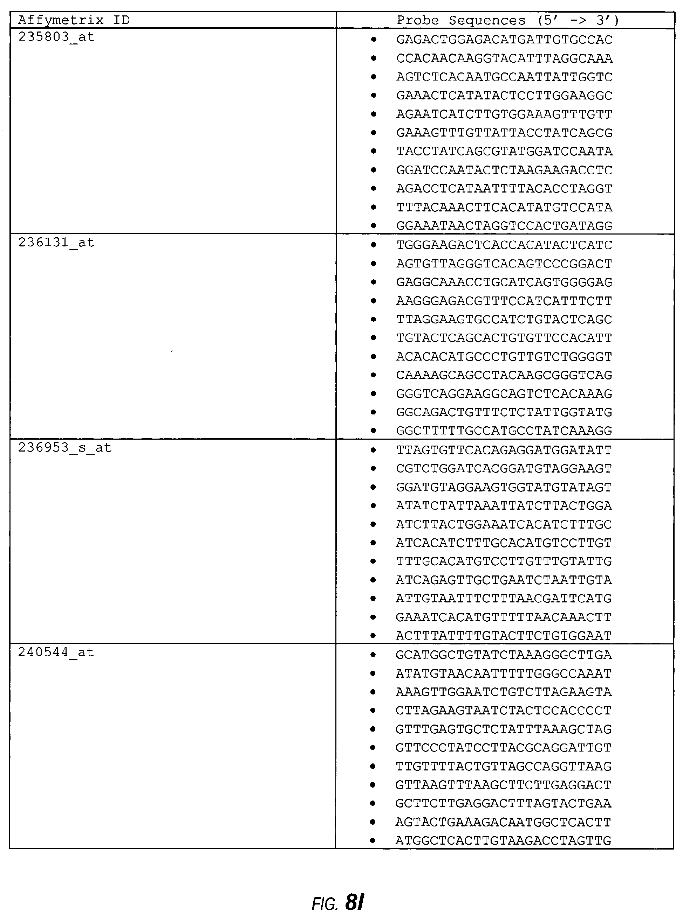

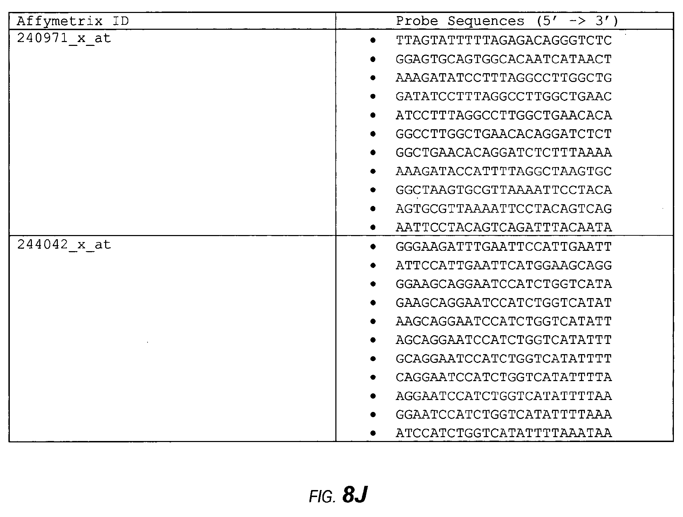

Figure 8A to 8J show Affymetrix IDs and corresponding probe sequences.

### DETAILED DESCRIPTION

The invention comprises molecular signatures that function as a very sensitive diagnostic biomarker for myocarditis. Myocarditis is a common disease that is estimated to cause up to 30% of dilated cardiomyopathy, even in patients initially asymptomatic. Myocarditis can also present as sudden cardiac death and affects individuals of all ages. In childhood, myocarditis causes a greater percentage of heart failure than in adulthood. The fact that the majority of viral induced cases pass in a clinically unapparent course, points out the significance of finding more reliable biomarkers than standard diagnostic tools which are currently available, e.g. ECG, cardiac enzymes and immunohistochemistry.

Current standard diagnostic tools for myocarditis (ECG, cardiac enzymes, immunohistochemistry) are not always reliable enough and many patients undergo clinically unapparent courses without getting treated. Especially in pediatrics it is crucial to detect myocarditis in an early stage as a fatal course has been observed many times in children.

### Definitions

In accordance with the present invention and as used herein, the following terms are defined with the following meanings, unless explicitly stated otherwise.

As used herein, "a", "an," and "the" include plural references unless the context clearly dictates otherwise.

As used herein, a "molecular signature" or "signature" or "biomarker" or "transcriptomic based biomarker" are used interchangeably herein and refers to all the biomolecules identified in Tables 1, 2, and 4. Thus, Table 1 comprising the biomolecules listed therein, represents one biomarker or molecular signature; Table 2 comprising the biomolecules listed therein, represents another one biomarker or molecular signature; and so forth. As more biomolecules are discovered, each newly identified biomolecules can be assigned to any one or more biomarker or molecular signature. Each biomolecule can also be removed, reassigned or reallocated to a molecular signature. Thus, in some embodiments the molecular signature comprises at least ten biomolecules. The ten biomolecules are selected from the genes identified herein, or from newly identified biomolecules. The biomarkers from Table 1 comprising the 38 upregulated genes is termed TBB-I for brevity. The biomarker comprising the biomolecules in Table 2 are termed TBB-II for brevity. The biomarker comprising the biomolecules in Table 4 is termed TBB-III for brevity. When making a diagnosis it is desirable to detect at least 10 or more biomolecules. Any one of TBB-I, TBB-II and TBB- III or combinations thereof can be used in the diagnosis of myocarditis. Any one of TBB-I, TBB-II and TBB-III or combinations thereof can be used in the diagnosis of myocarditis and idiopathic cardiomyopathy and differentiating between the two conditions.

The term "biomolecule" refers to DNA, RNA (including mRNA, rRNA, tRNA and tmRNA), nucleotides, nucleosides, analogs, polynucleotides, peptides and any combinations thereof.

A base "position" as used herein refers to the location of a given base or nucleotide residue within a nucleic acid.

As used herein, the term "array" refers to an ordered spatial arrangement, particularly an arrangement of immobilized biomolecules.

As used herein, the term "addressable array" refers to an array wherein the individual elements have precisely defined x and y coordinates, so that a given element at a particular position in the array can be identified.

As used herein, the terms "probe" and "biomolecular probe" refer to a biomolecule used to detect a complementary biomolecule. Examples include antigens that detect antibodies, oligonucleotides that detect complimentary oligonucleotides, and ligands that detect receptors. Such probes are preferably immobilized on a microelectrode comprising a substrate.

As used herein, the terms "bioarray," "biochip" and "biochip array" refer to an ordered spatial arrangement of immobilized biomolecules on a microelectrode arrayed on a solid supporting substrate. Preferred probe molecules include aptamers, nucleic acids, oligonucleotides, peptides, ligands, antibodies and antigens; peptides and proteins are the most preferred probe species. Biochips, as used in the art, encompass substrates containing arrays or microarrays, preferably ordered arrays and most preferably ordered, addressable arrays, of biological molecules that comprise one member of a biological binding pair. Typically, such arrays are oligonucleotide arrays comprising a nucleotide sequence that is complementary to at least one sequence that may be or is expected to be present in a biological sample. Alternatively, and preferably, proteins, peptides or other small molecules can be arrayed in such biochips for performing, *inter alia,* immunological analyses (wherein the arrayed molecules are antigens) or assaying biological receptors (wherein the arrayed molecules are ligands, agonists or antagonists of said receptors).

Expression/amount of a gene, biomolecule, or biomarker in a first sample is at a level "greater than" the level in a second sample if the expression level/amount of the gene or biomarker in the first sample is at least about 1 time, 1.2 times, 1.5 times, 1.75 times, 2 times, 3 times , 4 times, 5 times, 6 times, 7 times, 8 times, 9 times, 10 times, 20 times, 30 times, the expression level/amount of the gene or biomarker in the second sample or a normal sample. Expression levels/amounts can be determined based on any suitable criterion known in the art, including but not limited to mRNA, cDNA, proteins, protein fragments and/or gene copy. Expression levels/amounts can be determined qualitatively and/or quantitatively.

By the term "modulate," it is meant that any of the mentioned activities, are, e.g., increased, enhanced, increased, agonized (acts as an agonist), promoted, decreased, reduced, suppressed blocked, or antagonized (acts as an agonist). Modulation can increase activity more than 1-fold, 2-fold, 3-fold, 5-fold, 10-fold, 100-fold, etc., over baseline values. Modulation can also decrease its activity below baseline values.

An "allele" or "variant" is an alternative form of a gene. Variants may result from at least one mutation in the nucleic acid sequence and may result in altered mRNAs or in polypeptides whose structure or function may or may not be altered. Any given natural or recombinant gene may have none, one, or many allelic forms. Common mutational changes that give rise to variants are generally ascribed to natural deletions, additions, or substitutions of nucleotides. Each of these types of changes may occur alone, or in combination with the others, one or more times in a given sequence.

The term, "complementary" means that two sequences are complementary when the sequence of one can bind to the sequence of the other in an anti-parallel sense wherein the 3'-end of each sequence binds to the 5'-end of the other sequence and each A, T(U), G, and C of one sequence is then aligned with a T(U), A, C, and G, respectively, of the other sequence. Normally, the complementary sequence of the oligonucleotide has at least 80% or 90%, preferably 95%, most preferably 100%, complementarity to a defined sequence. Preferably, alleles or variants thereof can be identified. A BLAST program also can be employed to assess such sequence identity.

The term "complementary sequence" as it refers to a polynucleotide sequence, relates to the base sequence in another nucleic acid molecule by the base-pairing rules. More particularly, the term or like term refers to the hybridization or base pairing between nucleotides or nucleic acids, such as, for instance, between the two strands of a double stranded DNA molecule or between an oligonucleotide primer and a primer binding site on a single stranded nucleic acid to be sequenced or amplified. Complementary nucleotides are, generally, A and T (or A and U), or C and G. Two single stranded RNA or DNA molecules are said to be substantially complementary when the nucleotides of one strand, optimally aligned and compared and with appropriate nucleotide insertions or deletions, pair with at least about 95% of the nucleotides of the other strand, usually at least about 98%, and more preferably from about 99 % to about 100%. Complementary polynucleotide sequences can be identified by a variety of approaches including use of well-known computer algorithms and software, for example the BLAST program.

As used herein, the term "aptamer" or "selected nucleic acid binding species" shall include non-modified or chemically modified RNA or DNA. The method of selection may be by, but is not limited to, affinity chromatography and the method of amplification by reverse transcription (RT) or polymerase chain reaction (PCR).

As used herein, the term "signaling aptamer" shall include aptamers with reporter molecules, preferably a fluorescent dye, appended to a nucleotide in such a way that upon conformational changes resulting from the aptamer's interaction with a ligand, the reporter molecules yields a differential signal, preferably a change in fluorescence intensity.

As used herein, the term "fragment or segment", as applied to a nucleic acid sequence, gene or polypeptide, will ordinarily be at least about 5 contiguous nucleic acid bases (for nucleic acid sequence or gene) or amino acids (for polypeptides), typically at least about 10 contiguous nucleic acid bases or amino acids, more typically at least about 20 contiguous nucleic acid bases or amino acids, usually at least about 30 contiguous nucleic acid bases or amino acids, preferably at least about 40 contiguous nucleic acid bases or amino acids, more preferably at least about 50 contiguous nucleic acid bases or amino acids, and even more preferably at least about 60 to 80 or more contiguous nucleic acid bases or amino acids in length. "Overlapping fragments" as used herein, refer to contiguous nucleic acid or peptide fragments which begin at the amino terminal end of a nucleic acid or protein and end at the carboxy terminal end of the nucleic acid or protein. Each nucleic acid or peptide fragment has at least about one contiguous nucleic acid or amino acid position in common with the next nucleic acid or peptide fragment, more preferably at least about three contiguous nucleic acid bases or amino acid positions in common, most preferably at least about ten contiguous nucleic acid bases amino acid positions in common.

"Biological samples" include solid and body fluid samples. Preferably, the sample is obtained from heart. However, the biological samples used in the present invention can include cells, protein or membrane extracts of cells, blood or biological fluids such as ascites fluid or brain fluid (e.g., cerebrospinal fluid). Examples of solid biological samples include, but are not limited to, samples taken from tissues of the central nervous system, bone, breast, kidney, cervix, endometrium, head/neck, gallbladder, parotid gland, prostate, pituitary gland, muscle, esophagus, stomach, small intestine, colon, liver, spleen, pancreas, thyroid, heart, lung, bladder, adipose, lymph node, uterus, ovary, adrenal gland, testes, tonsils and thymus. Examples of "body fluid samples" include, but are not limited to blood, serum, semen, prostate fluid, seminal fluid, urine, saliva, sputum, mucus, bone marrow, lymph, and tears.

"Sample" is used herein in its broadest sense. A sample comprising polynucleotides, polypeptides, peptides, antibodies and the like may comprise a bodily fluid; a soluble fraction of a cell preparation, or media in which cells were grown; a chromosome, an organelle, or membrane isolated or extracted from a cell; genomic DNA, RNA, or cDNA, polypeptides, or peptides in solution or bound to a substrate; a cell; a tissue; a tissue print; a fingerprint, skin or hair; and the like.

"Diagnostic" means identifying the presence or nature of a pathologic condition. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

### Transcriptomic BiomarkerlMolecular Signatures

In a preferred embodiment, a biomarker (TBB-I) comprises nucleic acid sequences/biomolecules comprising: 1553145_at (hypothetical protein FLJ39653), 1553575_at, 1557236_at (apolipoprotein L, 6), 1558142_at (trinucleotide repeat containing 6B), 1560752_at (F-box and WD-40 domain protein 2), 1565614_at (Zinc finger protein 337), 1567100_at (Dachshund homolog 1 (Drosophila)), 200068_s_at (calnexin///calnexin), 201031_s_at (heterogeneous nuclear ribonucleoprotein H1 (H)), 202646_s_at (cold shock domain containing E1, RNA-binding), 205758_at (CD8a molecule /// CD8a molecule), 206188_at (zinc finger protein 623), 212637_s_at (WW domain containing E3 ubiquitin protein ligase 1), 212920_at, 213317_at (chloride intracellular channel 5), 213619_at (Heterogeneous nuclear ribonucleoprotein H1 (H)), 215443_at (thyroid stimulating hormone receptor), 216198_at (activating transcription factor 7 interacting protein), 217870_s_at (cytidylate kinase), 218087_s_at (sorbin and SH3 domain containing 1), 222145_at (CDNA: FLJ23572 fis, clone LNG12403), 223577_x_at (PRO1073 protein), 224321_at (transmembrane protein with EGF-like and two follistatin-like domains 2), 224373_s_at (IQ motif and WD repeats 1), 224644_at (CDNA clone IMAGE:5278517), 226173_at (ornithine aminotransferase-like 1), 226773_at (CDNA FLJ35131 fis, clone PLACE6008824), 226880_at (nuclear casein kinase and cyclin-dependent kinase substrate 1), 228980_at (ring finger and FYVE-like domain containing 1), 229569_at (CDNA clone IMAGE:5263455), 231735_s_at (PRO1073 protein), 233765_at (Hypothetical LOC197135), 235803_at (Cytokine receptor-like factor 3), 236131_at (CDNA clone IMAGE:6622963), 236953_s_at (similar to RIKEN cDNA 8030451K01), 240544_at (Zinc finger, AN 1-type domain 3), 240971_x_at (Cullin 4A), 244042_x_at (Similar to retinoic acid receptor responder (tazarotene induced) 2), complementary sequences, fragments, alleles, variants and gene products thereof.

In another preferred embodiment, the biomolecules comprising: 1553145_at (hypothetical protein FLJ39653), 1553575_at, 1557236_at (apolipoprotein L, 6), 1558142_at (trinucleotide repeat containing 6B), 1560752_at (F-box and WD-40 domain protein 2), 1565614_at (Zinc finger protein 337), 1567100_at (Dachshund homolog 1 (Drosophila)), 200068_s_at (calnexin /// calnexin), 201031_s_at (heterogeneous nuclear ribonucleoprotein H1 (H)), 202646_s_at (cold shock domain containing E1, RNA-binding), 205758_at (CD8a molecule /// CD8a molecule), 206188_at (zinc finger protein 623), 212637_s_at (WW domain containing E3 ubiquitin protein ligase 1), 212920_at, 213317_at (chloride intracellular channel 5), 213619_at (Heterogeneous nuclear ribonucleoprotein H1 (H)), 215443_at (thyroid stimulating hormone receptor), 216198_at (activating transcription factor 7 interacting protein), 217870_s_at (cytidylate kinase), 218087_s_at (sorbin and SH3 domain containing 1), 222145_at (CDNA: FLJ23572 fis, clone LNG12403), 223577_x_at (PRO1073 protein), 224321_at (transmembrane protein with EGF-like and two follistatin-like domains 2), 224373_s_at (IQ motif and WD repeats 1), 224644_at (CDNA clone IMAGE:5278517), 226173_at (ornithine aminotransferase-like 1), 226773_at (CDNA FLJ35131 fis, clone PLACE6008824), 226880_at (nuclear casein kinase and cyclin-dependent kinase substrate 1), 228980_at (ring finger and FYVE-like domain containing 1), 229569_at (CDNA clone IMAGE:5263455), 231735_s_at (PRO1073 protein), 233765_at (Hypothetical LOC197135), 235803_at (Cytokine receptor-like factor 3), 236131_at (CDNA clone IMAGE:6622963), 236953_s_at (similar to RIKEN cDNA 8030451K01), 240544_at (Zinc finger, AN1-type domain 3), 240971_x_at (Cullin 4A), 244042_x_at (Similar to retinoic acid receptor responder (tazarotene induced) 2) are upregulated in patients with myocarditis as compared to normal subjects. In some embodiments at least ten biomolecules are upregulated.

In another preferred embodiment, a transcriptomic biomarker (TBB- II) comprises biomolecules comprising: 1552419_s_at (tubulin tyrosine ligase-like family, member 10), 1553212_at (keratin 78), 1555124_at (hypothetical protein MGC40574), 1556192_x_at (Metastasis suppressor 1), 1556320_at (Stomatin (EPB72)-like 1), 1556510_at (CDNA clone IMAGE:4796864), 1558484_s_at (leucine rich repeat containing 27), 1565662_at (Mucin 6, oligomeric mucus/gel-forming), 1567410_at (zinc finger protein 135), 1568513_x_at (Protease, serine, 1 (trypsin 1)), 1570408_at (Serine/threonine kinase 24 (STE20 homolog, yeast)), 203307_at (guanine nucleotide binding protein-like 1), 204581_at (CD22 molecule, myelin associated glycoprotein), 205586_x_at (VGF nerve growth factor inducible), 206333_at (musashi homolog 1 (Drosophila)), 207004_at (B-cell CLL/lymphoma 2), 210059_s_at (mitogen-activated protein kinase 13), 210228_at (colony stimulating factor 2 (granulocyte-macrophage)), 210384_at (protein arginine methyltransferase 2), 210923_at (solute carrier family 1 (glutamate transporter), member 7), 211024_s_at (thyroid transcription factor 1 /// thyroid transcription factor 1), 211062_s_at (carboxypeptidase Z /// carboxypeptidase Z), 211096_at (pre-B-cell leukemia transcription factor 2), 211181_x_at (runt-related transcription factor 1 (acute myeloid leukemia 1; aml1 oncogene)), 211710_x_at (ribosomal protein L4 /// ribosomal protein L4), 213096_at (transmembrane and coiled-coil domain family 2), 213121_at (small nuclear ribonucleoprotein 70kDa polypeptide (RNP antigen)), 213242_x_at (KIAA0284), 213568_at (odd-skipped related 2 (Drosophila)), 213770_at (kinase suppressor of ras 1), 214171_s_at (U2 small nuclear RNA auxiliary factor 2), 216116_at (NCK interacting protein with SH3 domain), 216427_at ((CDNA: FLJ22786 fis, clone KAIA2150), 216820_at, 217054_at (CDNA FLJ39484 fis, clone PROST2014925), 217180_at (Hypothetical protein similar to KIAA0187 gene product), 217182_at (mucin 5AC, oligomeric mucus/gel-forming), 217322_x_at, 217430_x_at (collagen, type I, alpha 1), 219070_s_at (motile sperm domain containing 3), 219425_at (sulfotransferase family 4A, member 1), 221663_x_at (histamine receptor H3), 221684_s_at (Nyctalopin), 223974_at (hypothetical protein MGC11082), 226640_at (diacylglycerol lipase beta), 228074_at (hypothetical protein LOC162073), 229191_at (tubulin folding cofactor D), 229257_at (KIAA1856 protein), 229335_at (immunoglobulin superfamily, member 4C), 229358_at (Indian hedgehog homolog (Drosophila)), 230341_x_at (ADAM metallopeptidase with thrombospondin type 1 motif, 10), 230693_at (ATPase, Ca⁺⁺ transporting, cardiac muscle, fast twitch 1), 230768_at (FERM, RhoGEF and pleckstrin domain protein 2), 231510_at (GLI-Kruppel family member GLI2), 231629_x_at (Kallikrein-related peptidase 3), 231998_at, 233794_at (Single stranded DNA binding protein 3), 233974_s_at (family with sequence similarity 129, member B), 234495_at (kallikrein-related peptidase 15), 234637_at (keratin associated protein 4-5), 234881_at, 235568_at (chromosome 19 open reading frame 59), 235600_at (Transcribed locus), 236496_at (degenerative spermatocyte homolog 2, lipid desaturase (Drosophila)), 237087_at (Chromosome 14 open reading frame 105), 237144_at (Latent transforming growth factor beta binding protein 3), 237398_at (Transcribed locus), 237547_at (Hypothetical protein LOC728730), 237679_at (tripartite motif-containing 66), 238267_s_at, 238445_x_at (mannosyl (alpha-1,6-)-glycoprotein beta-1,6-N-acetyl-glucosaminyltransferase, isozyme B), 239026_x_at (centaurin, gamma 3), 239463_at (Transcribed locus), 239756_at (MAD1 mitotic arrest deficient-like 1 (yeast)), 240039_at (Transcribed locus /// Transcribed locus), 240147_at (hypothetical protein MGC11257), 240517_at (Cystathionine-beta-synthase), 241270_at (Rhomboid 5 homolog 2 (Drosophila)), 241431_at, 242365_at (Coiled-coil domain containing 32), 243297_at (Vacuolar protein sorting 13 homolog D (S. *cerevisiae*)), 243497_at (Transcribed locus), 243766_s_at (TEA domain family member 2), 43934_at (G protein-coupled receptor 137), complementary sequences, fragments, alleles, variants and gene products thereof.

In another preferred embodiment, the biomolecules comprising the transcriptomic biomarker (TBB-II) complementary sequences, fragments, alleles, variants and gene products thereof are downregulated in a patient suffering from myocarditis as compared to a normal subject.

In another preferred embodiment, a biomarker (TBB-III) comprises nucleic acid sequences/biomolecules comprising: 1553212_at (keratin 78), 1557236_at (apolipoprotein L), 1558142_at (trinucleotide repeat containing 6B), 1558484_s_at (leucine rich repeat containing 27), 1565614_at (Zinc finger protein 337), 1565662_at (Mucin 6, oligomeric mucus/gel-forming), 1567100_at (Dachshund homolog 1 (Drosophila)), 203307_at (guanine nucleotide binding protein-like 1), 205758_at (CD8a molecule /// CD8a molecule), 206333_at (musashi homolog 1 (Drosophila)), 212920_at, 213242_x_at (KIAA0284), 213619_at (Heterogeneous nuclear ribonucleoprotein H1 (H)), 213770_at (kinase suppressor of ras 1), 214171_s_at (U2 small nuclear RNA auxiliary factor 2), 215443_at (thyroid stimulating hormone receptor), 216198_at (activating transcription factor 7 interacting protein), 216427_at (CDNA: FLJ22786 fis, clone KAIA2150), 217054_at (CDNA FLJ39484 fis, clone PROST2014925), 217182_at (mucin 5AC, oligomeric mucus/gel-forming), 217322_x_at, 219425_at (sulfotransferase family 4A, member 1), 222145_at (CDNA: FLJ23572 fis, clone LNG12403), 229191_at (tubulin folding cofactor D), 229569_at (CDNA clone IMAGE:5263455), 231629_x_at (Kallikrein-related peptidase 3), 233765_at (Hypothetical LOC197135), 233794_at (Single stranded DNA binding protein 3), 233974_s_at (family with sequence similarity 129, member B), 234495_at (kallikrein-related peptidase 15), 235568_at (chromosome 19 open reading frame 59), 235803_at (Cytokine receptor-like factor 3), 236496_at (degenerative spermatocyte homolog 2, lipid desaturase (Drosophila)), 236953_s_at, 238445_x_at (mannosyl (alpha-1,6-)-glycoprotein beta-1,6-N-acetyl-glucosaminyltransferase, isozyme B), 239463_at (Transcribed locus), 240544_at (Zinc finger, AN1-type domain 3), 243766_s_at (TEA domain family member 2), and 244042_x_at complementary sequences, fragments, alleles, variants and gene products thereof.

In another preferred embodiment, the detection of any one of the biomarkers, TBB-I, TBB-II and TBB-III or combinations thereof, are diagnostic of myocarditis, idiopathic cardiomyopathy, heart diseases and disorders thereof.
In another preferred embodiment, the detection of at least ten biomolecules in TBB-I is diagnostic of myocarditis, idiopathic cardiomyopathy, heart diseases and disorders thereof.

In another preferred embodiment, the detection of at least ten molecules in TBB-II is diagnostic of myocarditis, idiopathic cardiomyopathy, heart diseases and disorders thereof. Since the biomolecules in TBB-II are downregulated in myocarditis, when the term "detection" is used with respect to the biomolecules of TBB-II, will refer to the downregulation of the biomolecules as compared to the expression in normal or healthy cells or subjects.

In another preferred embodiment, the detection of at least ten molecules in TBB-III is diagnostic of myocarditis, idiopathic cardiomyopathy, heart diseases and disorders thereof.

In another preferred embodiment, the detection in a cell or patient of the biomolecules, complementary sequences, fragments, alleles, variants and gene products thereof, is diagnostic of myocarditis, idiopathic cardiomyopathy, heart diseases and disorders thereof. Preferably, the biomolecule sequences, complementary sequences, fragments, alleles, variants and gene products thereof, are modulated at levels by at least between 1%, 2%, 5%, 10% in a cell or patient as compared to levels in a normal cell or normal subject; more preferably, the gene biomarker sequences, complementary sequences, fragments, alleles, variants and gene products thereof, are modulated by about 50% in a cell or a patient as compared to levels in a normal cell or normal subject; more preferably, the gene biomarker sequences, complementary sequences, fragments, alleles, variants and gene products thereof, are modulated by about 75% in a cell or a patient as compared to levels in a normal cell or normal subject. The term "modulated" refers to an increase or decrease in level, concentration, amount etc, as compared to a normal cell or normal healthy subject.

In another preferred embodiment, a biochip comprises a molecular signature comprising TBB-I, TBB-II, or TBB-III, complementary sequences, fragments, alleles, variants and gene products thereof.

In another preferred embodiment, a biochip comprises at least ten biomolecules selected from the biomolecules comprising TBB-I, TBB-II, or TBB-III, complementary sequences, fragments, alleles, variants and gene products thereof. Any ten or more can be selected from either one of TBB-I, TBB-II, or TBB-III biomarkers or selected form combinations thereof. There is nothing to preclude adding any newly identified biomarkers or any other known biomarkers or biomolecules thereof.
In another preferred embodiment, a method of differentiating between idiopathic cardiomyopathy and myocarditis, comprises: identifying in a biological sample from a patient a molecular signature comprising a transcriptomic based biomarker TBB-I, TBB-II and TBB-III.

In another preferred embodiment, a method of differentiating between idiopathic cardiomyopathy and myocarditis, comprises identifying in a biological sample from a patient a molecular signature comprising the biomolecules of transcriptomic based biomarker (TBB) complementary sequences, fragments, alleles, variants and gene products thereof; assessing the probability of identification of each component gene in each sample; and assigning each to a class.

In another preferred embodiment, the biomolecules are selected from TBB-I, TBB-II, and TBB-III, or combinations thereof.
In a preferred embodiment, phenotype specificity is identified by creating a classifier in a training set comprising about 66% of data obtained, with subsequent validation in a test set comprising about 33% of data obtained and defining a phenotype specific nearest shrunken centroid for classification.

In another preferred embodiment, a method of diagnosing heart disease or myocarditis comprises identifying in a biological sample from a patient a molecular signature comprising a transcriptomic based biomarker (TBB), TBB-I, TBB-II and TBB-III.
In another preferred embodiment, a method of diagnosing heart disease or myocarditis comprises detection of at least ten biomolecules, complementary sequences, fragments, alleles, variants and gene products thereof in a sample; assessing the probability of identification of each component gene in each sample; assigning each to a class; and, diagnosing heart disease or myocarditis.

### Alternative Methods and Materials for Identifying Molecular Signatures or Transcriptomic Biomarkers

*Detection of Nucleic Acids and Proteins as Markers:* In preferred embodiments, each biomarker is detected on chip based methods such as those described in detail in the examples which follow. In order to provide accurate diagnosis of cardiac disorders and diseases, for example, heart failure, myocarditis, idiopathic cardiomyopathy and the like. Other methods are also known in the art and one or more methods can be utilized.

The methods and assays disclosed herein are directed to the examination of expression of transcriptomic biomarkers in a mammalian tissue or cell sample, wherein the determination of that expression of one or more such transcriptomic biomarkers is predictive of prognostic outcome or diagnostic of cardiac and cardiovascular diseases and disorders, such as for example, myocarditis, Coronary Heart Disease, angina, Acute Coronary Syndrome, Aortic Aneurysm and Dissection, arrhythmias, Cardiomyopathy, Congenital Heart Disease, congestive heart failure or chronic heart failure, pericarditis, and the like. The Molecular signatures or Transcriptomic biomarker comprise the biomolecules identified in Tables 1, 2, and 4. When making a diagnosis it is desirable to detect at least 10 or more biomolecules. Any one of TBB-I (Table 1), TBB-II (Table 2), TBB-III (Table 4) or combinations thereof can be used in the diagnosis of myocarditis. Any one of TBB-I, TBB-II and TBB- III or combinations thereof can be used in the diagnosis of myocarditis and idiopathic cardiomyopathy and differentiating between the two conditions.

Preferred embodiments in the identification of biomolecules, analytical methods etc, are described in detail in the Examples which follow.

*Microarrays:* In general, using nucleic acid microarrays, test and control mRNA samples from test and control tissue samples are reverse transcribed and labeled to generate cDNA probes. The probes are then hybridized to an array of nucleic acids immobilized on a solid support. The array is configured such that the sequence and position of each member of the array is known. For example, a selection of genes that have potential to be expressed in certain disease states may be arrayed on a solid support. Hybridization of a labeled probe with a particular array member indicates that the sample from which the probe was derived expresses that gene. Differential gene expression analysis of disease tissue can provide valuable information. Microarray technology utilizes nucleic acid hybridization techniques and computing technology to evaluate the mRNA expression profile of thousands of genes within a single experiment. (see, e.g., WO 01/75166 published Oct. 11, 2001; (See, for example, U.S. Pat. No. 5,700,637, U.S. Pat. No. 5,445,934, and U.S. Pat. No. 5,807,522, Lockart, Nature Biotechnology, 14:1675-1680 (1996); Cheung, V. G. et al., Nature Genetics 21(Suppl):15-19 (1999) for a discussion of array fabrication). DNA microarrays are miniature arrays containing gene fragments that are either synthesized directly onto or spotted onto glass or other substrates. Thousands of genes are usually represented in a single array. A typical microarray experiment involves the following steps: 1) preparation of fluorescently labeled target from RNA isolated from the sample, 2) hybridization of the labeled target to the microarray, 3) washing, staining, and scanning of the array, 4) analysis of the scanned image and 5) generation of gene expression profiles. Currently two main types of DNA microarrays are being used: oligonucleotide (usually 25 to 70 mers) arrays and gene expression arrays containing PCR products prepared from cDNAs. In forming an array, oligonucleotides can be either prefabricated and spotted to the surface or directly synthesized on to the surface (*in situ*)*.* The Affymetrix GENECHIP™ system is a commercially available microarray system which comprises arrays fabricated by direct synthesis of oligonucleotides on a glass surface.

*Probe*/*Gene Arrays*: Oligonucleotides, usually 25 mers, are directly synthesized onto a glass wafer by a combination of semiconductor-based photolithography and solid phase chemical synthesis technologies. Each array contains up to 400,000 different oligonucleotides and each oligonucleotide is present in millions of copies. Since oligonucleotide probes are synthesized in known locations on the array, the hybridization patterns and signal intensities can be interpreted in terms of gene identity and relative expression levels by the Affymetrix Microarray Suite software. Each gene is represented on the array by a series of different oligonucleotide probes. Each probe pair consists of a perfect match oligonucleotide and a mismatch oligonucleotide. The perfect match probe has a sequence exactly complimentary to the particular gene and thus measures the expression of the gene. The mismatch probe differs from the perfect match probe by a single base substitution at the center base position, disturbing the binding of the target gene transcript. This helps to determine the background and nonspecific hybridization that contributes to the signal measured for the perfect match oligonucleotide. The Microarray Suite software subtracts the hybridization intensities of the mismatch probes from those of the perfect match probes to determine the absolute or specific intensity value for each probe set. Probes are chosen based on current information from GenBank and other nucleotide repositories. The sequences are believed to recognize unique regions of the 3' end of the gene. A GeneChip Hybridization Oven ("rotisserie" oven) is used to carry out the hybridization of up to 64 arrays at one time. The fluidics station performs washing and staining of the probe arrays. It is completely automated and contains four modules, with each module holding one probe array. Each module is controlled independently through Microarray Suite software using preprogrammed fluidics protocols. The scanner is a confocal laser fluorescence scanner which measures fluorescence intensity emitted by the labeled cRNA bound to the probe arrays. The computer workstation with Microarray Suite software controls the fluidics station and the scanner. Microarray Suite software can control up to eight fluidics stations using preprogrammed hybridization, wash, and stain protocols for the probe array. The software also acquires and converts hybridization intensity data into a presence/absence call for each gene using appropriate algorithms. Finally, the software detects changes in gene expression between experiments by comparison analysis and formats the output into .txt files, which can be used with other software programs for further data analysis.

The expression of a selected biomarker may also be assessed by examining gene deletion or gene amplification. Gene deletion or amplification may be measured by any one of a wide variety of protocols known in the art, for example, by conventional Southern blotting, Northern blotting to quantitate the transcription of mRNA (Thomas, Proc. Natl. Acad. Sci. USA, 77:5201-5205 (1980)), dot blotting (DNA analysis), or *in situ* hybridization (e.g., FISH), using an appropriately labeled probe, cytogenetic methods or comparative genomic hybridization (CGH) using an appropriately labeled probe.

*Detection of Polypeptides*: In another embodiment, a polypeptide corresponding to a marker is detected. A preferred agent for detecting a polypeptide is an antibody or aptamer capable of binding to a polypeptide corresponding to a marker of the invention, preferably an antibody with a detectable label. Antibodies can be polyclonal, or more preferably, monoclonal. An intact antibody, or a fragment thereof, e.g., Fab or F(ab')₂ can be used. The term "labeled", with regard to the probe or antibody, is intended to encompass direct-labeling of the probe or antibody by coupling, i.e., physically linking, a detectable substance to the probe or antibody, as well as indirect-labeling of the probe or antibody by reactivity with another reagent that is directly-labeled. Examples of indirect labeling include detection of a primary antibody using a fluorescently-labeled secondary antibody and end-labeling of a DNA probe with biotin such that it can be detected with fluorescently-labeled streptavidin.

Proteins from individuals can be isolated using techniques that are well-known to those of skill in the art. The protein isolation methods employed can, e.g., be such as those described in Harlow & Lane (1988), *supra.* A variety of formats can be employed to determine whether a sample contains a protein that binds to a given antibody. Expression of various biomarkers in a sample can be analyzed by a number of methodologies, many of which are known in the art and understood by the skilled artisan, including but not limited to, immunohistochemical and/or Western analysis, quantitative blood based assays (as for example Serum ELISA) (to examine, for example, levels of protein expression), biochemical enzymatic activity assays, *in situ* hybridization, Northern analysis and/or PCR analysis of mRNAs, as well as any one of the wide variety of assays that can be performed by gene and/or tissue array analysis. Typical protocols for evaluating the status of genes and gene products are found, for example in Ausubel et al. eds., 1995, Current Protocols In Molecular Biology, Units 2 (Northern Blotting), 4 (Southern Blotting), 15 (Immunoblotting) and 18 (PCR Analysis). A skilled artisan can readily adapt known protein/antibody detection methods for use in determining whether cells express a marker of the present invention and the relative concentration of that specific polypeptide expression product in blood or other body tissues.

In such alternative methods, a sample may be contacted with an antibody specific for said biomarker under conditions sufficient for an antibody-biomarker complex to form, and then detecting said complex. The presence of the biomarker may be detected in a number of ways, such as by Western blotting and ELISA procedures for assaying a wide variety of tissues and samples, including plasma or serum. A wide range of immunoassay techniques using such an assay format are available, see, e.g., U.S. Pat. Nos. 4,016,043, 4,424,279 and 4,018,653. These include both single-site and two-site or "sandwich" assays of the non-competitive types, as well as in the traditional competitive binding assays. These assays also include direct binding of a labeled antibody to a target biomarker.

Sandwich assays are among the most useful and commonly used assays. A number of variations of the sandwich assay technique exist. Briefly, in a typical forward assay, an unlabelled antibody is immobilized on a solid substrate, and the sample to be tested brought into contact with the bound molecule. After a suitable period of incubation, for a period of time sufficient to allow formation of an antibody-antigen complex, a second antibody specific to the antigen, labeled with a reporter molecule capable of producing a detectable signal is then added and incubated, allowing time sufficient for the formation of another complex of antibody-antigen-labeled antibody. Any unreacted material is washed away, and the presence of the antigen is determined by observation of a signal produced by the reporter molecule. The results may either be qualitative, by simple observation of the visible signal, or may be quantitated by comparing with a control sample containing known amounts of biomarker.

Variations on the forward assay include a simultaneous assay, in which both sample and labeled antibody are added simultaneously to the bound antibody. These techniques are well known to those skilled in the art, including any minor variations as will be readily apparent. In a typical forward sandwich assay, a first antibody having specificity for the biomarker is either covalently or passively bound to a solid surface. The solid surface is typically glass or a polymer, the most commonly used polymers being cellulose, polyacrylamide, nylon, polystyrene, polyvinyl chloride or polypropylene. The solid supports may be in the form of tubes, beads, discs of microplates, or any other surface suitable for conducting an immunoassay. The binding processes are well-known in the art and generally consist of cross-linking covalently binding or physically adsorbing, the polymer-antibody complex is washed in preparation for the test sample. An aliquot of the sample to be tested is then added to the solid phase complex and incubated for a period of time sufficient (e.g. 2-40 minutes or overnight if more convenient) and under suitable conditions (e.g. from room temperature to 40° C. such as between 25° C. and 32° C. inclusive) to allow binding of any subunit present in the antibody. Following the incubation period, the antibody subunit solid phase is washed and dried and incubated with a second antibody specific for a portion of the biomarker. The second antibody is linked to a reporter molecule which is used to indicate the binding of the second antibody to the molecular marker.

An alternative method involves immobilizing the target biomarkers in the sample and then exposing the immobilized target to specific antibody which may or may not be labeled with a reporter molecule. Depending on the amount of target and the strength of the reporter molecule signal, a bound target may be detectable by direct labeling with the antibody. Alternatively, a second labeled antibody, specific to the first antibody is exposed to the target-first antibody complex to form a target-first antibody-second antibody tertiary complex. The complex is detected by the signal emitted by the reporter molecule. By "reporter molecule", as used in the present specification, is meant a molecule which, by its chemical nature, provides an analytically identifiable signal which allows the detection of antigen-bound antibody. The most commonly used reporter molecules in this type of assay are either enzymes, fluorophores or radionuclide containing molecules (i.e. radioisotopes) and chemiluminescent molecules.

In the case of an enzyme immunoassay, an enzyme is conjugated to the second antibody, generally by means of glutaraldehyde or periodate. As will be readily recognized, however, a wide variety of different conjugation techniques exist, which are readily available to the skilled artisan. Commonly used enzymes include horseradish peroxidase, glucose oxidase, -galactosidase and alkaline phosphatase, amongst others. The substrates to be used with the specific enzymes are generally chosen for the production, upon hydrolysis by the corresponding enzyme, of a detectable color change. Examples of suitable enzymes include alkaline phosphatase and peroxidase. It is also possible to employ fluorogenic substrates, which yield a fluorescent product rather than the chromogenic substrates noted above. In all cases, the enzyme-labeled antibody is added to the first antibody-molecular marker complex, allowed to bind, and then the excess reagent is washed away. A solution containing the appropriate substrate is then added to the complex of antibody-antigen-antibody. The substrate will react with the enzyme linked to the second antibody, giving a qualitative visual signal, which may be further quantitated, usually spectrophotometrically, to give an indication of the amount of biomarker which was present in the sample. Alternately, fluorescent compounds, such as fluorescein and rhodamine, may be chemically coupled to antibodies without altering their binding capacity. When activated by illumination with light of a particular wavelength, the fluorochrome-labeled antibody adsorbs the light energy, inducing a state to excitability in the molecule, followed by emission of the light at a characteristic color visually detectable with a light microscope. As in the EIA, the fluorescent labeled antibody is allowed to bind to the first antibody-molecular marker complex. After washing off the unbound reagent, the remaining tertiary complex is then exposed to the light of the appropriate wavelength, the fluorescence observed indicates the presence of the molecular marker of interest. Immunofluorescence and EIA techniques are both very well established in the art. However, other reporter molecules, such as radioisotope, chemiluminescent or bioluminescent molecules, may also be employed. Methods of the invention further include protocols which examine the presence and/or expression of mRNAs, in a tissue or cell sample. Methods for the evaluation of mRNAs in cells are well known and include, for example, hybridization assays using complementary DNA probes (such as *in situ* hybridization using labeled riboprobes, Northern blot and related techniques) and various nucleic acid amplification assays (such as RT-PCR and other amplification type detection methods, such as, for example, branched DNA, SISBA, TMA and the like).

In an embodiment, the level of mRNA corresponding to the marker can be determined both by *in situ* and by *in vitro* formats in a biological sample using methods known in the art. Many expression detection methods use isolated RNA. For *in vitro* methods, any RNA isolation technique that does not select against the isolation of mRNA can be utilized for the purification of RNA from cells. See, e.g., Ausubel et al., Ed., Curr. Prot. Mol. Biol., John Wiley & Sons, NY (1987-1999). Additionally, large numbers of tissue samples can readily be processed using techniques well-known to those of skill in the art, such as, e.g., the single-step RNA isolation process of U.S. Pat. No. 4,843,155. The isolated mRNA can be used in hybridization or amplification assays that include, but are not limited to, Southern or Northern analyses, PCR analyses and probe arrays. One preferred diagnostic method for the detection of mRNA levels involves contacting the isolated mRNA with a nucleic acid molecule (probe) that can hybridize to the mRNA encoded by the gene being detected. The nucleic acid probe can be, e.g., a full-length cDNA, or a portion thereof, such as an oligonucleotide of at least 7, 15, 30, 50, 100, 250 or 500 nucleotides in length and sufficient to specifically hybridize under stringent conditions to a mRNA or genomic DNA encoding a marker of the present invention. Nucleic acid molecules for use in the diagnostic assays of the invention are set forth by the claims. Hybridization of an mRNA with the probe indicates that the marker in question is being expressed.

In one format, the mRNA is immobilized on a solid surface and contacted with a probe, for example, by running the isolated mRNA on an agarose gel and transferring the mRNA from the gel to a membrane, such as nitrocellulose. In an alternative format, the probe(s) are immobilized on a solid surface and the mRNA is contacted with the probe(s), for example, in an Affymetrix gene chip array. A skilled artisan can readily adapt known mRNA detection methods for use in detecting the level of mRNA encoded by the markers of the present invention.

Although amplification of molecules is not required in the present invention as discussed in the examples section, one of skill in the art could use amplification methods. One alternative method for determining the level of mRNA corresponding to a marker of the present invention in a sample involves the process of nucleic acid amplification, e.g., by RT-PCR (the experimental embodiment set forth in Mullis, U.S. Pat. No. 4,683,202 (1987); ligase chain reaction, self-sustained sequence replication, Guatelli et al., Proc. Natl. Acad. Sci. USA, Vol. 87, pp. 1874-1878 (1990); transcriptional amplification system, Kwoh et al., Proc. Natl. Acad. Sci. USA, Vol. 86, pp. 1173-1177 (1989); Q-Beta Replicase, Lizardi et al., Biol. Technology, Vol. 6, p. 1197 (1988); rolling circle replication, U.S. Pat. No. 5,854,033 (1988); or any other nucleic acid amplification method, followed by the detection of the amplified molecules using techniques well-known to those of skill in the art. These detection schemes are especially useful for the detection of the nucleic acid molecules if such molecules are present in very low numbers. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10-30 nucleotides in length and flank a region from about 50-200 nucleotides in length. Under appropriate conditions and with appropriate reagents, such primers permit the amplification of a nucleic acid molecule comprising the nucleotide sequence flanked by the primers.

For *in situ* methods, mRNA does not need to be isolated form the cells prior to detection. In such methods, a cell or tissue sample is prepared/processed using known histological methods. The sample is then immobilized on a support, typically a glass slide, and then contacted with a probe that can hybridize to mRNA that encodes the marker.

As an alternative to making determinations based on the absolute expression level of the marker, determinations may be based on the normalized expression level of the marker. Expression levels are normalized by correcting the absolute expression level of a marker by comparing its expression to the expression of a gene that is not a marker, e.g., a housekeeping gene that is constitutively expressed. Suitable genes for normalization include housekeeping genes, such as the actin gene or epithelial cell-specific genes. This normalization allows the comparison of the expression level in one sample, e.g., a patient sample, to another sample or between samples from different sources.

Alternatively, the expression level can be provided as a relative expression level. To determine a relative expression level of a marker, the level of expression of the marker is determined for 10 or more samples of normal versus disease biological samples, preferably 50 or more samples, prior to the determination of the expression level for the sample in question. The mean expression level of each of the genes assayed in the larger number of samples is determined and this is used as a baseline expression level for the marker. The expression level of the marker determined for the test sample (absolute level of expression) is then divided by the mean expression value obtained for that marker. This provides a relative expression level.

Preferably, the samples used in the baseline determination will be from patients who do not have the polymorphism. The choice of the cell source is dependent on the use of the relative expression level. Using expression found in normal tissues as a mean expression score aids in validating whether the marker assayed is specific (versus normal cells). In addition, as more data is accumulated, the mean expression value can be revised, providing improved relative expression values based on accumulated data.

### Antibodies and Aptamers

In a preferred embodiment, the antibodies and aptamers specifically bind each component of the biomarkers described herein. The components include the nucleic acid sequences, complementary sequences, fragments, alleles, variants and gene products thereof of each component in each biomarker.

Aptamer polynucleotides are typically single-stranded standard phosphodiester DNA (ssDNA). Close DNA analogs can also be incorporated into the aptamer as described below. A typical aptamer discovery procedure is described below:
A polynucleotide comprising a randomized sequence between "arms" having constant sequence is synthesized. The arms can include restriction sites for convenient cloning and can also function as priming sites for PCR primers. The synthesis can easily be performed on commercial instruments.

The target protein is treated with the randomized polynucleotide. The target protein can be in solution and then the complexes immobilized and separated from unbound nucleic acids by use of an antibody affinity column. Alternatively, the target protein might be immobilized before treatment with the randomized polynucleotide.

The target protein-polynucleotide complexes are separated from the uncomplexed material and then the bound polynucleotides are separated from the target protein. The bound nucleic acid can then be characterized, but is more commonly amplified, e.g. by PCR and the binding, separation and amplification steps are repeated. In many instances, use of conditions increasingly promoting separation of the nucleic acid from the target protein, e.g. higher salt concentration, in the binding buffer used in step 2) in subsequent iterations, results in identification of polynucleotides having increasingly high affinity for the target protein.

The nucleic acids showing high affinity for the target proteins are isolated and characterized. This is typically accomplished by cloning the nucleic acids using restriction sites incorporated into the arms, and then sequencing the cloned nucleic acid.

The affinity of aptamers for their target proteins is typically in the nanomolar range, but can be as low as the picomolar range. That is K_{D} is typically 1 pM to 500 nM, more typically from 1 pM to 100 nM. Apatmers having an affinity of K_{D} in the range of 1 pM to 10 nM are also useful.

Aptamer polynucleotides can be synthesized on a commercially available nucleic acid synthesizer by methods known in the art. The product can be purified by size selection or chromatographic methods.

Aptamer polynucleotides are typically from about 10 to 200 nucleotides long, more typically from about 10 to 100 nucleotides long, still more typically from about 10 to 50 nucleotides long and yet more typically from about 10 to 25 nucleotides long. A preferred range of length is from about 10 to 50 nucleotides.

The aptamer sequences can be chosen as a desired sequence, or random or partially random populations of sequences can be made and then selected for specific binding to a desired target protein by assay *in vitro.* Any of the typical nucleic acid-protein binding assays known in the art can be used, e.g. "Southwestern" blotting using either labeled oligonucleotide or labeled protein as the probe. See also U.S. Pat. No. 5,445,935 for a fluorescence polarization assay of protein-nucleic acid interaction.

Appropriate nucleotides for aptamer synthesis and their use, and reagents for covalent linkage of proteins to nucleic acids and their use, are considered known in the art. A desired aptamer-protein complex, for example, aptamer-thrombin complex can be labeled and used as a diagnostic agent *in vitro* in much the same manner as any specific protein-binding agent, e.g. a monoclonal antibody. Thus, an aptamer-protein complex can be used to detect and quantitate the amount of its target protein in a sample, e.g. a blood sample, to provide diagnosis of a disease state correlated with the amount of the protein in the sample.

A desired aptamer-target/bait molecular complex can also be used for diagnostic imaging. In imaging uses, the complexes are labeled so that they can be detected outside the body. Typical labels are radioisotopes, usually ones with short half-lives. The usual imaging radioisotopes, such as ¹²³**I,** ¹²⁴I, ¹²⁵I, ¹³¹I, ^{99m}TC, ¹⁸⁶Re, ¹⁸⁸Re, ⁶⁴Cu, ⁶⁷Cu, ²¹²Bi, ²¹³Bi, ⁶⁷Ga, ⁹⁰Y,¹¹¹In, ¹⁸F, ³H, ¹⁴C, ³⁵S or ³²P can be used. Nuclear magnetic resonance (NMR) imaging enhancers, such as gadolinium-153, can also be used to label the complex for detection by NMR. Methods and reagents for performing the labeling, either in the polynucleotide or in the protein moiety, are considered known in the art.

In a preferred embodiment, an antibody or aptamer is specific for each biomolecule of biomarker (TBB-I) comprising: 1553145_at (hypothetical protein FLJ39653), 1553575_at, 1557236_at (apolipoprotein L, 6), 1558142_at (trinucleotide repeat containing 6B), 1560752_at (F-box and WD-40 domain protein 2), 1565614_at (Zinc finger protein 337), 1567100_at (Dachshund homolog 1 (Drosophila)), 200068_s_at (calnexin /// calnexin), 201031Ts_at (heterogeneous nuclear ribonucleoprotein H1 (H)), 202646_s_at (cold shock domain containing E1, RNA-binding), 205758_at (CD8a molecule /// CD8a molecule), 206188_at (zinc finger protein 623), 212637_s_at (WW domain containing E3 ubiquitin protein ligase 1), 212920_at, 213317_art (chloride intracellular channel 5), 213619_at (Heterogeneous nuclear ribonucleoprotein H1 (H)), 215443_at (thyroid stimulating hormone receptor), 216198_at (activating transcription factor 7 interacting protein), 217870_s_at (cytidylate kinase), 218087_s_at (sorbin and SH3 domain containing 1), 222145_at (CDNA: FLJ23572 fis, clone LNG12403), 223577_x_at (PRO1073 protein), 224321_at (transmembrane protein with EGF-like and two follistatin-like domains 2), 224373_s_at (IQ motif and WD repeats 1), 224644_at (CDNA clone IMAGE:5278517), 226173_at (ornithine aminotransferase-like 1), 226773_at (CDNA FLJ35131 fis, clone PLACE6008824), 226880_at (nuclear casein kinase and cyclin-dependent kinase substrate 1), 228980_at (ring finger and FYVE-like domain containing 1), 229569_at (CDNA clone IMAGE:5263455), 231735_s_at (PRO1073 protein), 233765_at (Hypothetical LOCI 97135), 235803_at (Cytokine receptor-like factor 3), 236131_at (CDNA clone IMAGE:6622963), 236953_s_at (similar to RIKEN cDNA 8030451K01), 240544_at (Zinc finger, AN 1-type domain 3), 240971_x_at (Cullin 4A), 244042_x_at (Similar to retinoic acid receptor responder (tazarotene induced) 2), complementary sequences, fragments, alleles, variants and gene products thereof.

In a preferred embodiment, an antibody or aptamer is specific for each biomolecule of biomarker (TBB-II) comprising: 1552419_s_at (tubulin tyrosine ligase-like family, member 10), 1553212_at (keratin 78), 1555124_at (hypothetical protein MGC40574), 1556192_x_at (Metastasis suppressor 1), 1556320_at (Stomatin (EPB72)-like 1), 1556510_at (CDNA clone IMAGE:4796864), 1558484_s_at (leucine rich repeat containing 27), 1565662_at (Mucin 6, oligomeric mucus/gel-forming), 1567410_at (zinc finger protein 135), 1568513_x_at (Protease, serine, 1 (trypsin 1)), 1570408_at (Serine/threonine kinase 24 (STE20 homolog, yeast)), 203307_at (guanine nucleotide binding protein-like 1), 204581_at (CD22 molecule, myelin associated glycoprotein), 205586_x_at (VGF nerve growth factor inducible), 206333_at (musashi homolog 1 (Drosophila)), 207004_at (B-cell CLL/lymphoma 2), 210059_s_at (mitogen-activated protein kinase 13), 210228_at (colony stimulating factor 2 (granulocyte-macrophage)), 210384_at (protein arginine methyltransferase 2), 210923_at (solute carrier family 1 (glutamate transporter), member 7), 211024_s_at (thyroid transcription factor 1 /// thyroid transcription factor 1), 211062_s_at (carboxypeptidase Z /// carboxypeptidase Z), 211096_at (pre-B-cell leukemia transcription factor 2), 211181_x_at (runt-related transcription factor 1 (acute myeloid leukemia 1; aml1 oncogene)), 211710_x_at (ribosomal protein L4 /// ribosomal protein L4), 213096_at (transmembrane and coiled-coil domain family 2), 213121_at (small nuclear ribonucleoprotein 70kDa polypeptide (RNP antigen)), 213242_x_at (KIAA0284), 213568_at (odd-skipped related 2 (Drosophila)), 213770_at kinase suppressor of ras 1 (214171_s_at (U2 small nuclear RNA auxiliary factor 2), 216116_at (NCK interacting protein with SH3 domain), 216427_at (CDNA: FLJ22786 fis, clone KAIA2150), 216820_at, 217054_at (CDNA FLJ39484 fis, clone PROST2014925), 217180_at (Hypothetical protein similar to KIAA0187 gene product), 217182_at (mucin 5AC, oligomeric mucus/gel-forming), 2]7322_x_at, 217430_x_at (collagen, type I, alpha 1), 219070_s_at (motile sperm domain containing 3), 219425_at (sulfotransferase family 4A, member 1), 221663_x_at (histamine receptor H3), 221684_s_at (Nyctalopin), 223974_at (hypothetical protein MGC11082), 226640_at (diacylglycerol lipase beta), 228074_at (hypothetical protein LOC162073), 229191_at (tubulin folding cofactor D), 229257_at (KIAA1856 protein), 229335_at (immunoglobulin superfamily, member 4C), 229358_at (Indian hedgehog homolog (Drosophila)), 230341_x_at (ADAM metallopeptidase with thrombospondin type 1 motif, 10), 230693_at (ATPase, Ca⁺⁺ transporting, cardiac muscle, fast twitch 1), 230768_at (FERM, RhoGEF and pleckstrin domain protein 2), 231510_at (GLI-Kruppel family member GLI2), 231629_x_at (Kallikrein-related peptidase 3), 231998_at, 233794_at (Single stranded DNA binding protein 3), 233974_s_at (family with sequence similarity 129, member B), 234495_at (kallikrein-related peptidase 15), 234637_at (keratin associated protein 4-5), 234881_at, 235568_at (chromosome 19 open reading frame 59), 235600_at (Transcribed locus), 236496_at (degenerative spermatocyte homolog 2, lipid desaturase (Drosophila)), 237087_at (Chromosome 14 open reading frame 105), 237144_at (Latent transforming growth factor beta binding protein 3), 237398_at (Transcribed locus), 237547_at (Hypothetical protein LOC728730), 237679_at (tripartite motif-containing 66), 238267_s_at, 238445_x_at (mannosyl (aipha-1,6-)-glycoprotein beta-1,6-N-acetyl-glucosaminyltransferase, isozyme B), 239026_x_at (centaurin, gamma 3), 239463_at (Transcribed locus), 239756_at (MAD1 mitotic arrest deficient-like 1 (yeast)), 240039_at (Transcribed locus /// Transcribed locus), 240147_at (hypothetical protein MGC11257), 240517_at (Cystathionine-beta-synthase), 241270_at (Rhomboid 5 homolog 2 (Drosophila)), 241431_at, 242365_at (Coiled-coil domain containing 32), 243297_at (Vacuolar protein sorting 13 homolog D (*S*. *cerevisiae*)), 243497_at (Transcribed locus), 243766_s_at (TEA domain family member 2), 43934_at (G protein-coupled receptor 137), complementary sequences, fragments, alleles, variants and gene products thereof.

In a preferred embodiment, an antibody or aptamer is specific for each biomolecule of biomarker (TBB-III) comprising: 1553212_at (keratin 78), 1557236_at (apolipoprotein L), 1558142_at (trinucleotide repeat containing 6B), 1558484_s_at (leucine rich repeat containing 27), 1565614_at (Zinc finger protein 337), 1565662_at (Mucin 6, oligomeric mucus/gel-forming), 1567100_at (Dachshund homolog 1 (Drosophila)), 203307_at (guanine nucleotide binding protein-like 1), 205758_at (CD8a molecule /// CD8a molecule), 206333_at (musashi homolog 1 (Drosophila)), 212920_at, 213242_x_at (KIAA0284), 213619_at (Heterogeneous nuclear ribonucleoprotein H1 (H)), 213770_at (kinase suppressor of ras 1), 214171_s_at (U2 small nuclear RNA auxiliary factor 2), 215443_at (thyroid stimulating hormone receptor), 216198_at (activating transcription factor 7 interacting protein), 216427_at (CDNA: FLJ22786 fis, clone KAIA2150), 217054_at (CDNA FLJ39484 fis, clone PROST2014925), 217182_at (mucin 5AC, oligomeric mucus/gel-forming), 217322_x_at, 219425_at (sulfotransferase family 4A, member 1), 222145_at (CDNA: FLJ23572 fis, clone LNG12403), 229191_at (tubulin folding cofactor D), 229569_at (CDNA clone IMAGE:5263455), 231629_x_at (Kallikrein-related peptidase 3), 233765_at (Hypothetical LOC197135), 233794_at (Single stranded DNA binding protein 3), 233974_s_at (family with sequence similarity 129, member B), 234495_at (kallikrein-related peptidase 15), 235568_at (chromosome 19 open reading frame 59), 235803_at (Cytokine receptor-like factor 3), 236496_at (degenerative spermatocyte homolog 2, lipid desaturase (Drosophila)), 236953_s_at, 238445_x_at (mannosyl (alpha-1,6-)-glycoprotein beta-1,6-N-acetyl-glucosaminyltransferase, isozyme B), 239463_at (Transcribed locus), 240544_at (Zinc finger, AN 1-type domain 3), 243766_s_at (TEA domain family member 2), and 244042_x_at complementary sequences, fragments, alleles, variants and gene products thereof.

### Drug Discovery

In other preferred embodiments, the molecular signatures are useful for the identification of new drugs in the treatment of cardiovascular diseases and disorders.
*Small Molecules:* Small molecule test compounds or candidate therapeutic compounds can initially be members of an organic or inorganic chemical library. As used herein, "small molecules" refers to small organic or inorganic molecules of molecular weight below about 3,000 Daltons. The small molecules can be natural products or members of a combinatorial chemistry library. A set of diverse molecules should be used to cover a variety of functions such as charge, aromaticity, hydrogen bonding, flexibility, size, length of side chain, hydrophobicity, and rigidity. Combinatorial techniques suitable for synthesizing small molecules are known in the art, e.g., as exemplified by Obrecht and Villalgordo, Solid-Supported Combinatorial and Parallel Synthesis of Small-Molecular-Weight Compound Libraries, Pergamon-Elsevier Science Limited (1998), and include those such as the "split and pool" or "parallel" synthesis techniques, solid-phase and solution-phase techniques, and encoding techniques (see, for example, Czarnik, Curr. Opin. Chem. Bio., 1:60 (1997). In addition, a number of small molecule libraries are commercially available.

Particular screening applications relate to the testing of pharmaceutical compounds in drug research. The reader is referred generally to the standard textbook *"*In vitro Methods in Pharmaceutical Research", Academic Press, 1997, and U.S. Pat. No. 5,030,015). Assessment of the activity of candidate pharmaceutical compounds generally involves administering a candidate compound, determining any change in the morphology, marker phenotype and expression, or metabolic activity of the cells and function of the cells that is attributable to the compound (compared with untreated cells or cells treated with an inert compound), and then correlating the effect of the compound with the observed change.

The screening may be done, for example, either because the compound is designed to have a pharmacological effect on certain cell types, or because a compound designed to have effects elsewhere may have unintended side effects. Two or more drugs can be tested in combination (by combining with the cells either simultaneously or sequentially), to detect possible drug--drug interaction effects. In some applications, compounds are screened initially for potential toxicity (Castell et al., pp. 375-410 in "In vitro Methods in Pharmaceutical Research," Academic Press, 1997). Cytotoxicity can be determined in the first instance by the effect on cell viability, survival, morphology, and expression or release of certain markers, receptors or enzymes. Effects of a drug on chromosomal DNA can be determined by measuring DNA synthesis or repair. [³H]thymidine or BrdU incorporation, especially at unscheduled times in the cell cycle, or above the level required for cell replication, is consistent with a drug effect. Unwanted effects can also include unusual rates of sister chromatid exchange, determined by metaphase spread. The reader is referred to A. Vickers (PP 375-410 in "In vitro Methods in Pharmaceutical Research," Academic Press, 1997) for further elaboration.

In one embodiment, a method of identifying a candidate agent is provided said method comprising: (a) contacting a biological sample from a patient with the candidate agent and determining the level of expression of one or more biomarkers described herein; (b) determining the level of expression of a corresponding biomarker or biomarkers in an aliquot of the biological sample not contacted with the candidate agent; (c) observing the effect of the candidate agent by comparing the level of expression of the biomarker or biomarkers in the aliquot of the biological sample contacted with the candidate agent and the level of expression of the corresponding biomarker or biomarkers in the aliquot of the biological sample not contacted with the candidate agent; and (d) identifying said agent from said observed effect, wherein an at least 1%, 2%, 5%, 10% difference between the level of expression of the biomarker gene or combination of biomarker genes in the aliquot of the biological sample contacted with the candidate agent and the level of expression of the corresponding biomarker gene or combination of biomarker genes in the aliquot of the biological sample not contacted with the candidate agent is an indication of an effect of the candidate agent.

In preferred embodiments, the effects of the drug are correlated with the expression of the molecular signatures associated with a good prognosis as described in detail in the examples which follow.

In another embodiment, a candidate agent is derived by the method.

In another embodiment, a pharmaceutical preparation comprises an agent.

In another preferred embodiment, a method of producing a drug comprising the steps of the method (i) synthesizing the candidate agent identified in step (c) above or an analog or derivative thereof in an amount sufficient to provide said drug in a therapeutically effective amount to a subject; and/or (ii) combining the drug candidate the candidate agent identified in step (c) above or an analog or derivative thereof with a pharmaceutically acceptable carrier.

*Vectors*, *Cells:* In some embodiments it is desirable to express the biomolecules that comprise a biomarker, in a vector and in cells. The applications of such combinations are unlimited. The vectors and cells expressing the one or more biomolecules can be used in assays, kits, drug discovery, diagnostics, prognostics and the like. The cells can be stem cells isolated from the bone marrow as a progenitor cell, or cells obtained from any other source, such as for example, ATCC.

"Bone marrow derived progenitor cell" (BMDC) or "bone marrow derived stem cell" refers to a primitive stem cell with the machinery for self-renewal constitutively active. Included in this definition are stem cells that are totipotent, pluripotent and precursors. A "precursor cell" can be any cell in a cell differentiation pathway that is capable of differentiating into a more mature cell. As such, the term "precursor cell population" refers to a group of cells capable of developing into a more mature cell. A precursor cell population can comprise cells that are totipotent, cells that are pluripotent and cells that are stem cell lineage restricted (i.e. cells capable of developing into less than all hematopoietic lineages, or into, for example, only cells of erythroid lineage). As used herein, the term "totipotent cell" refers to a cell capable of developing into all lineages of cells. Similarly, the term "totipotent population of cells" refers to a composition of cells capable of developing into all lineages of cells. Also as used herein, the term "pluripotent cell" refers to a cell capable of developing into a variety (*albeit* not all) lineages and are at least able to develop into all hematopoietic lineages (e.g., lymphoid, erythroid, and thrombocytic lineages). Bone marrow derived stem cells contain two well-characterized types of stem cells. Mesenchymal stem cells (MSC) normally form chondrocytes and osteoblasts. Hematopoietic stem cells (HSC) are of mesodermal origin that normally give rise to cells of the blood and immune system (e.g., erythroid, granulocyte/macrophage, magakaryocite and lymphoid lineages). In addition, hematopoietic stem cells also have been shown to have the potential to differentiate into the cells of the liver (including hepatocytes, bile duct cells), lung, kidney (e.g., renal tubular epithelial cells and renal parenchyma), gastrointestinal tract, skeletal muscle fibers, astrocytes of the CNS, Purkinje neurons, cardiac muscle (e.g., cardiomyocytes), endothelium and skin.

In a preferred embodiment, a method of identifying candidate therapeutic compounds comprises culturing cells expressing at least one biomolecule selected from biomarker (TBB-I) having nucleic acid sequences/biomolecules comprising: 1553145_at (hypothetical protein FLJ39653), 1553575_at, 1557236_at (apolipoprotein L, 6), 1558142_at (trinucleotide repeat containing 6B), 1560752_at (F-box and WD-40 domain protein 2), 1565614_at (Zinc finger protein 337), 1567100_at (Dachshund homolog 1 (Drosophila)), 200068_s_at (calnexin /// calnexin), 201031_s_at (heterogeneous nuclear ribonucleoprotein H1 (H)), 202646_s_at (cold shock domain containing E1, RNA-binding), 205758_at (CD8a molecule /// CD8a molecule), 206188_at (zinc finger protein 623), 212637_s_at (WW domain containing E3 ubiquitin protein ligase 1), 212920_at, 213317_at (chloride intracellular channel 5), 213619_at (Heterogeneous nuclear ribonucleoprotein H1 (H)), 215443_at (thyroid stimulating hormone receptor), 216198_at (activating transcription factor 7 interacting protein), 217870_s_at (cytidylate kinase), 218087_s_at (sorbin and SH3 domain containing 1), 222145_at (CDNA: FLJ23572 fis, clone LNG12403), 223577_x_at (PRO1073 protein), 224321_at (transmembrane protein with EGF-like and two follistatin-like domains 2), 224373_s_at (IQ motif and WD repeats 1), 224644_at (CDNA clone IMAGE:5278517), 226173_at (ornithine aminotransferase-like 1), 226773_at (CDNA FLJ35131 fis, clone PLACE6008824), 226880_at (nuclear casein kinase and cyclin-dependent kinase substrate 1), 228980_at (ring finger and FYVE-like domain containing 1), 229569_at (CDNA clone IMAGE:5263455), 231735_s_at (PRO1073 protein), 233765_at (Hypothetical LOC197135), 235803_at (Cytokine receptor-like factor 3), 236131_at (CDNA clone IMAGE:6622963), 236953_s_at (similar to RIKEN cDNA 8030451K01), 240544_at (Zinc finger, AN1-type domain 3), 240971_x_at (Cullin 4A), 244042_x_at (Similar to retinoic acid receptor responder (tazarotene induced) 2), complementary sequences, fragments, alleles, variants and gene products thereof, complementary sequences, fragments, alleles, variants and gene products thereof, with a candidate therapeutic agent; identifying candidate therapeutic agents which modulate the expression of the biomolecules and identifying a candidate therapeutic agent. Preferably, a candidate therapeutic agent comprises organic molecules, inorganic molecules, vaccines, antibodies, nucleic acid molecules, proteins, peptides and vectors expressing nucleic acid molecules.

In a preferred embodiment, a method of identifying candidate therapeutic compounds comprises culturing cells expressing at least one biomolecule selected from: biomarker (TBB-I) having nucleic acid sequences/biomolecules comprising: 1553145_at (hypothetical protein FLJ39653), 1553575_at, 1557236_at (apolipoprotein L, 6), 1558142_at (trinucleotide repeat containing 6B), 1560752_at (F-box and WD-40 domain protein 2), 1565614_at (Zinc finger protein 337), 1567100_at (Dachshund homolog 1 (Drosophila)), 200068_s_at (calnexin /// calnexin), 201031_s_at (heterogeneous nuclear ribonucleoprotein H1 (H)), 202646_s_at (cold shock domain containing E1, RNA-binding), 205758_at (CD8a molecule /// CD8a molecule), 206188_at (zinc finger protein 623), 212637_s_at (WW domain containing E3 ubiquitin protein ligase 1), 212920_at, 213317_at (chloride intracellular channel 5), 213619_at (Heterogeneous nuclear ribonucleoprotein H1 (H)), 215443_at (thyroid stimulating hormone receptor), 216198_at (activating transcription factor 7 interacting protein), 217870_s_at (cytidylate kinase), 218087_s_at (sorbin and SH3 domain containing 1), 222145_at (CDNA: FLJ23572 fis, clone LNG12403), 223577_x_at (PRO1073 protein), 224321_at (transmembrane protein with EGF-like and two follistatin-like domains 2), 224373_s_at (IQ motif and WD repeats 1), 224644_at (CDNA clone IMAGE:5278517), 226173_at (ornithine aminotransferase-like 1), 226773_at (CDNA FLJ35131 fis, clone PLACE6008824), 226880_at (nuclear casein kinase and cyclin-dependent kinase substrate 1), 228980_at (ring finger and FYVE-like domain containing 1), 229569_at (CDNA clone IMAGE:5263455), 231735_s_at (PRO1073 protein), 233765_at (Hypothetical LOC197135), 235803_at (Cytokine receptor-like factor 3), 236131_at (CDNA clone IMAGE:6622963), 236953_s_at (similar to RIKEN cDNA 8030451K01), 240544_at (Zinc finger, AN 1-type domain 3), 240971_x_at (Cullin 4A), 244042_x_at (Similar to retinoic acid receptor responder (tazarotene induced) 2), complementary sequences, fragments, alleles, variants and gene products thereof, complementary sequences, fragments, alleles, variants and gene products thereof.

In a preferred embodiment, a method of identifying candidate therapeutic compounds comprises culturing cells expressing at least one biomolecule selected from: biomarker (TBB-II) having nucleic acid sequences/biomolecules comprising: 1552419_s_at (tubulin tyrosine ligase-like family, member 10), 1553212_at (keratin 78), 1555124_at (hypothetical protein MGC40574), 1556192_x_at (Metastasis suppressor 1), 1556320_at (Stomatin (EPB72)-like 1), 1556510_at (CDNA clone IMAGE:4796864), 1558484_s_at (leucine rich repeat containing 27), 1565662_at (Mucin 6, oligomeric mucus/gel-forming), 1567410_at (zinc finger protein 135), 1568513_x_at (Protease, serine, 1 (trypsin 1)), 1570408_at (Serine/threonine kinase 24 (STE20 homolog, yeast)), 203307_at (guanine nucleotide binding protein-like 1), 204581_at (CD22 molecule, myelin associated glycoprotein), 205586_x_at (VGF nerve growth factor inducible), 206333_at (musashi homolog 1 (Drosophila)), 207004_at (B-cell CLL/lymphoma 2), 210059_s_at (mitogen-activated protein kinase 13), 210228_at (colony stimulating factor 2 (granulocyte-macrophage)), 210384_at (protein arginine methyltransferase 2), 210923_at (solute carrier family 1 (glutamate transporter), member 7), 211024_s_at (thyroid transcription factor 1 /// thyroid transcription factor 1), 211062_s_at (carboxypeptidase Z /// carboxypeptidase Z), 211096_at (pre-B-cell leukemia transcription factor 2), 211181_x_at (runt-related transcription factor 1 (acute myeloid leukemia 1; aml1 oncogene)), 211710_x_at (ribosomal protein L4 /// ribosomal protein L4), 213096_at (transmembrane and coiled-coil domain family 2), 213121_at (small nuclear ribonucleoprotein 70kDa polypeptide (RNP antigen)), 213242_x_at (KIAA0284), 213568_at (odd-skipped related 2 (Drosophila)), 213770_at kinase suppressor of ras 1 (214171_s_at (U2 small nuclear RNA auxiliary factor 2), 216116_at (NCK interacting protein with SH3 domain), 216427_at (CDNA: FLJ22786 fis, clone KAIA2150), 216820_at, 217054_at (CDNA FLJ39484 fis, clone PROST2014925), 217180_at (Hypothetical protein similar to KIAA0187 gene product), 217182_at (mucin 5AC, oligomeric mucus/gel-forming), 217322_x_at, 217430_x_at (collagen, type I, alpha 1), 219070_s_at (motile sperm domain containing 3), 219425_at (sulfotransferase family 4A, member 1), 221663_x_at (histamine receptor H3), 221684_s_at (Nyctalopin), 223974_at (hypothetical protein MGC11082), 226640_at (diacylglycerol lipase beta), 228074_at (hypothetical protein LOC162073), 229191_at (tubulin folding cofactor D), 229257_at (KIAA1856 protein), 229335_at (immunoglobulin superfamily, member 4C), 229358_at (Indian hedgehog homolog (Drosophila)), 230341 1_x_at (ADAM metallopeptidase with thrombospondin type 1 motif, 10), 230693_at (ATPase, Ca⁺⁺ transporting, cardiac muscle, fast twitch 1), 230768_at (FERM, RhoGEF and pleckstrin domain protein 2), 231510_at (GLI-Kruppel family member GLI2), 231629_x_at (Kallikrein-related peptidase 3), 231998_at, 233794_at (Single stranded DNA binding protein 3), 233974_s_at (family with sequence similarity 129, member B), 234495_at (kallikrein-related peptidase 15), 234637_at (keratin associated protein 4-5), 234881_at, 235568_at (chromosome 19 open reading frame 59), 235600_at (Transcribed locus), 236496_at (degenerative spermatocyte homolog 2, lipid desaturase (Drosophila)), 237087_at (Chromosome 14 open reading frame 105), 237144_at (Latent transforming growth factor beta binding protein 3), 237398_at (Transcribed locus), 237547_at (Hypothetical protein LOC728730), 237679_at (tripartite motif-containing 66), 238267_s_at, 238445_x_at (mannosyl (alpha-1,6-)-glycoprotein beta-1,6-N-acetyl-glucosaminyltransferase, isozyme B), 239026_x_at (centaurin, gamma 3), 239463_at (Transcribed locus), 239756_at (MAD1 mitotic arrest deficient-like 1 (yeast)), 240039_at (Transcribed locus /// Transcribed locus), 240147_at (hypothetical protein MGC11257), 240517_at (Cystathionine-beta-synthase), 241270_at (Rhomboid 5 homolog 2 (Drosophila)), 24143 1_at, 242365_at (Coiled-coil domain containing 32), 243297_at (Vacuolar protein sorting 13 homolog D (S. *cerevisiae*)), 243497*_*at (Transcribed locus), 243766_s_at (TEA domain family member 2), 43934_at (G protein-coupled receptor 137), complementary sequences, fragments, alleles, variants and gene products thereof.

In a preferred embodiment, a method of identifying candidate therapeutic compounds comprises culturing cells expressing at least one biomolecule selected from: biomarker (TBB-III) having nucleic acid sequences/biomolecules comprising: 1553212_at (keratin 78), 1557236_at (apolipoprotein L), 1558142_at (trinucleotide repeat containing 6B), 1558484_s_at (leucine rich repeat containing 27), 1565614_at (Zinc finger protein 337), 1565662_at (Mucin 6, oligomeric mucus/gel-forming), 1567100_at (Dachshund homolog 1 (Drosophila)), 203307_at (guanine nucleotide binding protein-like 1), 205758_at (CD8a molecule /// CD8a molecule), 206333_at (musashi homolog 1 (Drosophila)), 212920_at, 213242_x_at (KIAA0284), 213619_at (Heterogeneous nuclear ribonucleoprotein H1 (H)), 213770_at (kinase suppressor of ras 1), 214171_s_at (U2 small nuclear RNA auxiliary factor 2), 215443_at (thyroid stimulating hormone receptor), 216198_at (activating transcription factor 7 interacting protein), 216427_at (CDNA: FLJ22786 fis, clone KAIA2150), 217054_at (CDNA FLJ39484 fis, clone PROST2014925), 217182_at (mucin 5AC, oligomeric mucus/gel-forming), 217322_x_at, 219425_at (sulfotransferase family 4A, member 1), 222145_at (CDNA: FLJ23572 fis, clone LNG12403), 229191_at (tubulin folding cofactor D), 229569_at (CDNA clone IMAGE:5263455), 231629_x_at (Kallikrein-related peptidase 3), 233765_at (Hypothetical LOC197135), 233794_at (Single stranded DNA binding protein 3), 233974_s_at (family with sequence similarity 129, member B), 234495_at (kallikrein-related peptidase 15), 235568_at (chromosome 19 open reading frame 59), 235803_at (Cytokine receptor-like factor 3), 236496_at (degenerative spermatocyte homolog 2, lipid desaturase (drosophilia)) 236953_s_at, 238445_x_at (mannosyl (alpha-1,6-)-glycoprotein beta-1,6-N-acetyl-glucosaminyltransferase, isozyme B), 239463_at (Transcribed locus), 240544_at (Zinc finger, AN1-type domain 3), 243766_s_at (TEA domain family member 2), and 244042_x_at complementary sequences, fragments, alleles, variants and gene products thereof.

Such compounds are useful, e.g., as candidate therapeutic compounds for the treatment of heart disease, heart disorders and conditions thereof. Thus, included herein are methods for screening for candidate therapeutic compounds for the treatment of, for example, myocarditis, Coronary Heart Disease, angina, Acute Coronary Syndrome, Aortic Aneurysm and Dissection, arrhythmias, Cardiomyopathy, Congenital Heart Disease, congestive heart failure or chronic heart failure, pericarditis, and the like. The methods include administering the compound to a model of the condition, e.g., contacting a cell (*in vitro*) model with the compound, or administering the compound to an animal model of the condition, e.g., an animal model of a condition associated with heart disease. The model is then evaluated for an effect of the candidate compound on the clinical outcome in the model and can be considered a candidate therapeutic compound for the treatment of the condition. Such effects can include clinically relevant effects, decreased pain; increased life span; and so on. Such effects can be determined on a macroscopic or microscopic scale. Candidate therapeutic compounds identified by these methods can be further verified, e.g., by administration to human subjects in a clinical trial.

The biomolecules can be expressed from one or more vectors. A "vector" (sometimes referred to as gene delivery or gene transfer "vehicle") refers to a macromolecule or complex of molecules comprising a polynucleotide to be delivered to a host cell, either in vitro or in vivo. The polynucleotide to be delivered may comprise a coding sequence of interest in gene therapy. Vectors include, for example, viral vectors (such as adenoviruses ("Ad"), adeno-associated viruses (AAV), and retroviruses), liposomes and other lipid-containing complexes, and other macromolecular complexes capable of mediating delivery of a polynucleotide to a host cell. Vectors can also comprise other components or functionalities that further modulate gene delivery and/or gene expression, or that otherwise provide beneficial properties to the targeted cells. As described and illustrated in more detail below, such other components include, for example, components that influence binding or targeting to cells (including components that mediate cell-type or tissue-specific binding); components that influence uptake of the vector nucleic acid by the cell; components that influence localization of the polynucleotide within the cell after uptake (such as agents mediating nuclear localization); and components that influence expression of the polynucleotide. Such components also might include markers, such as detectable and/or selectable markers that can be used to detect or select for cells that have taken up and are expressing the nucleic acid delivered by the vector. Such components can be provided as a natural feature of the vector (such as the use of certain viral vectors which have components or functionalities mediating binding and uptake), or vectors can be modified to provide such functionalities. Other vectors include those described by Chen et al; BioTechniques, 34: 167-171 (2003). A large variety of such vectors are known in the art and are generally available.
In another preferred embodiment, a vector expresses one or more biomolecules that comprise or make up TBB-I.

In another preferred embodiment, a vector expresses one or more biomolecules that comprise or make up TBB-II.
In another preferred embodiment, a vector expresses one or more biomolecules that comprise or make up TBB-III.

### Kits

In another preferred embodiment, a kit is provided comprising any one or more of the biomarkers or molecular signatures comprising Tables 1, 2, and 4.
For use in the applications described or suggested above, kits for use according to the invention are also provided. Such kits may comprise a carrier means being compartmentalized to receive in close confinement one or more container means such as vials, tubes, and the like, each of the container means comprising one of the separate elements to be used in the method. For example, one of the container means may comprise a probe that is or can be detectably labeled. Where the kit utilizes nucleic acid hybridization to detect the target nucleic acid, the kit may also have containers containing nucleotide(s) for amplification of the target nucleic acid sequence and/or a container comprising a reporter-means, such as a biotin-binding protein, such as avidin or streptavidin, bound to a reporter molecule, such as an enzymatic, florescent, or radioisotope label.

The kit for use according to the invention will typically comprise the container described above and one or more other containers comprising materials desirable from a commercial and user standpoint, including buffers, diluents, filters, needles, syringes, and package inserts with instructions for use. A label may be present on the container to indicate that the composition is used for a specific therapy or non-therapeutic application, and may also indicate directions for either in vivo or in vitro use, such as those described above.

The kits for use according to the invention have a number of embodiments. A typical embodiment is a kit comprising a container, a label on said container, and a composition contained within said container; wherein the composition includes a primary antibody that binds to the biomolecules of each molecular signature and instructions for using the antibody for evaluating the presence of biomolecules in at least one type of mammalian cell. The kit can further comprise a set of instructions and materials for preparing a tissue sample and applying antibody and probe to the same section of a tissue sample. The kit may include both a primary and secondary antibody, wherein the secondary antibody is conjugated to a label, e.g., an enzymatic label.

Another embodiment is a kit comprising a container, a label on said container, and a composition contained within said container; wherein the composition includes a polynucleotide that hybridizes to a complement of the polynucleotides under stringent conditions, the label on said container indicates that the composition can be used to evaluate the presence of a molecular signature in at least one type of mammalian cell, and instructions for using the polynucleotide for evaluating the presence of biomolecule RNA or DNA in at least one type of mammalian cell.

Other optional components in the kit include, microarrays, one or more buffers (e.g., block buffer, wash buffer, substrate buffer, etc), other reagents such as substrate (e.g., chromogen) which is chemically altered by an enzymatic label, epitope retrieval solution, control samples (positive and/or negative controls), control slide(s) etc.

Embodiments of inventive compositions and methods are illustrated in the following examples. These examples are provided for illustrative purposes and are not considered limitations on the scope of inventive compositions and methods.

### EXAMPLES

### Materials and Methods:

*Patients:* EMBs were collected from patients that were referred to Johns Hopkins Hospital between 1997 and 2006 for evaluation of cardiomyopathy (n=350). A clinical data base of patient outcome was maintained concurrently for a 10 year period beginning in 1997. All patients gave written informed consent for sample collection and medical chart abstraction. Transvenous EMBs from the right ventricular septum were obtained as previously described (Felker GM, Thompson RE, Hare JM, Hruban RH, Clemetson DE, Howard DL, Baughman KL, Kasper EK. N Engl J Med 2000 April 13;342(15):1077-84) for subsequent microarray analysis. In order to avoid possible disease specific confounding factors, only samples from patients with IDCM were selected. IDCM was a diagnosis of exclusion after extensive histological work-up without any detectable pathological signs. Within a repository of 180 IDCM samples, biopsies were selected in a case-control fashion based on the phenotypic extremes in survival of the cohort. A group with good prognosis (GP, n=25), was defined as having event free survival for at least 5 years after initial presentation with heart failure symptoms; a group with poor prognosis (PP, n=18), experienced an event within the first 2 years. Events included death (n=14), requirement for left ventricular assist device (n=2) or cardiac transplantation (n=2).

*Processing of biopsies:* EMBs were immediately flash frozen in liquid nitrogen for storage in a biorepository. All steps of RNA isolation and processing were performed according to MIAME guidelines (Minimum Information about a Microarray Experiment). Tissue samples (average diameter ∼2mm) were homogenized with the MM 301 Mixer Mill (Retsch, Cat.No. 85120). Trizol reagent together with the Micro-to-Midi Total RNA Purification System (Invitrogen, Cat.No. 12183-018) was used for extraction of total RNA (success rate: 97% of samples). Concentration and integrity of total RNA was measured with the Agilent 2100 Bioanalyzer. All RNA samples exhibited intact 28S and 18S ribosomal RNA on denaturing agarose gel electrophoresis and the 260/280 nm absorbance readings fell in the acceptable range of 1.8-2.1. An average amount of 568 ± 92ng (SEM) total RNA was isolated and preprocessed with the Ovation Biotin RNA Amplification and Labeling System (NuGen, Cat.No. 2300-12).

*Microarray hybridization:* Samples were hybridized to the Human Genome U133 Plus 2.0 Array from Affymetrix without additional amplification step. The microarray experiments were judged successful when RNA isolation and microarray hybridization met all the indices of quality control as specified in the Affymetrix Guideline for Assessing Sample and Array Quality (Kittleson MM, Irizarry RA, Heidecker B, Hare J.M. Transcriptomics: Translation of Global Expression Analysis to Genomic Medicine. In: Willard H.F., Ginsburg G.S., eds. Handbook of Genomic Medicine. 1st ed. Elsevier; 2008). Average background and noise of all chips registered within acceptable ranges and hybridization efficiencies were similar for all samples.

*Statistical Analysis:* Raw intensity values from microarray hybridization were normalized with Robust Multiarray Average (RMA) implemented in the R package for statistical computing (available at www.R-project.org). In the next step, Significance Analysis of Microarrays (SAM) (Tusher VG, et al. Proc Natl Acad Sci U S A 2001 April 24;98(9):5116-21) was used to identify phenotype specific differences in gene expression. SAM defines significance with the q-value, an adjusted p-value for multiple comparisons. For the development of a TBB, Prediction Analysis of Microarrays (PAM) (Tibshirani R, et al. Proc Natl Acad Sci U S A 2002 May 14;99(10):6567-72) was used to create a classifier in a training set (containing 2/3 of the data, n=29), with subsequent validation in a test set (containing 1/3 of the data, n=14)( Kittleson MM, et al. Circulation 2004 November 30;110(22):3444-51). PAM is a software that allows to find the minimum number of genes necessary to create a phenotype specific "nearest shrunken centroid" for classification (Tibshirani R, *et al.* 2002, *supra*)*.* This was done by a balanced 10-fold cross validation in a training set, which enables one to choose a threshold that minimizes classification errors. Overall accuracy of the discovered transcriptomic biomarker was assessed after 50 random partitions. To test for balanced baseline conditions of our cohort with good vs. poor prognosis, a student t-test for numerical and Fisher's exact test for categorical variables was used.

### Example 1: Transcriptomic Biomarker

To identify this biomarker (gene signature) heart samples were collected from patients undergoing heart biopsy at an early stage of heart failure, originating from either myocarditis or idiopathic cardiomyopathy, and stored them in a biorepository over 10 years. Endomyocardial biopsy samples from patients with myocarditis or idiopathic cardiomyopathy have been collected at the Johns Hopkins Hospital between 1997-2004 and stored in liquid nitrogen. The biorepository was reviewed and biopsy samples from 19 patients with myocarditis and 42 patients with idiopathic cardiomyopathy were chosen. Myocarditis was diagnosed from endomyocardial biopsy samples by immunohistochemistry. The MIAME (Minimum information about a microarray experiment) guidelines were followed for all experiments. The tissue was homogenized with the MM301 instrument from Retsch and total RNA was isolated with the Micro-to-Midi Total RNA purification system from Invitrogen. Microarray analysis of total RNA was performed with the Human Genome U 133 Plus 2.0 Array from Affymetrix. In all samples, both RNA isolation and microarray hybridization met all indices of quality control as specified in the Affymetrix Guideline for Assessing Sample and Array Quality. Raw expression values of all microarray chips were preprocessed with Robust Multiarray Average (RMA) in R. We performed Significance Analysis of Microarrays (SAM) on a training set of 28 samples (11 samples from myocarditis, 17 samples from idiopathic cardiomyopathy) to select a subset of 122 differentially expressed genes (FC>1.2, q = 0%). These 122 contained 38 genes that were overexpressed and 84 genes were downregulated in patients with myocarditis (Figure 1).

With this predefined subset of 122 genes, a heatmap was created including all samples of the study (n=61) standardized by mean levels of expression (Figure 2, Table 1, Table 2). A heatmap is a classification method of unsupervised machine learning. Each row represents one of the 122 genes, and each column is a patient sample. A red cell depicts an underexpressed gene in a given patient relative to the average gene expression in all patients, while a blue cell denotes an overexpressed gene. The dendrogram at the top is an unsupervised hierarchical clustering algorithm that divides samples into groups based on the similarity of the gene expression profiles. This algorithm performed with 77% sensitivity and 97% specificity. To increase predictive power, the same subset of 122 genes for a "nearest shrunken centroid" classification approach with Prediction Analysis of Microarrays (PAM) implemented in R was used. After training of the classifier (supervised clustering) in a subset of 11 samples of patients with myocarditis and 17 samples of patients with idiopathic cardiomyopathy, a cluster of 39 genes that identified myocarditis with 90% sensitivity and 88% specificity was found (Figure 3, Figure 4, Figure 5, Table 3, Table 4). To test if the predictive power of the discovered transcriptomic biomarker could be increased, heatmap with the new subset of 39 candidate genes was created (Figure 6). While the first heatmap of 122 genes produced 6 misclassifed samples, only 4 samples were classified incorrectly by this algorithm (sensitivity: 89%, specificity: 95%). With this extremely powerful diagnostic method of gene expression profiling, even patients that do not present with characteristic clinical signs of myocarditis, will be detected at a very early stage of the disease and treated appropriately. The application of this highly sensitive biomarker will significantly reduce the number of cases that undergo a fatal course of myocarditis in the future. In addition this biomarker will allow appropriate use of therapies specifically for myocarditis.
Table 1 shows the 38 genes that were significantly upregulated in patients with myocarditis versus idiopathic cardiomyopathy. First column contains the ID from Affymetrix for each transcript.

**Table 1: 38 significantly upregulated genes in patients with myocarditis (q=0.1%; FC>1.2)**

| *Probe Set ID* | *Gene Title* | *GO Biological Process Description* |
|---|---|---|
| 1553145_at | hypothetical protein FLJ39653 | --- |
| 1553575_at | --- | --- |
| 1557236_at | apolipoprotein L, 6 | regulation of transcription, DNA-dependent /// lipid transport |
| 1558142_at | trinucleotide repeat containing | --- |
| 1560752_at | F-box and WD-40 domain protein 2 | ubiquitin cycle /// protein modification /// proteolysis |
| 1565614_at | Zinc finger protein 337 | transcription /// regulation of transcription, DNA-dependent |
| 1567100_at | Dachshund homolog 1 (Drosophila) | transcription /// regulation of transcription, DNA-dependent |
| 200068_s_at | calnexin *III* calnexin | angiogenesis /// protein folding /// protein secretion |
| 201031_s_at | heterogeneous nuclear ribonucleoprotein H1 (H) | mRNA processing /// RNA splicing /// RNA processing |
| 202646_s_at | cold shock domain containing E1, RNA-binding | regulation of transcription, DNA-dependent /// male gonad development |
| 205758_at | CD8a molecule /// CD8a molecule | immune response /// antigen processing and presentation /// T cell activation /// cytoloxic T cell differentiation /// immune response |
| 206183_ at | zinc finger protein 623 | transcription /// regulation of transcription. DNA-dependent |
| 212637_s_at | WW domain containing E3 ubiquitin protein ligase 1 | signal transduction /// T cell differentiation /// entry of virus into host cell |
| 212920_at | --- | --- |
| 213317_at | chloride intracellular channel 5 | ion transport /// chloride transport /// chloride transport /// pregnancy /// transport /// transport |
| 213619_at | Heterogeneous nuclear ribortucleoprotein H1 (H) | RNA splicing |
| 215443_at | thyroid stimulating hormone receptor | signal transduction /// G-protein signaling |
| 216198_at | activating transcription factor 7 interacting protein | DNA methylation |
| 217870_s_at | cyidylate kinase | pyrimidine ribonucleotide biosynthesis |
| 218087_s_at | sorbin and SH3 domain containing I | transport /// insulin receptor signaling pathway |
| 222145_at | CDNA: FLJ23572 fis, clone LNG 12403 | --- |
| 223577_x_at | PRO1073 protein | --- |
| 224321_at | transmembrane protein with EGF-like and two follistatin-like domains 2 | --- |
| 224373_s_at | IQ motif and WD repeats 1 | apoptosis /// signal transduction /// development /// ATP synthesis coupled electron transport |
| 224644_at | CDNA clone IMAGE:5278517 | --- |
| 226173_at | ornithine aminotransferase-like I | --- |
| 226773_at | CDNA FLJ35131 fis, clone PLACE6008824 | --- |
| 226880_at | nuclear casein kinase and cyclin-dependent kinase substrate 1 | --- |
| 228980_at | ring finger and FYVE-like containing 1 | ubiquitin cycle /// apoplosis |
| 229569_at | CDNA clone IMAGE:5263455 | --- |
| 231735_s_at | PRO1073 protein | --- |
| 233765_at | Hypothetical LOC197135 | --- |
| 235803_at | Cytokine receptor-like factor 3 | --- |
| 236131_at | CDNA clone IMAGE:6622963 | --- |
| 236953_s_at | similar to RIKEN cDNA 8030451K01 | --- |
| 240544_at | Zinc finger. AN1-type domain 3 | --- |
| 240971_x_at | Cullin 4A | GI/S transition of mitotic cell cycle /// DNA repair /// *ubiquitin* cycle /// cell cycle /// cell cycle arrest |
| 244042_x_at | Similar to retinoic acid receptor responder (tazarotene induced) 2 | --- |

Table 2 shows 84 genes that were significantly downregulated in patients with myocarditis versus patients with idiopathic cardiomyopathy (FC> 1.2, q=0%).

**Table 2: 84 significantly downregulated genes in myocarditis (FC>1.2, q=0.1%))**

| *Probe Set ID* | *Gene Title* | *GO Biological Process Description* |
|---|---|---|
| 1552419_s_at | tubulin tyrosine ligase-like family, member 10 | protein modification |
| 1553212_at | keratin 78 | --- |
| 1555124_at | hypothetical protein MGC40574 | --- |
| 1556192_x_at | Metastasis suppressor 1 | cell motiiity |
| 1556320_at | Stomatin (EPB72)-like I | biological_process |
| 1556510_at | CDNA clone IMAGE:4796864 | --- |
| 1558484_s_at | leucine rich repeat containing 27 | --- |
| 1565662_at | Mucin 6. oligomeric mucus/gel-forming | maintenance of gastrointestinal epithelium |
| 1567410_at | zinc finger protein 135 | regulation of transcription. DNA-dependent |
| 1568513_x_at | Protease, serine, I (trypsin 1) | collagen catabolism, proteolysis, collagen catabolism, immune response |
| 1570408_at | Serine/threonine kinase 24 (STE20 homolog, yeast) | protein amino acid phosphorylation, signal transduction |
| 203307_al | guanine nucleotide binding protein-like 1 | signal transduction |
| 204581_at | CD22 molecule, myelin associated glycoprotein | immune response, cell adhesion, antimicrobial humoral response (sensu Vertebrata) |
| 205586_x_at | VGF nerve growth factor inducible | biological_process |
| 206333_at | musashi homolog I (Drosophila) | nervous system development |
| 207004_at | B-cell CLL/lymphoma 2 | regulation of progression through cell cycle, humoral immune response |
| 210059_s_at | mitogen-activated protein kinase 13 | regulation of translation, protein amino acid phosphorylation |
| 210228_at | colony stimulating factor 2 (granulocyte-macrophage) | cellular defense response |
| 210384_at | protein arginine methyltransferase 2 | protein amino acid methylation |
| 210923_at | solute carrier family 1 (glutamate transporter), member 7 | dicarboxylic acid transport |
| 211024_s_at | thyroid transcription factor 1 /// thyroid transcription factor 1 | neuron migration |
| 211062_s_at | carboxypeptidase Z /// carboxypeptidase Z | proteolysis, Wnt receptor signaling pathway |
| 211096_at | pre-B-cell leukemia transcription factor 2 | regulation of transcription, DNA-dependent |
| 211181_x_at | runt-related transcription factor 1 (acute myeloid leukemia I; am1 I oncogene) | skeletal development, hemopoiesis |
| 211710_x_at | ribosomal protein L4 /// ribosomal protein L4 | protein biosynthesis, metabolism |
| 213096_at | transmembrane and coiled-coil domain family 2 | --- |
| 213121_at | small nuclear ribonucleoprotein 70kDa polypeptide (RNP antigen) | mRNA processing |
| 213242_x_at | KIAA0284 | --- |
| 213569_al | odd-skipped related 2 (Drosophila) | --- |
| 213770_at | kinase suppressor of ras I | protein amino acid phosphorylation |
| 214171_s_at | U2 small nuclear RNA auxiliary factor 2 | mRNA processing |
| 216116_at | NCK interacting protein with SH3 domain | NLS-bearing substrate import into nucleus |
| 216427_at | CDNA: FLJ22786 fis, clone KAIA2150 | --- |
| 216820_at | | --- |
| 217054_at | CDNA FLJ39484 fis, clone PROST2014925 | --- |
| 217180_at | Hypothetical protein similar to KIAA0187 gene product | immune response |
| 217182_at | mucin 5AC. oligomeric mucus/gel-forming | cell adhesion |
| 217322_x_at | --- | --- |
| 217430_x_at | collagen, type 1, alpha I | skeletal development |
| 219070_s_at | motile sperm domain containing 3 | heart development |
| 219425_at | sulfotransferase family 4A, member I | lipid metabolism |
| 221663_x_at | histamine receptor H3 | G-protein coupled receptor protein signaling pathway |
| 221684_s_at | Nyctalopin | response to stimulus |
| 223974_at | hypothetical protein MGC11082 | --- |
| 226640_at | diacylglycerol lipase beta | lipid catabolism, lipid metabolism |
| 228074_at | hypothetical protein LOC162073 | --- |
| 129191_at | tubulin folding cofactor D | beta-tubulin folding |
| 229257_at | KIAA1856 protein | --- |
| 229335_at | immunoglobulin superfamily, member 4C | --- |
| 229358_at | Indian hedgehog homolog (Drosophila) | skeletal development, patterning of blood vessels |
| 230341_x_at | ADAM metallopeptidase with thrombospondin type I motif, 10 | proteolysis, integrin-mediated signaling pathway |
| 230693_at | ATPase, Ca++ transporting, cardiac muscle, fast twitch 1 | calcium ion transport, regulation of striated muscle contraction |
| 230768_at | FERM, RhoGEF and pleckstrin domain protein 2 | neuron remodeling |
| 231510_at | GLI-Kruppel family member GL12 | transcription from RNA polymerase II promoter |
| 231629_x_at | Kallikrein-related peptidase 3 | proteolysis, negative regulation of angiogenesis |
| 231998_at | --- | --- |
| 233794 _at | Single stranded DNA binding protein 3 | regulation of transcription, DNA-dependent |
| 233974_s_at | family with sequence similarity 129, member B | --- |
| 234495_at | kallikrein-related (peptidase 15 | proteolysis, |
| 234637_at | keratin associated protein 4-5 | --- |
| 234881_at | --- | --- |
| 235568_at | chromosome 19 open reading frame 59 | --- |
| 235600_at | Transcribed locus | --- |
| 236496_at | degenerative spermatocyte homolog 2, lipid desaturase (Drosophila) | lipid metabolism |
| 237087_at | Chromosome 14 open reading frame 105 | --- |
| 237144_at | Latent transforming growth factor beta binding protein 3 | skeletal development, transforming growth factor beta receptor signaling pathway |
| 237398_at | Transcribed locus | --- |
| 237547_at | Hypothetical protein LOC728730 | --- |
| 237679_at | tripartite motif-containing 66 | negative regulation of transcription, DNA-dependent |
| 238267_s_at | --- | --- |
| 238445_x_at | mannosyl (alpha-1,6-)-glycoprotein beta-1,6-N-acetyl-glucosaminyltransferase, isozyme B | --- |
| 239026_x_at | centaurin, gamma 3 | regulation of transcription, DNA-dependent |
| 239463_at | Transcribed locus | --- |
| 239756_at | MADI mitotic arrest deficient-like 1 (yeast) | mitosis checkpoint |
| 240039_at | Transcribed locus /// Transcribed locus | --- |
| 240147_at | hypothetical protein MGC11257 | --- |
| 240517_at | Cystathionine-beta-synthase | cysteine biosynthesis from serine |
| 241270_at | Rhomboid 5 homolog 2 (Drosophila) | --- |
| 241431_at | --- | --- |
| 242365_at | Coiled-coil domain containing 32 | --- |
| 243297_at | Vacuolar protein sorting 13 homolog D (S. cerevisiae) | protein localization, cell cycle, mitosis |
| 243497_at | Transcribed locus | |
| 243766_s_at | TEA domain family member 2 | transcription, regulation of transcription, DNA-dependent |
| 43934_at | G protein-coupled receptor 137 | biological process |

Table 3 shows the prediction results of the test set (n=28): 39 genes signature was used. The sample labels are listed above the row highlighted in red, followed by the predicted classes. Myocarditis (Myo) samples were assigned to class 2 - idiopathic cardiomyopathy samples (GP, BP) samples were assigned to class 1. 25 samples were correctly classified (probabilities between 75% and 99%). Only three samples were misclassified (with probabilities between 48% and 99%). Predicted probabilities are listed for each class in the last two lines.

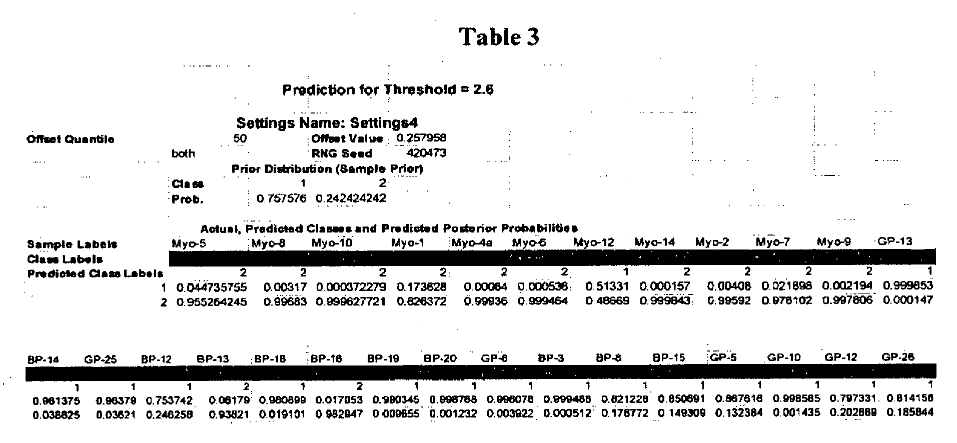

Table 4: 39 genes transcriptomic biomarker for detection of patients with myocarditis: First column contains the IDs from Affymetrix for every gene. Annotations for biological function were derived from the gene ontology database.

**Table 4: 39 genes biomarker to detect myocarditis**

| *Probe Set ID* | *Gene Title* | *GO Biological Process Description* |
|---|---|---|
| 1553212_at | keratin 78 | --- |
| 1557236_at | apolipoproicin L. 6 | regulation of transcription. DNA-dependent /// lipid transport /// lipoprotein metabolism |
| 1558142_at | trinucleotide repeat containing 6B | --- |
| 1558484_s_at | leucine rich repeat containing 27 | --- |
| 1565614_at | Zinc finger protein 337 dependent | regulation of transcription, DNA-dependent |
| 1565662_at | Mucin 6, oligomeric mucus/gel-forming | maintenance of gastrointestinal epithelium |
| 1567100_at | Dachshund homolog I (Drosophila) | regulation of transcription, DNA-dependent |
| 203307_at | guanine nucleotide binding protein-like 1 | signal transduction |
| 205758_at | CD8a molecule *lll* CD8a molecule | antigen processing and presentation, cytotoxic T cell differentiation immune response |
| 206333_at | musashi homolog (Drosophila) | nervous system development |
| 212920_at | --- | --- |
| 213242_x_at | KIAA0284 | --- |
| 213619_at | Heterogeneous nuclear ribonucleoprotein | mRNA processing |
| 213770_at | H1 (H) kinase suppressor of ras 1 | prolein amino acid phosphorylation |
| 214171_s_at | U2 small nuclear RNA auxiliary factor 2 | nuclear mRNA splicing, via spliceosome |
| 215443_at | thyroid stimulating hormone receptor | signal transduction, G-protein signaling, coupled to cyclic nucleotide second messenger |
| 216198_at | activating transcription factor 7 interacting protein | DNA methylation |
| 216427_at | CDNA: FLJ22786 fis, clone KAIA2150 | --- |
| 217054_at | CDNA FLJ39484 fis, clone PROS12014925 | --- |
| 217182_at | mucin 5AC. oligomeric mucus/gel-forming | cell adhesion |
| 217322_x_at | | --- |
| 219425_at | sulfotransferase family 4A, member 1 | lipid metabolism |
| 222145_at | CDNA: FLJ23572 fis, clone LNG12403 | --- |
| 229191_at | tubulin folding cofactor D | protein folding |
| 229569_at | CDNA clone IMAGE:5263455 | --- |
| 231624_x_at | Kallikrein-related peptidase 3 | proteolysis, negative regulation of angiogenesis |
| 233765_at | Hypothetical LOC197135 | --- |
| 233794_at | Single stranded DNA binding protein 3 | regulation of transcription, DNA- |
| 233974_s_at | family with sequence similarity 129. member B | --- |
| 234495_at | kallikrein-related peptidase 15 | proteolysis, proteolysis |
| 235568_at | chromosome 19 open reading frame 59 | --- |
| 235803_at | Cytokine receptor-like factor 3 | |
| 236496_at | degenerative spermatocyte homolog 2, lipid desaturase (Drosophila) | lipid metabolism |
| 236953_s_at | similar to RIKEN cDNA 8030451K01 | --- |
| 238445_x_at | mannosyl (alpha-1,6-)-glycoprotein beta-1,6-N-acetyl-glucosaminyltransferase, isozyme B | --- |
| 239463_at | Transcribed locus | --- |
| 240544_at | Zinc finger, AN1-type domain 3 | --- |
| 243766_s_at | TEA domain family member 2 | regulation of transcription, DNA-dependent |
| 244042_x_at | Similar to retinoic acid receptor responder (tazarotene induced) 2 | --- |

### Other Embodiments

It is to be understood that while the invention has been described in conjunction with the detailed description thereof, the foregoing description is intended to illustrate and not limit the scope of the invention. Other aspects, advantages, and modifications are within the scope of the following claims.

## Claims

1. Use of a molecular composition comprising nucleic acid molecules consisting of nucleic acid sequences of: 1553145_at (hypothetical protein FLJ39653), 1553575_at, 1557236_at (apolipoprotein L, 6), 1558142_at (trinucleotide repeat containing 6B), 1560752_at (F-box and WD-40 domain protein 2), 1565614_at (Zinc finger protein 337), 1567100_at (Dachshund homolog I (Drosophila)), 200068_s_at (calnexin /// calnexin), 201031_s_at (heterogeneous nuclear ribonucleoprotein H1 (H)), 202646_s_at (cold shock domain containing E1, RNA-binding), 205758_at (CD8a molecule /// CD8a molecule), 206188_at (zinc finger protein 623), 212637_s_at (WW domain containing E3 ubiquitin protein ligase 1), 212920_ at, 213317_ at (chloride intracellular channel 5), 213619_at (Heterogeneous nuclear ribonucleoprotein H1 (H)), 215443_at (thyroid stimulating hormone receptor), 216198_at (activating transcription factor 7 interacting protein), 217870_s_at (cytidylate kinase), 218087_s_at (sorbin and SH3 domain containing 1), 222145_at (CDNA: FLJ23572 fis, clone LNG12403), 223577_x_at (PRO1073 protein), 224321_at (transmembrane protein with EGF-like and two follistatin-like domains 2), 224373_s_at (IQ motif and WD repeats 1), 224644_at (CDNA clone IMAGE:5278517), 226173_at (ornithine aminotransferase-like 1), 226773_at (CDNA FLJ35131 fis, clone PLACE6008824), 226880_at (nuclear casein kinase and cyclin-dependent kinase substrate 1), 228980 at (ring finger and FYVE-like domain containing 1), 229569_ at (CDNA clone IMAGE:5263455), 231735_s_at (PRO107 protein), 233765_at (Hypothetical LOCI97135), 235803_at (Cytokine receptor-like factor 3), 236131_at (CDNA clone IMAGE:6622963), 236953_s_at (similar to RIKEN cDNA 8030451K01), 240544_at (Zinc finger, AN1-type domain 3), 240971_x_at (Cullin 4A), and 244042_x_at (Similar to retinoic acid receptor responder (tazarotene induced) 2) for the *in vitro* diagnosis of myocarditis.

2. The use of claim 1, wherein detection of overexpression of the nucleic acid molecules, complementary sequences, alleles, or gene products thereof, in a biological sample is diagnostic of myocarditis.

3. The use of claim 1, wherein the nucleic acid molecules, or gene products thereof, are over-expressed at levels by at least 10% in a cell or patient as compared to levels in a normal cell or normal subject.

4. The use of claim 1, wherein the nucleic acid molecules, complementary sequences, alleles, or gene products thereof, are overexpressed by about 50% in a cell or a patient as compared to levels in a normal cell or normal subject.

5. The use of claim 1, wherein the nucleic acid molecules, or gene products thereof, are overexpressed by about 75% in a cell or a patient as compared to levels in a normal cell or normal subject.

6. The use of claim 1, wherein a biochip comprises the nucleic acid molecules hybridized to the biochip.

7. Use of a transcriptomic biomarker consisting of nucleic acid molecules complementary to: 1553212_at (keratin 78), 1557236_at (apolipoprotein L), 1558142_at (trinucleotide repeat containing 6B), 1558484_s_at (leucine rich repeat containing 27), 1565614_at (Zinc finger protein 337), 1565662_at (Mucin 6, oligomeric mucus/gel-forming), 1567100_at (Dachshund homolog 1 (Drosophila)), 203307_at (guanine nucleotide binding protein-like 1), 205758_at (CD8a molecule /// CD8a molecule), 206333_at (musashi homolog 1 (Drosophila)), 212920_at, 213242_x_at (KIAA0284), 213619_at (Heterogeneous nuclear ribonucleoprotein HI (H)), 213770_at (kinase suppressor of ras 1), 214171_s_at (U2 small nuclear RNA auxiliary factor 2), 215443_at (thyroid stimulating hormone receptor), 216198_at (activating transcription factor 7 interacting protein), 216427_ at (CDNA: FLJ22786 fis, clone KAIA2150), 217054_at (cDNA FLJ39484 fis, clone PROST2014925), 217182_at (mucin 5AC, oligomeric mucus/gel-forming), 217322_x_at, 219425_at (sulfotransferase family 4A, member 1), 222145_at (cDNA: FLJ23572 fis, clone LNG12403), 229191_at (tubulin folding cofactor D), 229569 at (cDNA clone IMAGE:5263455), 231629 _x_at (Kallikrein-related peptidase 3), 233765_at (Hypothetical LOCI97135), 233794_at (Single stranded DNA binding protein 3), 233974_s_at (family with sequence similarity 129, member B), 234495_at (kallikrein-related peptidase 15), 235568_at (chromosome 19 open reading frame 59), 235803_at (Cytokine receptor-like factor 3), 236496_at (degenerative spermatocyte homolog 2, lipid desaturase (Drosophila)), 236953_s_at, 238445_x_at (mannosyl (alpha-1,6-)-glycoprotein beta-1,6-N-acetyl-glucosaminyltransferase, isozyme B), 239463_at (Transcribed locus), 240544_at (Zinc finger, ANI-type domain 3) and 243766_s_at (TEA domain family member 2), for the *in vitro* diagnosis of myocarditis.

8. The use of claim 7, wherein detection of overexpression of the nucleic acid molecules, complementary sequences, alleles, or gene products thereof, in a biological sample is diagnostic of myocarditis.

9. The use of claim 7, wherein nucleic acid molecules, complementary sequences, alleles, or gene products thereof, are overexpressed at levels by at least 10% in a cell or patient as compared to levels in a normal cell or normal subject.

10. The use of claim 7, wherein the nucleic acid molecules, complementary sequences, alleles, or gene products thereof, are overexpressed by about 50% in a cell or a patient as compared to levels in a normal cell or normal subject.

11. The use of claim 7, wherein the nucleic acid molecules, complementary sequences, alleles, or gene products thereof, are overexpressed by about 75% in a cell or a patient as compared to levels in a normal cell or normal subject.

12. An in vitro method of diagnosing myocarditis, comprising: generating from a patient a molecular signature, wherein the generating comprises measuring the expression of nucleic acid molecules comprising nucleic acid sequences: 1553145_at (hypothetical protein FLJ39653), 1553575_at, 1557236_at (apolipoprotein L, 6), 1558142_at (trinucleotide repeat containing 6B), 1560752_at (F-box and WD-40 domain protein 2), 1565614_at (Zinc finger protein 337), 1567100_at (Dachshund homolog I (Drosophila)), 200068_s_at (calnexin /// calnexin), 201031_s_at (heterogeneous nuclear ribonucleoprotein H1 (H)), 202646_s_at (cold shock domain containing E1, RNA-binding), 205758_at (CD8a molecule /// CD8a molecule), 206188_at (zinc finger protein 623), 212637_s_at (WW domain containing E3 ubiquitin protein ligase 1), 212920_ at, 213317_ at (chloride intracellular channel 5), 213619_at (Heterogeneous nuclear ribonucleoprotein H1 (H)), 215443_at (thyroid stimulating hormone receptor), 216198_at (activating transcription factor 7 interacting protein), 217870_s_at (cytidylate kinase), 218087_s_at (sorbin and SH3 domain containing 1), 222145_at (CDNA: FLJ23572 fis, clone LNG12403), 223577_x_at (PRO1073 protein), 224321_at (transmembrane protein with EGF-like and two follistatin-like domains 2), 224373_s_at (IQ motif and WD repeats 1), 224644_at (CDNA clone IMAGE:5278517), 226173_at (ornithine aminotransferase-like 1), 226773_at (CDNA FLJ35131 fis, clone PLACE6008824), 226880_at (nuclear casein kinase and cyclin-dependent kinase substrate 1), 228980 at (ring finger and FYVE-like domain containing 1), 229569_ at (CDNA clone IMAGE:5263455), 231735_s_at (PRO1073 protein), 233765_at (Hypothetical LOCI97135), 235803_at (Cytokine receptor-like factor 3), 236131_at (CDNA clone IMAGE:6622963), 236953_s_at (similar to RIKEN cDNA 8030451K01), 240544_at (Zinc finger, AN1-type domain 3), 240971_x_at (Cullin 4A) and 244042_x_at (Similar to retinoic acid receptor responder (tazarotene induced) 2);
analyzing the generated molecular signature;
and diagnosing whether or not the patient has myocarditis upon the analysis of the generated molecular signature.

13. The method of claim 12, wherein the patient is diagnosed as having myocarditis if: 1553212_at (keratin 78), 1557236_at (apolipoprotein L), 1558142_at (trinucleotide repeat containing 6B), 1558484_s_at (leucine rich repeat containing 27), 1565614_at (Zinc finger protein 337), 1565662_at (Mucin 6, oligomeric mucus/gel-forming), 1567100_at (Dachshund homolog 1 (Drosophila)), 203307_at (guanine nucleotide binding protein-like 1), 205758_at (CD8a molecule /// CD8a molecule), 206333_at (musashi homolog 1 (Drosophila)), 212920_at, 213242_x_at (KIAA0284), 213619_at (Heterogeneous nuclear ribonucleoprotein HI (H)), 213770_at (kinase suppressor of ras 1), 214171_s_at (U2 small nuclear RNA auxiliary factor 2), 215443_art (thyroid stimulating hormone receptor), 216198_at (activating transcription factor 7 interacting protein), 216427_ at (CDNA: FLJ22786 fis, clone KAIA2150), 217054_at (cDNA FLJ39484 fis, clone PROST2014925), 217182_at (mucin 5AC, oligomeric mucus/gel-forming), 217322_x_at, 219425_at (sulfotransferase family 4A, member 1), 222145_at (cDNA: FLJ23572 fis, clone LNG12403), 229191_at (tubulin folding cofactor D), 229569 at (cDNA clone IMAGE:5263455), 231629_x_at (Kallikrein-related peptidase 3), 233765_at (Hypothetical LOCI97135), 233794_at (Single stranded DNA binding protein 3), 233974_s_at (family with sequence similarity 129, member B), 234495_at (kallikrein-related peptidase 15), 235568_at (chromosome 19 open reading frame 59), 235803_at (Cytokine receptor-like factor 3), 236496_at (degenerative spermatocyte homolog 2, lipid desaturase (Drosophila)), 236953_s_at, 238445_x_at (mannosyl (alpha-1,6-)-glycoprotein beta-1,6-N-acetyl-glucosaminyltransferase, isozyme B), 239463 _at (Transcribed locus), 240544_at (Zinc finger, ANI-type domain 3), 243766_s_at (TEA domain family member 2) and 244042_x_at are upregulated.

14. The method of claims 12 or 13, wherein the generating further comprises isolating nucleic acid molecules obtained from a biological sample, and
the isolated nucleic acid molecules are preferably hybridized to a biochip comprising complementary nucleic acid molecules and raw intensity values from the hybridization are preferably normalized and phenotype specific differences in gene expression are preferably identified, and
wherein the differences in gene expression preferably are identified by significance analysis of microarrays, wherein significance is defined with a q-value and multiple comparisons comprise an adjusted p-value, and
wherein the phenotype specificity is preferably identified by creating a classifier in a training set comprising about 66% of data obtained, with subsequent validation in a test set comprising about 33% of data obtained and defining a phenotype specific nearest shrunken centroid for classification, and
wherein the phenotype specific nearest shrunken centroid preferably comprises balancing about a 10-fold cross validation in a training set.

15. The use of claim 1, wherein a kit comprises the nucleic acid molecules

16. The use of claim 15, wherein a biochip comprises the nucleic acid molecules.

17. Use of a biochip comprising nucleic acid molecules hybridized to the biochip, wherein the nucleic acid molecules consist of: 1553212_at (keratin 78), 1557236_at (apolipoprotein L), 1558142_at (trinucleotide repeat containing 6B), 1558484_s_at (leucine rich repeat containing 27), 1565614_at (Zinc finger protein 337), 1565662_at (Mucin 6, oligomeric mucus/gel-forming), 1567100_at (Dachshund homolog 1 (Drosophila)), 203307_at (guanine nucleotide binding protein-like 1), 205758_at (CD8a molecule /// CD8a molecule), 206333_at (musashi homolog 1 (Drosophila)), 212920_at, 213242_x_at (KIAA0284), 213619_at (Heterogeneous nuclear ribonucleoprotein HI (H)), 213770_at (kinase suppressor of ras 1), 214171_s_at (U2 small nuclear RNA auxiliary factor 2), 215443_at (thyroid stimulating hormone receptor), 216198_at (activating transcription factor 7 interacting protein), 216427_ at (CDNA: FLJ22786 fis, clone KAIA2150), 217054_at (cDNA FLJ39484 fis, clone PROST2014925), 217182_at (mucin 5AC, oligomeric mucus/gel-forming), 217322_x_at, 219425_at (sulfotransferase family 4A, member 1), 222145_at (cDNA: FLJ23572 fis, clone LNG12403), 229191_at (tubulin folding cofactor D), 229569 at (cDNA clone IMAGE:5263455), 231629_x_at (Kallikrein-related peptidase 3), 233765_at (Hypothetical LOCI97135), 233794_at (Single stranded DNA binding protein 3), 233974_s_at (family with sequence similarity 129, member B), 234495_at (kallikrein-related peptidase 15), 235568_at (chromosome 19 open reading frame 59), 235803_at (Cytokine receptor-like factor 3), 236496_at (degenerative spermatocyte homolog 2, lipid desaturase (Drosophila)), 236953_s_at, 238445_x_at (mannosyl (alpha-1,6-)-glycoprotein beta-1,6-N-acetyl-glucosaminyltransferase, isozyme B), 239463 _at (Transcribed locus), 240544_at (Zinc finger, ANI-type domain 3), 243766_s_at (and TEA domain family member 2) for the *in vitro* diagnosis of myocarditis.

## Patentansprüche

1. Verwendung einer molekularen Zusammensetzung umfassend Nukleinsäuremoleküle bestehend aus folgenden Nukleinsäuresequenzen: 1553145_at (hypothetisches Protein FLJ39653), 1553575_at, 1557236_at (Apolipoprotein L, 6), 1558142_at (Trinukleotid-Wiederholungssequenz enthaltend 6B), 1560752_at (F-Box und WD-40-Domäne-Protein 2), 1565614_at (Zinkfingerprotein 337), 1567100_at (Dachshund-Homolog I (Drosophila)), 200068_s_at (Calnexin /// Calnexin), 201031_s_at (heterogenes nukleäres Ribonukleoprotein H1 (H)), 202646_s_at (Kälteschockdomäne enthaltend E1, RNA-bindend), 205758_at (CD8a-Molekül /// CD8a-Molekül), 206188_at (Zinkfingerprotein 623), 212637_s_at (WW-Domäne-enthaltende E3-Ubiquitin-Proteinligase 1), 212920_at, 213317_at (intrazellulärer Chloridkanal 5), 213619_at (heterogenes nukleäres Ribonukleoprotein H1 (H)), 215443_at (Rezeptor des schilddrüsenstimulierenden Hormons), 216198_at (Protein, das mit dem aktivierenden Transkriptionsfaktor 7 interagiert), 217870_s_at (Cytidylatkinase), 218087_s_at (Sorbin und SH3-Domäne enthaltend 1), 222145_at (CDNA: FLJ23572 fis, Klon LNG12403), 223577_x_at (PRO 1073-Protein), 224321_at (Transmembranprotein mit EGF-artiger und zwei Follistatin-artigen Domänen 2), 224373_s_at (IQ_Motiv und WD-Wiederholungssequenzen 1), 224644_at (CDNA-Klon IMAGE:5278517), 226173_at (Ornithinaminotransferase-artig 1), 226773_at (CDNA FLJ35131 fis, Klon PLACE6008824), 226880_at (nukleäre Caseinkinase und Cyclin-abhängige Kinase-Substrat 1), 228980_at (Ringfinger und FYVE-artige Domäne enthaltend 1), 229569_at (CDNA-Klon IMAGE:5263455), 231735_s_at (PRO 1073-Protein), 233765_at (hypothetisches LOCI97135), 235803_at (Zytokinrezeptor-artiger Faktor 3), 236131_at (CDNA-Klon IMAGE:6622963), 236953_s_at (ähnlich wie RIKEN cDNA 8030451K01), 240544_at (Zinkfinger, AN1-Typ-Domäne 3), 240971_x_at (Cullin 4A), 244042_x_at (ähnliche Retinolsäurerezeptor-Responder (Tazaroten-induziert) 2) für die In-vitro-Diagnose von Myokarditis.

2. Verwendung nach Anspruch 1, wobei die Detektion einer Überexpression der Nukleinsäuremoleküle, komplementären Sequenzen, Allele oder Genprodukte davon in einer biologischen Probe für Myokarditis diagnostisch ist.

3. Verwendung nach Anspruch 1, wobei die Nukleinsäuremoleküle oder Genprodukte davon in einer Zelle oder einem Patienten verglichen mit einer normalen Zelle oder einem normalen Individuum um mindestens 10 % überexprimiert sind.

4. Verwendung nach Anspruch 1, wobei die Nukleinsäuremoleküle, komplementären Sequenzen, Allele oder Genprodukte davon in einer Zelle oder einem Patienten verglichen mit einer normalen Zelle oder einem normalen Individuum um mindestens 50 % überexprimiert sind.

5. Verwendung nach Anspruch 1, wobei die Nukleinsäuremoleküle oder Genprodukte davon in einer Zelle oder einem Patienten verglichen mit einer normalen Zelle oder einem normalen Individuum um mindestens 75 % überexprimiert sind.

6. Verwendung nach Anspruch 1, wobei ein Biochip die Nukleinsäuremoleküle hybridisiert an den Biochip umfasst.

7. Verwendung eines transkriptomischen Biomarkers, bestehend aus Nukleinsäuremolekülen, die komplementär sind zu: 1553212_at (Keratin 78), 1557236_at (Apolipoprotein L), 1558142_at (Trinukleotid-Wiederholungssequenz enthaltend 6B), 1558484_s_at (Leucin-reiche Sequenzwiederholung enthaltend 27), 1565614_at (Zinkfingerprotein 337), 1565662_at (Mucin 6, oligomeren Mucus/Gel-bildend), 1567100_at (Dachshund-Homolog 1 (Drosophila)), 203307_at (Guaninnukleotidbindungsprotein-artig 1), 205758_at (CD8a-Molekül /// CD8a-Molekül), 206333_at (Musashi-Homolog 1 (Drosophila)), 212920_at, 213242_x_at (KIAA0284), 213619_at (heterogenes nukleäres Ribonukleoprotein HI (H)), 213770_at (Kinasesuppressor von ras 1), 214171_s_at (U2-kleiner nukleärer RNA-Hilfsfaktor 2), 215443_at (Rezeptor des schilddrüsenstimulierenden Hormons), 216198_at (Protein, das mit dem aktivierenden Transkriptionsfaktor 7 interagiert), 216427_at (CDNA: FLJ22786 fis, Klon KAIA2150), 217054_at (cDNA FLJ39484 fis, Klon PROST2014925), 217182_at (Mucin 5AC, oligomeren Mucus/Gel-bildend), 217322_x_at, 219425_at (Sulfotransferase-Familie 4A, Mitglied 1), 222145_at (cDNA: FLJ23572 fis, Klon LNG12403), 229191_at (Tubulinfaltungs-Cofaktor D), 229569 at (cDNA-Klon IMAGE:5263455), 231629_x_at (Kallikrein-verwandte Peptidase 3), 233765_at (hypothetisches LOCI97135), 233794_at (einzelsträngiges DNA-Bindungsprotein 3), 233974_s_at (Familie mit Sequenzähnlichkeit 129, Mitglied B), 234495_at (mit Kallikrein verwandte Peptidase 15), 235568_at (offener Leserahmen auf Chromosom 19 59), 235803_at (Zytokinrezeptor-artiger Faktor 3), 236496_at (degenerative Spermatozyten-Homolog 2, Lipiddesaturase (Drosophila)), 236953_s_at, 238445_x_at (Mannosyl(alpha-1,6-)-glycoprotein beta-1,6-N-acetylglucosaminyltransferase, Isozym B), 239463_at (transkribierter Lokus), 240544_at (Zinkfinger, ANI-Typ-Domäne 3) und 243766_s_at (Mitglied der Familie mit TEA-Domäne 2) für die In-vitro-Diagnose von Myokarditis.

8. Verwendung nach Anspruch 7, wobei die Detektion einer Überexpression der Nukleinsäuremoleküle, komplementären Sequenzen, Allele oder Genprodukte davon in einer biologischen Probe für Myokarditis diagnostisch ist.

9. Verwendung nach Anspruch 7, wobei die Nukleinsäuremoleküle, komplementären Sequenzen, Allele oder Genprodukte davon in einer Zelle oder einem Patienten verglichen mit einer normalen Zelle oder einem normalen Individuum um mindestens 10 % überexprimiert sind.

10. Verwendung nach Anspruch 7, wobei die Nukleinsäuremoleküle, komplementären Sequenzen, Allele oder Genprodukte davon in einer Zelle oder einem Patienten verglichen mit einer normalen Zelle oder einem normalen Individuum um etwa 50 % überexprimiert sind.

11. Verwendung nach Anspruch 7, wobei die Nukleinsäuremoleküle oder Genprodukte davon in einer Zelle oder einem Patienten verglichen mit einer normalen Zelle oder einem normalen Individuum um etwa 75 % überexprimiert sind.

12. In-vitro-Verfahren zum Diagnostizieren von Myokarditis, umfassend: Erzeugen einer molekularen Signatur von einem Patienten, wobei die Erzeugung das Messen der Expression von Nukleinsäuremolekülen umfasst, die folgende Nukleinsäuresequenzen umfassen: 1553145_at (hypothetisches Protein FLJ39653), 1553575_at, 1557236_at (Apolipoprotein L, 6), 1558142_at (Trinukleotid-Wiederholungssequenz enthaltend 6B), 1560752_at (F-Box und WD-40-Domäne-Protein 2), 1565614_at (Zinkfingerprotein 337), 1567100_at (Dachshund-Homolog I (Drosophila)), 200068_s_at (Calnexin /// Calnexin), 201031_s_at (heterogenes nukleäres Ribonukleoprotein H1 (H)), 202646_s_at (Kälteschockdomäne enthaltend E1, RNA-bindend), 205758_at (CD8a-Molekül /// CD8a-Molekül), 206188_at (Zinkfingerprotein 623), 212637_s_at (WW-Domäne-enthaltende E3-Ubiquitin-Proteinligase 1), 212920_at, 213317_at (intrazellulärer Chloridkanal 5), 213619_at (heterogenes nukleäres Ribonukleoprotein H1 (H)), 215443_at (Rezeptor des schilddrüsenstimulierenden Hormons), 216198_at (Protein, das mit dem aktivierenden Transkriptionsfaktor 7 interagiert), 217870_s_at (Cytidylatkinase), 218087_s_at (Sorbin und SH3-Domäne enthaltend 1), 222145_at (CDNA: FLJ23572 fis, Klon LNG12403), 223577_x_at (PRO 1073-Protein), 224321_at (Transmembranprotein mit EGF-artiger und zwei Follistatin-artigen Domänen 2), 224373_s_at (IQ_Motiv und WD-Wiederholungssequenzen 1), 224644_at (CDNA-Klon IMAGE:5278517), 226173_at (Ornithinaminotransferaseartig 1), 226773_at (CDNA FLJ35131 fis, Klon PLACE6008824), 226880_at (nukleäre Caseinkinase und Cyclin-abhängige Kinase-Substrat 1), 228980_at (Ringfinger und FYVE-artige Domäne enthaltend 1), 229569_at (CDNA-Klon IMAGE:5263455), 231735_s_at (PRO 1073-Protein), 233765_at (hypothetisches LOCI97135), 235803_at (Zytokinrezeptor-artiger Faktor 3), 236131_at (CDNA-Klon IMAGE:6622963), 236953_s_at (ähnlich wie RIKEN cDNA 8030451K01), 240544_at (Zinkfinger, AN1-Typ-Domäne 3), 240971_x_at (Cullin 4A), 244042_x_at (ähnlich wie Retinolsäurerezeptor-Responder (Tazaroten-induziert) 2);
Analysieren der erzeugten molekularen Signatur;
und Diagnostizieren, ob der Patient Myokarditis hat, ausgehend von der Analyse der erzeugten molekularen Signatur.

13. Verfahren nach Anspruch 12, wobei bei dem Patienten die Diagnose Myokarditis gestellt wird, wenn: 1553212_at (Keratin 78), 1557236_at (Apolipoprotein L), 1558142_at (Trinukleotid-Wiederholungssequenz enthaltend 6B), 1558484_s_at (Leucin-reiche Sequenzwiederholung enthaltend 27), 1565614_at (Zinkfingerprotein 337), 1565662_at (Mucin 6, oligomeren Mucus/Gel-bildend), 1567100_at (Dachshund-Homolog 1 (Drosophila)), 203307_at (Guaninnukleotidbindungsprotein-artig 1), 205758_at (CD8a-Molekül /// CD8a-Molekül), 206333_at (Musashi-Homolog 1 (Drosophila)), 212920_at, 213242_x_at (KIAA0284), 213619_at (heterogenes nukleäres Ribonukleoprotein HI (H)), 213770_at (Kinasesuppressor von ras 1), 214171_s_at (U2-kleiner nukleärer RNA-Hilfsfaktor 2), 215443_at (Rezeptor des schilddrüsenstimulierenden Hormons), 216198_at (Protein, das mit dem aktivierenden Transkriptionsfaktor 7 interagiert), 216427_at (CDNA: FLJ22786 fis, Klon KAIA2150), 217054_at (cDNA FLJ39484 fis, Klon PROST2014925), 217182_at (Mucin 5AC, oligomeren Mucus/Gel-bildend), 217322_x_at, 219425_at (Sulfotransferase-Familie 4A, Mitglied 1), 222145_at (cDNA: FLJ23572 fis, Klon LNG12403), 229191_at (Tubulinfaltungs-Cofaktor D), 229569 at (cDNA-Klon IMAGE:5263455), 231629_x_at (Kallikrein-verwandte Peptidase 3), 233765_at (hypothetisches LOCI97135), 233794_at (einzelsträngiges DNA-Bindungsprotein 3), 233974_s_at (Familie mit Sequenzähnlichkeit 129, Mitglied B), 234495_at (mit Kallikrein verwandte Peptidase 15), 235568_at (offener Leserahmen auf Chromosom 19 59), 235803_at (Zytokinrezeptor-artiger Faktor 3), 236496_at (degenerative Spermatozyten-Homolog 2, Lipiddesaturase (Drosophila)), 236953_s_at, 238445_x_at (Mannosyl(alpha-1,6-)-glycoprotein beta-1,6-N-acetylglucosaminyltransferase, Isozym B), 239463 _at (transkribierter Lokus), 240544_at (Zinkfinger, ANI-Typ-Domäne 3) und 243766_s_at (Mitglied der Familie mit TEA-Domäne 2) und 244042 x hochreguliert sind.

14. Verfahren nach Anspruch 12 oder 13, wobei das Erzeugen ferner das Isolieren von Nukleinsäuremoleküle umfasst, die aus einer biologischen Probe erhalten wurden, und
die isolierten Nukleinsäuremoleküle vorzugsweise an einen Biochip hybridisiert sind, der komplementäre Bukleinsäuremoleküle umfasst, und Intensitätsrohwerte aus der Hybridisierung vorzugsweise normalisiert sind und phänotypspezifische Unterschiede der Genexpression vorzugsweise identifiziert werden, und
wobei die Unterschiede in der Genexpression vorzugsweise durch Signifikanzanalyse von Mikroarrays identifiziert werden, wobei Signifikanz mit einem q-Wert definiert ist und Mehrfachvergleiche einen angepassten p-Wert umfassen, und
wobei die Phänotypspezifität vorzugsweise identifiziert wird, indem ein Klassifikator in einem Trainingssatz umfassend etwa 66 % der erhaltenen Daten erstellt wird, mit anschließender Validierung in einem Testsatz umfassend etwa 33 % der erhaltenen Daten, und Definieren eines phänotypspezifischen "Nearest Shrunken Centroids" zur Klassifizierung, und
wobei das phänotypspezifische "Nearest Shrunken Centroid" vorzugsweise das Balancieren einer etwa 10-fachen Kreuzvalidierung in einem Trainingssatz umfasst.

15. Verwendung nach Anspruch 1, wobei ein Kit die Nukleinsäuremoleküle umfasst.

16. Verwendung nach Anspruch 15, wobei ein Biochip die Nukleinsäuremoleküle umfasst.

17. Verwendung eines Biochips, umfassend an den Biochip hybridisierte Nukleinsäuremoleküle, wobei die Nukleinsäuremoleküle bestehen aus: 1553212_at (Keratin 78), 1557236_at (Apolipoprotein L), 1558142_at (Trinukleotid-Wiederholungssequenz enthaltend 6B), 1558484_s_at (Leucin-reiche Sequenzwiederholung enthaltend 27), 1565614_at (Zinkfingerprotein 337), 1565662_at (Mucin 6, oligomeren Mucus/Gel-bildend), 1567100 at (Dachshund-Homolog 1 (Drosophila)), 203307_at (Guaninnukleotidbindungsprotein-artig 1), 205758_at (CD8a-Molekül /// CD8a-Molekül), 206333_at (Musashi-Homolog 1 (Drosophila)), 212920_at, 213242_x_at (KIAA0284), 213619_at (heterogenes nukleäres Ribonukleoprotein H1 (H)), 213770_at (Kinasesuppressor von ras 1), 214171_s_at (U2-kleiner nukleärer RNA-Hilfsfaktor 2), 215443_at (Rezeptor des schilddrüsenstimulierenden Hormons), 216198_at (Protein, das mit dem aktivierenden Transkriptionsfaktor 7 interagiert), 216427_at (CDNA: FLJ22786 fis, Klon KAIA2150), 217054_at (cDNA FLJ39484 fis, Klon PROST2014925), 217182_at (Mucin 5AC, oligomeren Mucus/Gel-bildend), 217322_x_at, 219425_at (Sulfotransferase-Familie 4A, Mitglied 1), 222145_at (cDNA: FLJ23572 fis, Klon LNG12403), 229191_at (Tubulinfaltungs-Cofaktor D), 229569 at (cDNA-Klon IMAGE:5263455), 231629_x_at (Kallikrein-verwandte Peptidase 3), 233765_at (hypothetisches LOCI97135), 233794 at (einzelsträngiges DNA-Bindungsprotein 3), 233974_s_at (Familie mit Sequenzähnlichkeit 129, Mitglied B), 234495_at (mit Kallikrein verwandte Peptidase 15), 235568_at (offener Leserahmen auf Chromosom 19 59), 235803_at (Zytokinrezeptor-artiger Faktor 3), 236496_at (degenerative Spermatozyten-Homolog 2, Lipiddesaturase (Drosophila)), 236953_s_at, 238445_x_at (Mannosyl(alpha-1,6-)-glycoprotein beta-1,6-N-acetylglucosaminyltransferase, Isozym B), 239463 _at (transkribierter Lokus), 240544_at (Zinkfinger, ANI-Typ-Domäne 3) und 243766_s_at (Mitglied der Familie mit TEA-Domäne 2) für die In-vitro-Diagnose von Myokarditis.

## Revendications

1. Utilisation d'une composition moléculaire comprenant des molécules d'acide nucléique constituées de séquences d'acide nucléique de : 1553145_at (protéine hypothétique FLJ39653), 1553575_at, 1557236_at (apolipoprotéine L, 6), 1558142_at (répétition de trinucléotide contenant 6B), 1560752_at (Protéine 2 des domaines F-box et WD-40), 1565614_at (protéine en doigt de zinc 337), 1567100_at (homologue du Dachshund I (Drosophile)), 200068_s_at (calnexine /// calnexine), 201031_s_at (ribonucléoprotéine nucléaire hétérogène H1 (H)), 202646_s_at (domaine de choc thermique contenant E1, liaison à l'ARN), 205758_at (molécule CD8a /// molécule CD8a), 206188_at (protéine en doigt de zinc 623), 212637_s_at (domaine WW contenant la protéine ubiquitine E3 ligase 1), 212920_ at, 213317_ at (canal intracellulaire du chlorure 5), 213619_at (ribonucléoprotéine nucléaire hétérogène H1 (H)), 215443_at (récepteur de l'hormone stimulatrice de la thyroïde), 216198_at (protéine d'interaction avec le facteur de transcription activateur 7), 217870_s_at (cytidylate kinase), 218087_s_at (sorbine et domaine SH3 contenant 1), 222145_at (ADNc:FLJ23572 fis, clone LNG12403), 223577_x_at (protéine PR01073), 224321_at (protéine transmembranaire avec un domaine du type EGF et deux domaines du type de la follistatine 2), 224373_s_at (motif IQ et répétitions WD 1), 224644_at (clone image d'ADNc:5278517), 226173_at (omithine de type aminotransférase 1), 226773_at (ADNc FLJ35131 fis, clone PLACE6008824), 226880_at (caséine kinase nucléaire et substrat de kinase cyclino-dépendante 1), 228980 at (domaine ring finger et domaine de type FYVE contenant 1), 229569_ at (clone image d'ADNc:5263455), 231735_s_at (protéine PR01073), 233765_at (LOCI97135 hypothétique), 235803_at (Facteur 3 de type récepteur de cytokines), 236131_at (clone image d'ADNc:6622963), 236953_s_at (semblable au RIKEN ADNc 8030451K01), 240544_at (Doigt de zinc, Domaine de type ANI 3), 240971_x_at (Cullin 4A), et 244042_x_at (Semblable au récepteur de l'acide rétinoïque à chaîne invariant (induit par le tazarotène) 2) pour le diagnostic in vitro de la myocardite.

2. Utilisation selon la revendication 1, dans laquelle la détection de la surexpression des molécules d'acide nucléique, des séquences complémentaires, des allèles, ou des produits de gènes de ceux-ci, dans un échantillon biologique représente le diagnostic de la myocardite.

3. Utilisation selon la revendication 1, dans laquelle des molécules d'acide nucléique, ou des produits de gènes de ceux-ci, sont surexprimés à des niveaux d'au moins 10% dans une cellule ou un patient par rapport aux niveaux d'une cellule normale ou d'un sujet normal.

4. Utilisation selon la revendication 1, dans laquelle des molécules d'acide nucléique, des séquences complémentaires, des allèles, ou des produits de gènes de ceux-ci, sont surexprimés d'environ 50% dans une cellule ou un patient par rapport aux niveaux d'une cellule normale ou d'un sujet normal.

5. Utilisation selon la revendication 1, dans laquelle des molécules d'acide nucléique, ou des produits de gènes de ceux-ci, sont surexprimés d'environ 75% dans une cellule ou un patient par rapport aux niveaux d'une cellule normale ou d'un sujet normal.

6. Utilisation selon la revendication 1, dans laquelle une biopuce comprend des molécules d'acide nucléique hybridées à la biopuce.

7. Utilisation d'un biomarqueur transcriptomique constitué de molécules d'acide nucléique complémentaires à : 1553212_at (kératine 78), 1557236_at (apolipoprotéine L), 1558142_at (répétition de trinucléotide contenant 6B), 1558484_s_at (répétition riche en leucine contenant 27), 1565614_at (Protéine en doigt de zinc 337), 1565662_at (Mucine 6, mucus oligomérique/formant un gel), 1567100_at (Homologue du Dachshund 1 (Drosophile)), 203307_at (liaison guanine-nucléotide de type protéinique 1), 205758_at (molécule CD8a /// molécule CD8a), 206333_at (homologue de musashi 1 (Drosophile)), 212920_at, 213242_x_at (KIAA0284), 213619_at (ribonucléoprotéine nucléaire hétérogène HI (H)), 213770_at (suppresseur kinase de ras 1), 214171_s_at (U2 petit facteur auxiliaire d'ARN nucléaire 2), 215443_at (récepteur de l'hormone stimulatrice de la thyroïde), 216198_at (protéine d'interaction avec le facteur de transcription activateur 7), 216427_at (ADNc:FLJ22786 fis, clone KAIA2150), 217054_at (ADNc FLJ39484 fis, clone PROST2014925), 217182_at (mucine 5AC, mucus oligomérique/formant un gel), 217322_x_at, 219425_at (famille de la sulfotransférase 4A, membre 1), 222145_at (ADNc:FLJ23572 fis, clone LNG12403), 229191_at (cofacteur de repliement des tubulines D), 229569 at (clone image d'ADNc:5263455), 231629_x_at (peptidase associée à la kallicréine 3), 233765_at (LOCI97135 hypothétique), 233794_at (protéine liante (BP) d'ADN à brin unique 3), 233974_s_at (famille à similarité de séquence 129, membre B), 234495_at (peptidase associée à la kallicréine 15), 235568_at (chromosome 19 cadre de lecture ouvert 59), 235803_at (facteur 3 de type récepteur de cytokines), 236496_at (homologue de spermatocyte dégénératif 2, désaturase de lipide (Drosophile)), 236953_s_at, 238445_x_at (mannosyl (alpha-1,6-)-glycoprotéine béta-1,6-N-acétyl-glucosaminyltransférase, isozyme B), 239463 _at (Locus transcrit), 240544_at (Doigt de zinc, domaine de type ANI 3) et 243766_s_at (membre 2 de la famille du domaine TEA), pour le diagnostic in vitro de la myocardite.

8. Utilisation selon la revendication 7, dans laquelle la détection de la surexpression des molécules d'acide nucléique, des séquences complémentaires, des allèles, ou des produits de gènes de ceux-ci, dans un échantillon biologique représente le diagnostic de la myocardite.

9. Utilisation selon la revendication 7, dans laquelle des molécules d'acide nucléique, des séquences complémentaires, des allèles, ou des produits de gènes de ceux-ci, sont surexprimés à des niveaux d'au moins 10% dans une cellule ou un patient par rapport aux niveaux d'une cellule normale ou d'un sujet normal.

10. Utilisation selon la revendication 7, dans laquelle des molécules d'acide nucléique, des séquences complémentaires, des allèles, ou des produits de gènes de ceux-ci, sont surexprimés d'environ 50% dans une cellule ou un patient par rapport aux niveaux d'une cellule normale ou d'un sujet normal.

11. Utilisation selon la revendication 7, dans laquelle des molécules d'acide nucléique, des séquences complémentaires, des allèles, ou des produits de gènes de ceux-ci, sont surexprimés d'environ 75% dans une cellule ou un patient par rapport aux niveaux d'une cellule normale ou d'un sujet normal.

12. Procédé in vitro de diagnostic de la myocardite, comprenant les étapes consistant à : générer à partir d'un patient une signature moléculaire, dans laquelle la génération comprend la mesure de l'expression des molécules d'acide nucléique comprenant des séquences d'acide nucléique : 1553145_at (protéine hypothétique FLJ39653), 1553575_at, 1557236_at (apolipoprotéine L, 6), 1558142_at (répétition de trinucléotide contenant 6B), 1560752_at (protéine 2 des domaines F-box et WD-40), 1565614_at (Protéine en doigt de zinc 337), 1567100_at (Homologue du Dachshund I (Drosophile)), 200068_s_at (calnexine /// calnexine), 201031_s_at (ribonucléoprotéine nucléaire hétérogène Hl (H)), 202646_s_at (domaine de choc thermique contenant E1, liaison à l'ARN), 205758_at (molécule CD8a /// molécule CD8a), 206188_at (protéine en doigt de zinc 623), 212637_s_at (domaine WW contenant la protéine ubiquitine E3 ligase 1), 212920_ at, 213317_ at (canal intracellulaire du chlorure 5), 213619_at (ribonucléoprotéine nucléaire hétérogène H1 (H)), 215443_at (récepteur de l'hormone stimulatrice de la thyroïde), 216198_at (protéine d'interaction avec le facteur de transcription activateur 7), 217870_s_at (cytidylate kinase), 218087_s_at (sorbine et domaine SH3 contenant 1), 222145_at (ADNc:FLJ23572 fis, clone LNG12403), 223577_x_at (protéine PRO1073), 224321_at (protéine transmembranaire avec un domaine du type EGF et deux domaines du type de la follistatine 2), 224373_s_at (motif IQ et répétitions WD 1), 224644_at (clone image d'ADNc:5278517), 226173_at (omithine de type aminotransférase 1), 226773_at (ADNc FLJ35131 fis, clone PLACE6008824), 226880_at (caséine kinase nucléaire et substrat de kinase cyclino-dépendante 1), 228980 at (domaine ring finger et domaine de type FYVE contenant 1), 229569_at (clone image d'ADNc:5263455), 231735_s_at (Protéine PRPO1073), 233765_at (LOCI97135 hypothétique), 235803_at (Facteur 3 de type récepteur de cytokines), 236131_at (clone image d'ADNc:6622963), 236953_s_at (semblable au RIKEN ADNc 8030451K01), 240544_at (Doigt de zinc, domaine de type ANI 3), 240971_x_at (Cullin 4A) et 244042_x_at (Semblable au récepteur de l'acide rétinoïque à chaîne invariant (induit par le tazarotène) 2) ;
analyser la signature moléculaire générée ;
et diagnostiquer si oui ou non le patient souffre de myocardite après analyse de la signature moléculaire générée.

13. Procédé selon la revendication 12, dans lequel le patient souffre de myocardite si : 1553212_at (kératine 78), 1557236_at (apolipoprotéine L), 1558142_at (répétition de trinucléotide contenant 6B), 1558484_s_at (répétition riche en leucine contenant 27), 1565614_at (Protéine en doigt de zinc 337), 1565662_at (Mucine 6, mucus oligomérique/formant un gel), 1567100_at (Homologue du Dachshund 1 (Drosophile)), 203307_at (liaison guanine-nucléotide de type protéinique 1), 205758_at (molécule CD8a /// molécule CD8a), 206333_at (homologue de musashi 1 (Drosophile)), 212920_at, 213242_x_at (KIAA0284), 213619_at (ribonucléoprotéine nucléaire hétérogène HI (H)), 213770_at (suppresseur kinase de ras 1), 214171_s_at (petit facteur auxiliaire U2 d'ARN nucléaire 2), 215443_at (récepteur de l'hormone stimulatrice de la thyroïde), 216198_at (protéine d'interaction avec le facteur de transcription activateur 7), 216427_ at (ADNC: FLJ22786 fis, clone KAIA2150), 217054_at (ADNc FLJ39484 fis, clone PROST2014925), 217182_at (mucine 5AC, mucus oligomérique/formant un gel), 3217322_x_at, 219425 at (famille de la sulfotransférase 4A, membre 1), 222145_at (ADNc:FLJ23572 fis, clone LNG12403), 229191_at (cofacteur de repliement des tubulines D), 229569 at (clone image d'ADNc:5263455), 231629_x_at (peptidase associée à la kallicréine 3), 233765_at (LOCI97135 hypothétique), 233794_at (Protéine liante (BP) d'ADN à brin unique 3), 233974_s_at (famille à similarité de séquence 129, membre B), 234495_at (peptidase associée à la kallicréine 15), 235568_at (chromosome 19 cadre de lecture ouvert 59), 235803_at (Facteur 3 de type récepteur de cytokines), 236496_at (homologue de spermatocyte dégénératif 2, désaturase de lipide (Drosophile)), 236953_s_at, 238445_x_at (mannosyl (alpha-1,6-)-glycoprotéine béta-1,6-N-acétyl-glucosaminyltransférase, isozyme B), 239463 at (Locus transcrit), 240544_at (Doigt de zinc, domaine de type ANI 3), 243766_s_at (Membre 2 de la famille de domaine TEA) et 244042_x_at sont régulés.

14. Procédé selon la revendication 12 ou la revendication 13, dans lequel la génération comprend en outre l'isolation des molécules d'acide nucléique obtenues à partir d'un échantillon biologique, et
les molécules d'acide nucléique isolées sont préférablement hybridées à une biopuce comprenant des molécules d'acide nucléique complémentaires et des valeurs d'intensité brute à partir de l'hybridation sont préférablement normalisées et des différences spécifiques au phénotype dans l'expression génétique sont préférablement identifiées, et
dans lequel les différences dans l'expression génétique sont préférablement identifiées par l'analyse de signification de micro-réseaux, dans laquelle signification est définie par une valeur q et de multiples comparaisons comprennent une valeur p ajustée, et
dans lequel la spécificité du phénotype est préférablement identifiée en créant un classificateur dans un ensemble de formation comprenant environ 66% de données obtenues, avec une validation subséquente dans un ensemble de tests comprenant environ 33% de données obtenues et définissant un centre de gravité ratatiné le plus proche spécifique du phénotype pour une classification, et
dans lequel le centre de gravité ratatiné le plus proche spécifique du phénotype comprend préférablement l'équilibrage d'une validation croisée par 10 fois dans un ensemble de formation.

15. Utilisation selon la revendication 1, dans laquelle un kit comprend des molécules d'acide nucléique

16. Utilisation selon la revendication 15, dans laquelle une biopuce comprend des molécules d'acide nucléique.

17. Utilisation d'une biopuce comprenant des molécules d'acide nucléique hybridées à la biopuce, dans laquelle des molécules d'acide nucléique sont composées de : 1553212_at (kératine 78), 1557236_at (apolipoprotéine L), 1558142_at (répétition de trinucléotide contenant 6B), 1558484_s_at (répétition riche en leucine contenant 27), 1565614_at (Protéine en doigt de zinc 337), 1565662_at (Mucine 6, mucus oligomérique/formant un gel), 1567100_at (Homologue du Dachshund 1 (Drosophile)), 203307_at (liaison guanine-nucléotide de type protéinique 1), 205758_at (molécule CD8a /// molécule CD8a), 206333_at (homologue de musashi 1 (Drosophile)), 212920_at, 213242_x_at (KIAA0284), 213619_at (ribonucléoprotéine nucléaire hétérogène H1 (H)), 213770_at (suppresseur kinase de ras 1), 214171,_s_at (petit facteur auxiliaire U2 d'ARN nucléaire 2), 215443_at (récepteur de l'hormone stimulatrice de la thyroïde), 216198_at (protéine d'interaction avec le facteur de transcription activateur 7), 216427_ at (ADNC: FLJ22786 fis, clone KAIA2150), 217054_at (ADNc FLJ39484 fis, clone PROST2014925), 217182_at (mucine 5AC, mucus oligomérique/formant un gel), 217322_x_at, 219425_at (famille de la sulfotransférase 4A, membre 1), 222145_at (ADNc:FLJ23572 fis, clone LNG12403), 229191_at (cofacteur de repliement des tubulines D), 229569 at (clone image d'ADNc:5263455), 231629_x_at (peptidase associée à la kallicréine 3), 233765_at (LOCI97135 hypothétique), 233794_at (protéine liante (BP) d'ADN à brin unique 3), 233974_s_at (famille à similarité de séquence 129, membre B), 234495_at (peptidase associée à la kallicréine 15), 235568_at (chromosome 19 cadre de lecture ouvert 59), 235803_at (Facteur 3 de type récepteur de cytokines), 236496_at (homologue de spermatocyte dégénératif 2, désaturase de lipide (Drosophile)), 236953_s_at, 238445_x_at (mannosyl (alpha-1,6)-glycoprotéine béta-1,6-N-acétyl-glucosaminyltransférase, isozyme B), 239463 _at (Locus transcrit), 240544_at (Doigt de zinc, domaine de type ANI 3), 243766_s_at (et Membre 2 de la famille de domaine TEA) pour le diagnostic in vitro de la myocardite.
